# EUROPEAN PATENT APPLICATION

(11) **EP 4 556 475 A1**
(43) Date of publication of application: **21.05.2025**
(21) Application number: 23838997.7
(22) Date of filing: 13.07.2023
(51) Int. Cl.: C07D 471/04, A61K 31/519, A61P 35/00

(54) **BICYCLIC-TYPE MAT2A INHIBITOR AND USE THEREOF**

(30) Priority: 13.07.2022 CN 202210828873
(71) Applicant: Haihe Biopharma Co., Ltd., Shanghai 201203 (CN)
(72) Inventor: LI, Zheng, Shanghai 201203 (CN); YANG, Junyu, Shanghai 201203 (CN); TU, Wangyang, Shanghai 201203 (CN); YU, Bing, Shanghai 201203 (CN); ZHANG, Yixiang, Shanghai 201203 (CN); LI, Leping, Shanghai 201203 (CN)
(74) Representative: Grünecker Patent- und Rechtsanwälte PartG mbB
(86) International application number: PCT/CN2023/107109
(87) International publication number: WO 2024/012507

(57) **Abstract**

The present invention relates to a bicyclic-type MAT2A inhibitor and use thereof. Specifically, the present invention discloses a bicyclic compound represented by formula (I) or a pharmaceutically acceptable salt, an enantiomer, a diastereomer, a racemate, an atropisomer, a polymorph, a solvate, or an isotope labeled compound thereof. The compound represented by formula (I) has MAT2A enzyme inhibitory activity, can be used as a good MAT2A inhibitor, and is further used for preparing drugs for treating and/or preventing MTAP-related diseases, especially tumors.

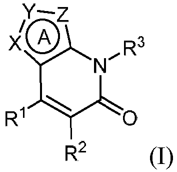

## Description

The present application claims priority for Chinese patent application with application number 202210828873.6, filed on July 13, 2022, and named "BICYCLIC-TYPE MAT2A INHIBITOR AND USE THEREOF", the entire disclosures of which are incorporated herein by reference in their entirety.

### TECHNICAL FIELD

The present invention relates to a MAT2A inhibitor, specifically to a bicyclic compound represented by formula I below, a pharmaceutical composition comprising the compound, and a use in preparing drugs for treating and/or preventing MAT2A-related diseases, especially tumors.

### BACKGROUND

Cancer treatment is a huge challenge the world faces today. The biggest problem with existing commonly used therapies, such as chemotherapy and immunotherapy, is that cell killing effects often not only target cancer cells, but also have significant side effects on normal cells and tissues. Therefore, there is an urgent need to develop a new therapeutic method to better target cancer cells.

Synthetic lethality is defined as the loss of two or more genes leading to cell death, wherein the individual loss of any one gene has no effect. In recent years, a large number of studies have shown that there are multiple gene mutations in cancer cells that make them more sensitive to treatment methods of the synthetic lethality. These tumor specific gene mutations can prompt us to use appropriate targeted therapeutic drugs to kill cancer cells without affecting normal cells.

Methionine adenosyltransferase 2A (MAT2A) is an enzyme that catalyzes the reaction of methionine (Met) with ATP to produce S-adenosyl-L-methionine (SAM). SAM is a main methyl donor in the body, which can regulate gene expression through a transmethylation
reaction of DNA, RNA, and proteins, thereby exerting significant effects on cell differentiation, growth, and death. Arginine N-methyltransferase 5 (PRMT5) is a methylation enzyme that uses SAM as a methyl donor. SAM is crucial for the activity of PRMT5, while 5' methylthioadenosine (MTA) can inhibit the activity of PRMT5. MTA is a product of a methionine compensation pathway, which catalyzes the production of 5-methylthioribose-1-phosphate and adenine in cells through methylthioadenosine monophosphate (MTAP) and maintains them at low levels.

MTAP gene is located on chromosome 9, which is deleted in cells of many cancer patients, including pancreatic cancer, esophageal cancer, bladder cancer and lung cancer (cBioPortal database). The deletion of MTAP leads to the enrichment of intracellular MTA, making these cells more dependent on SAM production and MAT2A activity compared to normal cells. Research has shown that inhibiting the expression of MAT2A in MTAP deficient cancer cells can selectively suppress cell activity compared to MTAP normal cancer cells (McDonald et al., 2017 Cell 170, 577-592). Meanwhile, reducing MAT2A expression can selectively inhibit tumor growth in a mouse xenograft tumor model with MTAP deficient tumor cells (Marjon et al., 2016 Cell Reports 15(3), 574-587). These results indicate that a MAT2A inhibitor can provide a novel and effective treatment method for cancer patients, especially those with tumors containing MTAP deficiency.

### SUMMARY

The inventor unexpectedly discovered that a compound of formula (I) of the present invention has a MAT2A enzyme activity inhibitory effect, and its tumor cell activity inhibitory effect was verified through cell experiments. Therefore, the compound of formula (I) can serve as a good MAT2A inhibitor.

One object of the present invention is to provide a bicyclic compound represented by formula (I) or a pharmaceutically acceptable salt, an enantiomer, a diastereomer, a racemate, an atropisomer, a polymorph, a solvate, or an isotope labeled compound thereof,

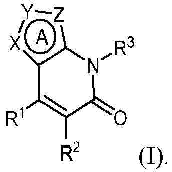

Another object of the present invention is to provide a pharmaceutical composition, which comprises a therapeutically effective dose of one or more selected from the compound represented by formula (I) or a pharmaceutically acceptable salt, an enantiomer, a diastereomer, a racemate, an atropisomer, a polymorph, a solvate, or an isotope labeled compound thereof, and at least one pharmaceutically acceptable carrier.

A further object of the present invention is to provide the compound represented by formula (I) or a pharmaceutically acceptable salt, an enantiomer, a diastereomer, a racemate, an atropisomer, a polymorph, a solvate, or an isotope labeled compound thereof, or the pharmaceutical composition, in preparing drugs for inhibiting MAT2A activity.

A still further object of the present invention is to provide a use of the compound represented by formula (I) or a pharmaceutically acceptable salt, an enantiomer, a diastereomer, a racemate, an atropisomer, a polymorph, a solvate, or an isotope labeled compound thereof, or the pharmaceutical composition, in preparing drugs for treating and/or preventing MAT2A-related diseases, especially tumors.

In order to achieve the above object, a first aspect of the present invention provides an bicyclic compound represented by formula (I) or a pharmaceutically acceptable salt, an enantiomer, a diastereomer, a racemate, an atropisomer, a polymorph, a solvate or an isotope labeled compound thereof, 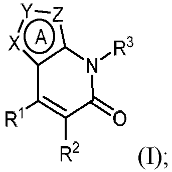 wherein,
R¹ is selected from halogen, C₁-C₆ alkyl, C₂-C₆ alkenyl, C₂-C₆ alkynyl, C₁-C₆ alkylsulfonyl, C₃-C₇ cycloalkyl, 3- to 6-membered heterocycloalkyl, cyano, nitro, carboxyl, -NR^{a}R^{a2}, -NHCOR^{a}, -OR^{a}, and -SR^{a}, the C₁-C₆ alkyl, C₂-C₆ alkenyl, C₂-C₆ alkynyl, C₃-C₇ cycloalkyl, and 3- to 6-membered heterocycloalkyl are unsubstituted or optionally substituted with one or more substituents selected from D and halogen;
R^{a} and R^{a2} are each independently selected from H, C₁-C₁₀ alkyl, C₃-C₁₀ cycloalkyl, C₆-C₁₀ aryl, 5- to 10-membered heteroaryl, 3- to 6-membered heterocycloalkyl, C₁-C₁₀ alkyl substituted with one or more substituents selected from group A, and C₃-C₁₀ cycloalkyl substituted with one or more substituents selected from the group A; the group A substituents include D, halogen, C₁-C₃ alkoxy, hydroxyl, C₆-C₁₀ aryl, 5- to 10-membered heteroaryl, and C₃-C₁₀ cycloalkyl that is unsubstituted or substituted with one or more substituents selected from group A2; the group A2 substituents include D, halogen, hydroxyl, C₁-C₆ alkyl, and C₁-C₁₀ alkoxy;
R² and R³ are each independently selected from unsubstituted or substituted C₃-C₁₀ cycloalkyl, unsubstituted or substituted C₆-C₁₀ aryl, unsubstituted or substituted 4- to 6-membered heterocycloalkyl, and unsubstituted or substituted 5- to 10-membered heteroaryl; wherein the substitution refers to being substituted by one or more substituents selected from group B, the group B substituents include halogen, cyano (-CN), hydroxyl (-OH), oxo (=O), mercapto (-SH), amino (-NH₂), nitro (-NO₂), 4- to 6-membered heterocycloalkyl, C₁-C₄ alkyl that is unsubstituted or substituted with one or more substituents selected from group C, C₃-C₇ cycloalkyl that is unsubstituted or substituted with halogen, C₁-C₄ alkoxy that is unsubstituted or substituted with halogen, -COOH, -CONHR^{b}, -NHCOR^{b}, and -NHSO₂R^{b}; the group C substituents include D, halogen, hydroxyl, C₃-C₆ cycloalkyl, 4- to 6-membered heterocycloalkyl, and C₁-C₄ alkoxy;
R^{b} is selected from H, C₁-C₄ alkyl, C₃-C₁₀ cycloalkyl, C₁-C₁₀ alkoxy, and C₆-C₁₀ aryl, wherein the C₁-C₄ alkyl, C₃-C₁₀ cycloalkyl, C₁-C₁₀ alkoxy, and C₆-C₁₀ aryl in R^{b} are unsubstituted or substituted with one or more groups selected from halogen, hydroxyl, and cyano;
ring A is a five-membered heteroaromatic ring, wherein at most one of X, Y, and Z is CR⁴, and the rest are each independently selected from N, NR⁵, O, and S; and
R⁴ and R⁵ are each independently selected from H, D, halogen, amino, C₁-C₆ alkyl, and C₃-C₆ cycloalkyl; alternatively, R⁴ and R⁵ are each independently selected from H, D, halogen, amino, C₁-C₆ alkyl, and C₃-C₆ cycloalkyl; the C₁-C₆ alkyl and C₃-C₆ cycloalkyl are unsubstituted or substituted with hydroxyl.

In some embodiments, R¹ is selected from C₁-C₆ alkyl, C₃-C₇ cycloalkyl, 3-6 membered heterocycloalkyl, -OR^{a}, and NR^{a}R^{a2}; the C₁-C₆ alkyl, C₃-C₇ cycloalkyl, and 3-6 membered heterocycloalkyl are unsubstituted or substituted with one or more substituents selected from D and halogen; R^{a} and R^{a2} are each independently selected from H, C₃-C₇ cycloalkyl, and C₁-C₆ alkyl that is unsubstituted or substituted with one or more substituents selected from group A, the group A substituents include: D, halogen, C₁-C₃ alkoxy, hydroxyl, C₆-C₁₀ aryl, 5-10 membered heteroaryl, and C₃-C₁₀ cycloalkyl that is unsubstituted or substituted with one or more substituents selected from group A2; the group A2 substituents includes: D, halogen, hydroxyl, C₁-C₆ alkyl, and C₁-C₆ alkoxy.

In some embodiments, R¹ is selected from C₁-C₆ alkyl, C₃-C₇ cycloalkyl, 3-6 membered heterocycloalkyl, -OR^{a}, -SR^{a}, and NR^{a}R^{a2}; the C₁-C₆ alkyl, C₃-C₇ cycloalkyl, and 3-6 membered heterocycloalkyl are unsubstituted or substituted with one or more substituents selected from D and halogen; R^{a} and R^{a2} are each independently selected from H, C₃-C₇ cycloalkyl, and C₁-C₆ alkyl that is unsubstituted or substituted with one or more substituents selected from group A, the group A substituents include: D, halogen, C₁-C₃ alkoxy, hydroxyl, C₆-C₁₀ aryl, 5-10 membered heteroaryl, and C₃-C₁₀ cycloalkyl that is unsubstituted or substituted with one or more substituents selected from group A2; the group A2 substituents includes: D, halogen, hydroxyl, C₁-C₆ alkyl, and C₁-C₆ alkoxy.

In some embodiments, R² and R³ are each independently selected from unsubstituted or substituted C₃-C₁₀ cycloalkyl, unsubstituted or substituted C₆-C₁₀ aryl, and unsubstituted or substituted 5- to 10-membered heteroaryl, the substitution refers to being substituted by one or more substituents selected from group B; the group B substituents include halogen, cyano, hydroxyl, mercapto, nitro, amino, oxo, unsubstituted or substituted 4- to 6-membered heterocycloalkyl, C₁-C₄ alkyl that is unsubstituted or substituted with one or more substituents selected from group C, C₃-C₇ cycloalkyl that is unsubstituted or substituted with halogen, and C₁-C₄ alkoxy that is unsubstituted or substituted with halogen; the group C substituents include D, halogen, hydroxyl, C₃-C₆ cycloalkyl, 4- to 6-membered heterocycloalkyl, and C₁-C₄ alkoxy; particularly, the 5- to 10-membered heteroaryl is selected from benzene-fused 5-membered heteroaryl, benzene-fused 6-membered heteroaryl, 6-membered heteroaryl-fused 5-membered heteroaryl, 6-membered heteroaryl-fused 6-membered heteroaryl, and

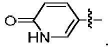

In some embodiments, R¹ is selected from C₂-C₆ alkyl, C₃-C₆ cycloalkyl, -OR^{a}, and NR^{a}R^{a2}, wherein the C₂-C₆ alkyl and C₃-C₆ cycloalkyl are unsubstituted or substituted with one or more substituents selected from D and halogen; R^{a} and R^{a2} are each independently selected from H, C₃-C₆ cycloalkyl, and C₂-C₆ alkyl that is unsubstituted or substituted with one or more substituents selected from group A, the group A substituents include D, halogen, methoxy, hydroxyl, and C₃-C₆ cycloalkyl.

In some embodiments, R¹ is selected from C₂-C₆ alkyl, C₃-C₆ cycloalkyl, -OR^{a}, -SR^{a}, and NR^{a}R^{a2}; wherein the C₂-C₆ alkyl and C₃-C₆ cycloalkyl are unsubstituted or substituted with one or more substituents selected from D and halogen; R^{a} and R^{a2} are each independently selected from H, C₃-C₆ cycloalkyl, and C₂-C₆ alkyl that is unsubstituted or substituted with one or more substituents selected from group A, the group A substituents include D, halogen, methoxy, hydroxyl, and C₃-C₆ cycloalkyl.

In some embodiments, R² and R³ are each independently selected from unsubstituted or substituted cyclohexyl, unsubstituted or substituted phenyl, and unsubstituted or substituted 5- to 10-membered heteroaryl, the 5- to 10-membered heteroaryl is selected from the following structures: 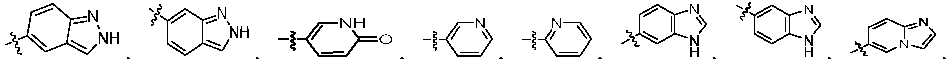 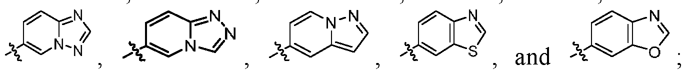 the substitution in the unsubstituted or substituted phenyl, and unsubstituted or substituted 5- to 10-membered heteroaryl refers to being substituted by one or more substituents selected from group B, the group B substituents include halogen, cyano, hydroxyl, mercapto, nitro, amino, 4- to 6-membered heterocycloalkyl, C₁-C₄ alkyl that is unsubstituted or substituted with one or more substituents selected from group C, C₃-C₇ cycloalkyl that is unsubstituted or substituted with halogen, and C₁-C₄ alkoxy that is unsubstituted or substituted with halogen; the group C substituents include D, halogen, C₃-C₆ cycloalkyl, hydroxyl, 4- to 6-membered heterocycloalkyl, and C₁-C₄ alkoxy.

In some embodiments, R² and R³ are each independently selected from unsubstituted or substituted cyclohexyl, unsubstituted or substituted phenyl, and unsubstituted or substituted 5- to 10-membered heteroaryl, the 5- to 10-membered heteroaryl is selected from the following structures: 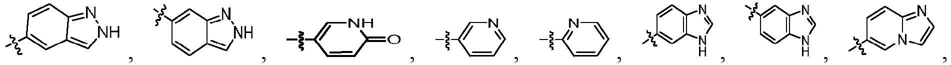 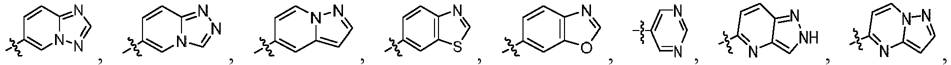 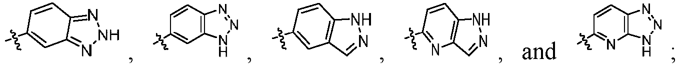 the substitution in the unsubstituted or substituted phenyl, and unsubstituted or substituted 5- to 10-membered heteroaryl refers to being substituted by one or more substituents selected from group B, the group B substituents include halogen, cyano, hydroxyl, mercapto, nitro, amino, 4- to 6-membered heterocycloalkyl, C₁-C₄ alkyl that is unsubstituted or substituted with one or more substituents selected from group C, C₃-C₇ cycloalkyl that is unsubstituted or substituted with halogen, and C₁-C₄ alkoxy that is unsubstituted or substituted with halogen; the group C substituents include D, halogen, C₃-C₆ cycloalkyl, hydroxyl, 4- to 6-membered heterocycloalkyl, and C₁-C₄ alkoxy.

In some embodiments, R⁵ is independently H, D, C₁-C₃ alkyl, or C₃-C₆ cycloalkyl at each occurrence; alternatively, R⁵ is independently H, D, C₁-C₃ alkyl, or C₃-C₆ cycloalkyl at each occurrence, the C₁-C₃ alkyl and C₃-C₆ cycloalkyl are unsubstituted or substituted with hydroxyl.

In some embodiments, R¹ is selected from -OR^{a} and NHR^{a}.

In some embodiments, R¹ is selected from C₂-C₆ alkyl, -OR^{a}, and NHR^{a}.

In some embodiments, R^{a} is selected from C₃-C₆ cycloalkyl, and C₂-C₆ alkyl that is unsubstituted or substituted with one or more substituents selected from group A; the group A substituents include D, halogen, methoxy, hydroxyl, and C₃-C₆ cycloalkyl.

In some embodiments, R² and R³ are independently selected from: 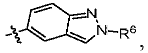 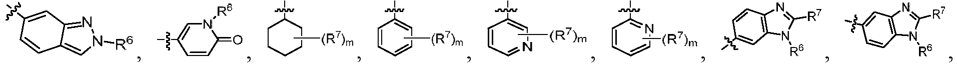 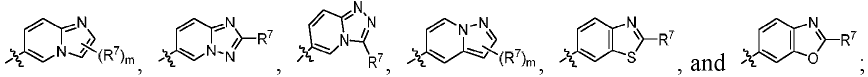
alternatively, R² and R³ are independently selected from: 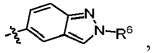 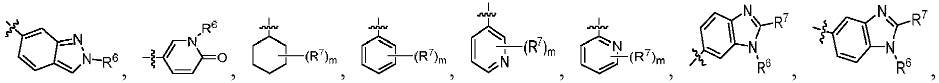 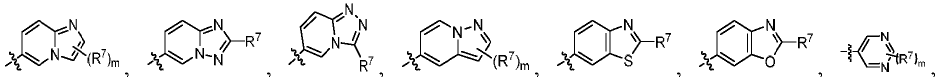 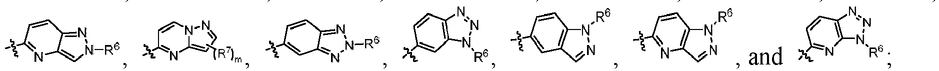
wherein (R⁷)ₘ represents m identical or different R₇ substituents on the ring where it is located; m is 1, 2 or 3; R⁶ and each R⁷ are each independently selected from H, halogen, cyano, hydroxyl, mercapto, nitro, amino, 4- to 6-membered heterocycloalkyl, C₁-C₄ alkyl that is unsubstituted or substituted with one or more substituents selected from group C, C₃-C₇ cycloalkyl that is unsubstituted or substituted with halogen, and C₁-C₄ alkoxy that is unsubstituted or substituted with halogen; the group C substituents include D, halogen, C₃-C₆ cycloalkyl, hydroxyl, 4- to 6-membered heterocycloalkyl, and C₁-C₄ alkoxy; preferably, m is 1 or 2; R⁶ and each R⁷ are each independently selected from H, halogen, cyano, hydroxyl, mercapto, nitro, amino, 4- to 6-membered heterocycloalkyl, C₁-C₂ alkyl that is unsubstituted or substituted with one or more substituents selected from group C, cyclopropyl that is unsubstituted or substituted with halogen, and C₁-C₂ alkoxy that is unsubstituted or substituted with halogen; the group C substituents include D, halogen, C₃-C₆ cycloalkyl, hydroxyl, 4- to 6-membered heterocycloalkyl, and C₁-C₄ alkoxy.

In some embodiments, R⁵ is independently H or methyl at each occurrence.

In some embodiments, R⁵ is independently H, methyl, or hydroxyethyl at each occurrence.

In some embodiments, R¹ is selected from -OR^{a} and NHR^{a}; R^{a} is selected from C₃-C₆ cycloalkyl, unsubstituted C₂-C₄ alkyl, C₂-C₄ alkyl substituted with halogen, C₂-C₄ alkyl substituted with cyclopropyl, C₂-C₄ alkyl substituted with methoxy, and C₂-C₄ alkyl substituted with hydroxyl.

In some embodiments, R¹ is selected from C₂-C₆ alkyl, -OR^{a}, -SR^{a}, and NHR^{a}; R^{a} is selected from C₃-C₆ cycloalkyl, unsubstituted C₂-C₄ alkyl, C₂-C₄ alkyl substituted with halogen, C₂-C₄ alkyl substituted with cyclopropyl, C₂-C₄ alkyl substituted with methoxy, and C₂-C₄ alkyl substituted with hydroxyl.

In some embodiments, R² and R³ are independently selected from:

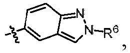

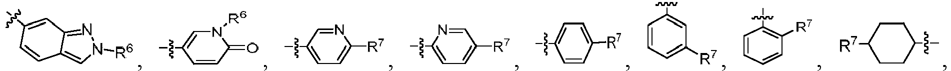

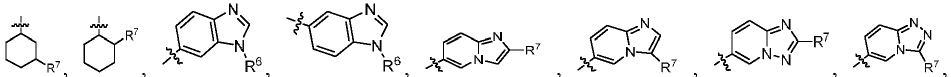

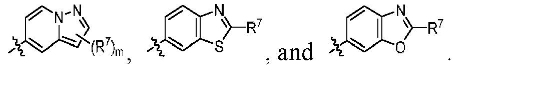

In some embodiments, R² and R³ are each independently selected from:

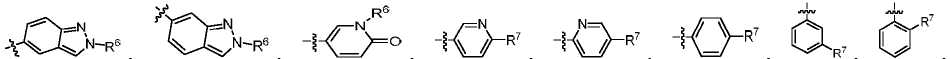

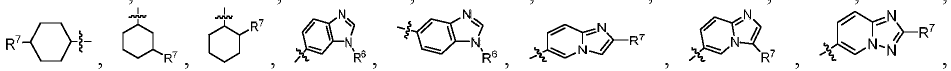

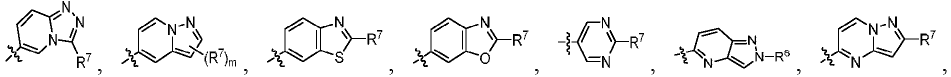

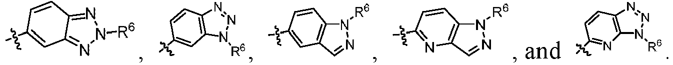

In some embodiments, m is 1, 2, or 3; R⁶ and R⁷ are each independently selected from H, halogen, cyano, hydroxyl, mercapto, nitro, amino, 4- to 6-membered heterocycloalkyl, C₁-C₄ alkyl that is unsubstituted or substituted with one or more substituents selected from group C, C₃-C₇ cycloalkyl that is unsubstituted or substituted with halogen, and C₁-C₄ alkoxy that is unsubstituted or substituted with halogen; the group C substituents include D, halogen, C₃-C₆ cycloalkyl, hydroxyl, 4- to 6-membered heterocycloalkyl, and C₁-C₄ alkoxy.

In some embodiments, m is 1, 2, or 3; R⁶ and R⁷ are each independently selected from H, halogen, cyano, hydroxyl, mercapto, nitro, amino, 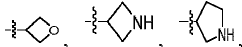 and 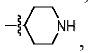 C₁-C₃ alkyl that is unsubstituted or substituted with one or more substituents selected from group C, cyclopropyl that is unsubstituted or substituted with halogen, and C₁-C₂ alkoxy that is unsubstituted or substituted with halogen; the group C substituents include D, halogen, C₃-C₆ cycloalkyl, hydroxyl, methoxy, 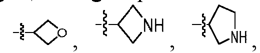 and

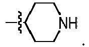

In some embodiments, R¹ is selected from -OC₂H₅, -OCH₂CF₃, -NHCH₃, -NHC₂H₅,

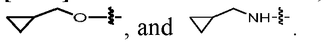

In some embodiments, R¹ is selected from propyl, -OC₂H₅, -SC₂H₅, -OCH₂CF₃, -NHCH₃, -NHC₂H₅,

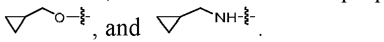

In some embodiments, R² is selected from 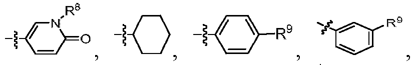 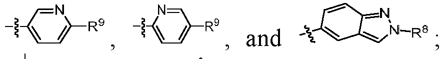 alternatively, R² is selected from 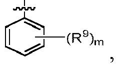 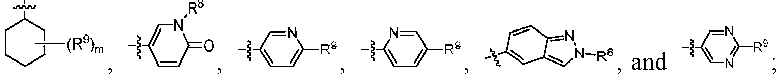 wherein m is 1, 2, or 3; R⁸ and R⁹ are each independently selected from H, halogen, CN, methoxy, methyl substituted with one or more halogens, methoxy substituted with one or more halogens. and cyclopropyl; preferably, R² is selected from 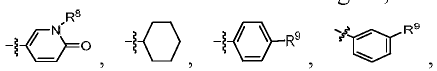 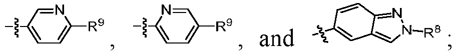 alternatively, R² is selected from 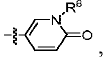 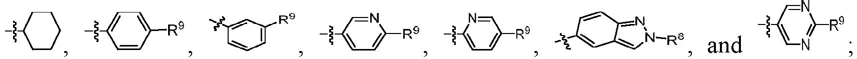 wherein R⁸ and R⁹ are each independently selected from H, halogen, cyano, methoxy, methyl substituted with one or more halogens, methoxy substituted with one or more halogens, and cyclopropyl; and

R³ is selected from 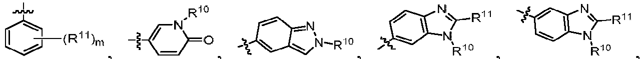 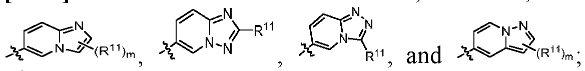 alternatively, R³ is selected from 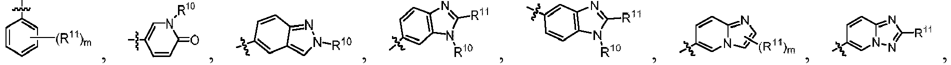 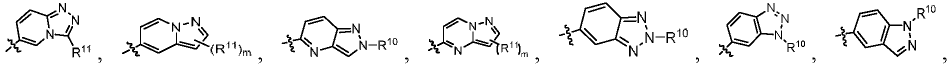 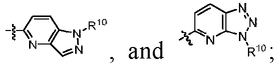 wherein m is 1, 2, or 3; R¹⁰ and R¹¹ are each independently selected from hydrogen, methyl, ethyl, isopropyl, cyano, methoxy, ethyl substituted with hydroxyl, ethyl substituted with methoxy, cyclopropyl,

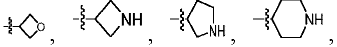

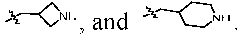

In some embodiments, the compound of formula (I) is selected from the following structures: 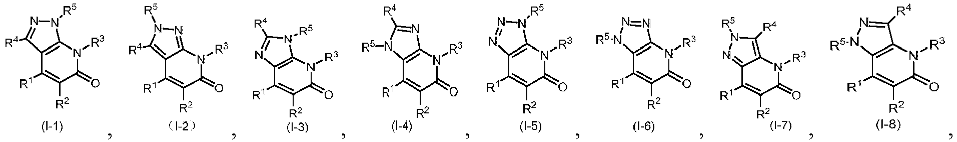 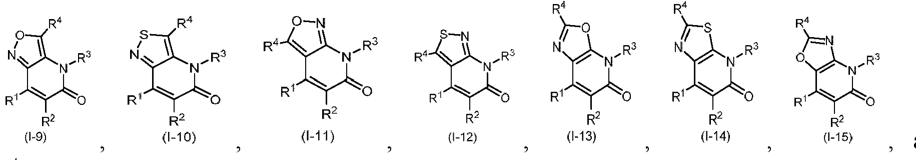 and 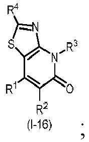 wherein R¹, R², R³, R⁴ and R⁵ are as defined above.

In some embodiments, the compound of formula (I) is selected from the following structures: 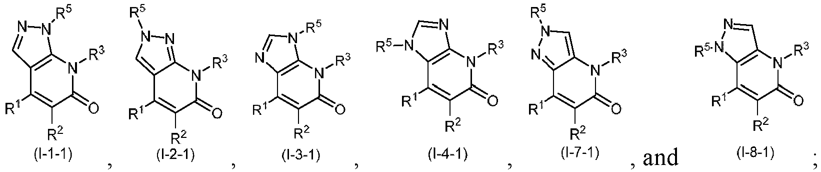 wherein R¹, R², R³ and R⁵ are as defined above.

In some embodiments, the compound represented by formula (I) is selected from the following structures:

| No. | Structure | No. | Structure | No. | Structure |
|---|---|---|---|---|---|
| 2 | 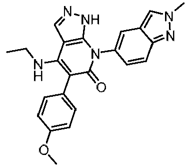 | 3 | 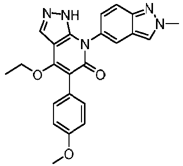 | 4 | 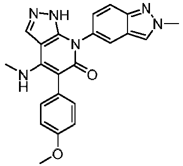 |
| 5 | 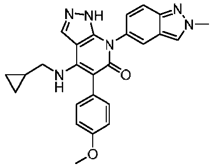 | 6 | 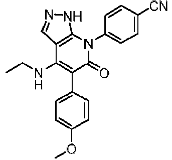 | 7 | 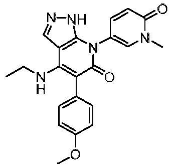 |
| 8 | 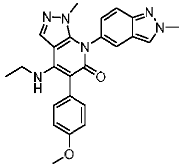 | 9 | 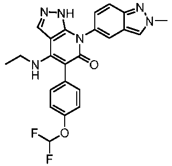 | 10 | 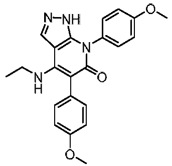 |
| 11 | 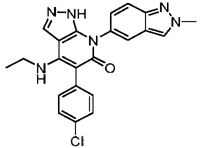 | 12 | 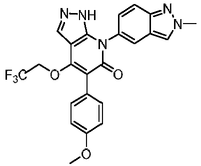 | 13 | 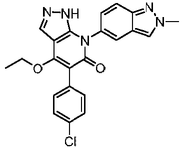 |
| 14 | 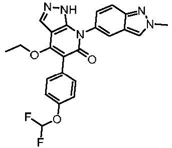 | 15 | 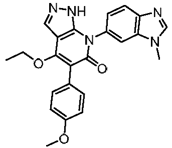 | 16 | 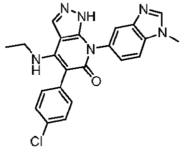 |
| 17 | 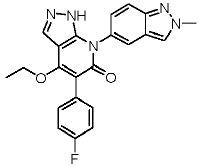 | 18 | 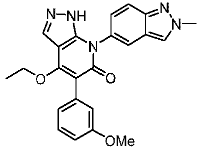 | 19 | 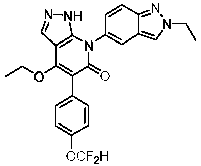 |
| 20 | 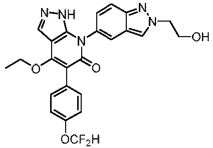 | 21 | 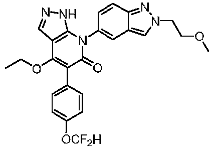 | 22 | 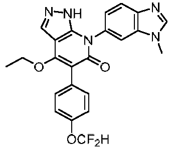 |
| 23 | 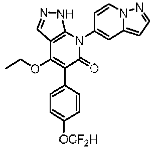 | 24 | 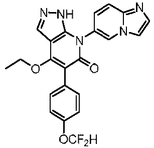 | 25 | 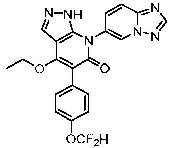 |
| 26 | 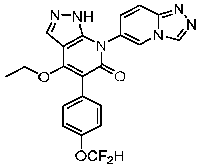 | 27 | 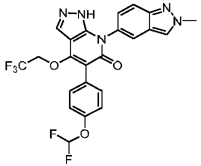 | 28 | 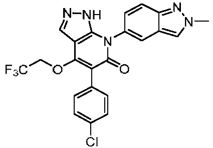 |
| 29 | 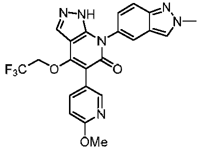 | 30 | 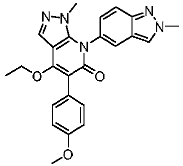 | 31 | 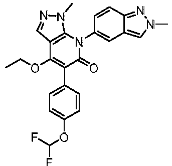 |
| 32 | 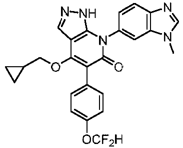 | 33 | 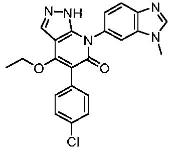 | 34 | 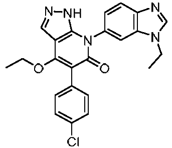 |
| 35 | 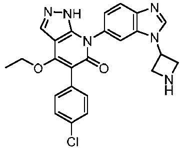 | 36 | 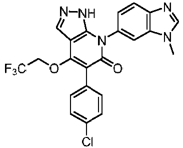 | 37 | 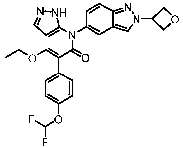 |
| 38 | 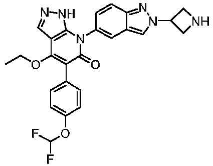 | 39 | 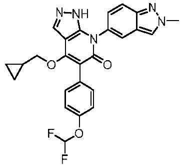 | 40 | 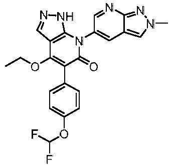 |
| 41 | 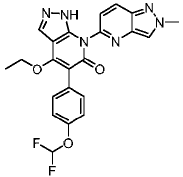 | 42 | 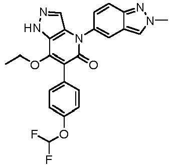 | 43 | 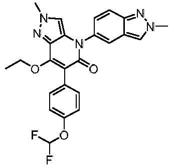 |
| 44 | 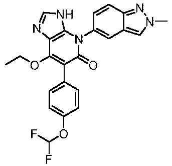 | 45 | 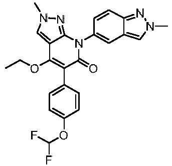 | 46 | 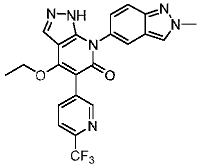 |
| 47 | 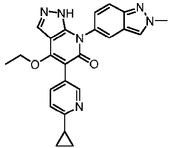 | 48 | 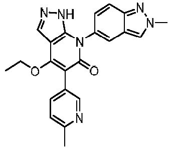 | 49 | 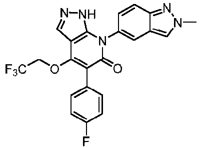 |
| 50 | 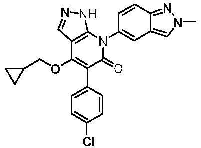 | 51 | 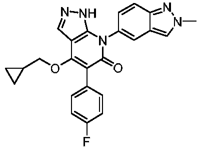 | 52 | 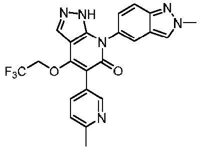 |
| 53 | 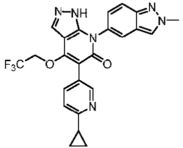 | 54 | 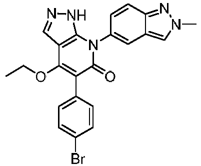 | 55 | 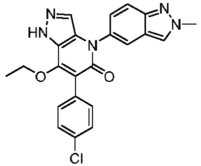 |
| 56 | 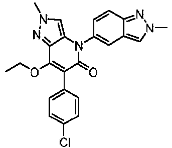 | 57 | 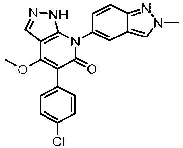 | 58 | 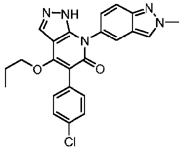 |
| 59 | 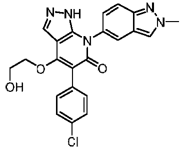 | 60 | 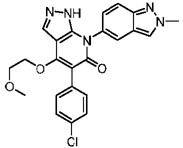 | 61 | 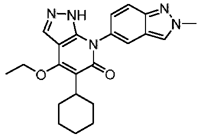 |
| 62 | 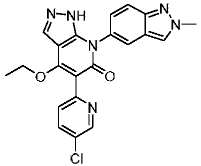 | 63 | 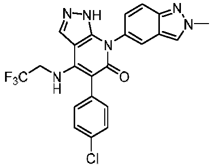 | 64 | 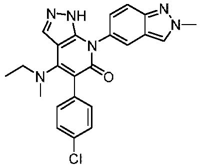 |
| 65 | 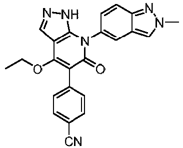 | 66 | 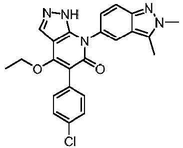 | 67 | 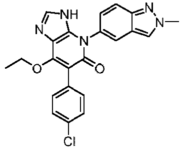 |
| 68 | 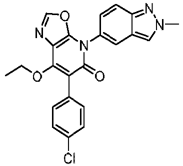 | 69 | 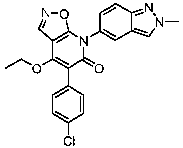 | 70 | 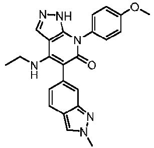 |
| 71 | 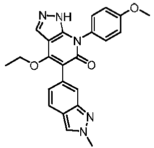 | 72 | 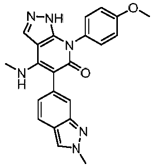 | 73 | 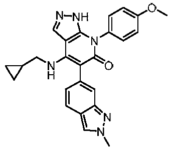 |
| 74 | 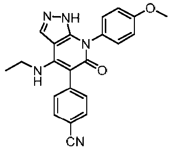 | 75 | 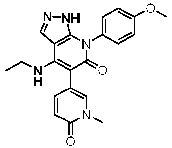 | 84 | 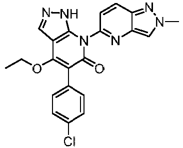 |
| 86 | 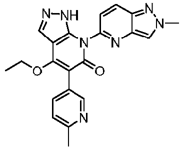 | 87 | 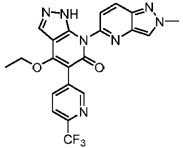 | 89 | 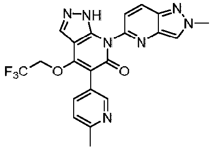 |
| 90 | 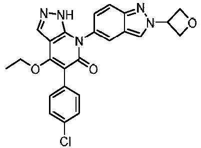 | 92 | 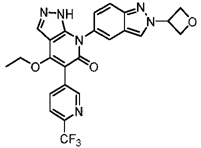 | 93 | 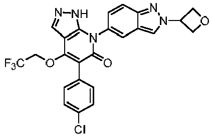 |
| 96 | 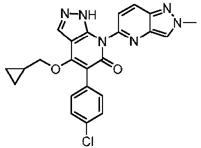 | 99 | 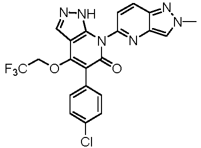 | 100 | 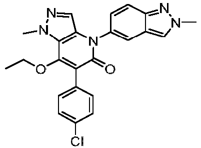 |
| 101 | 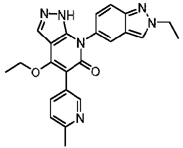 | 102 | 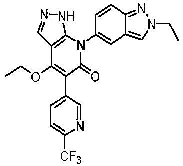 | 104 | 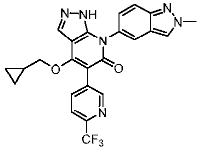 |
| 105 | 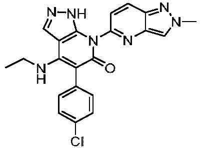 | 106 | 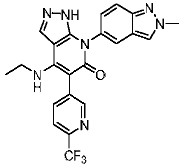 | 107 | 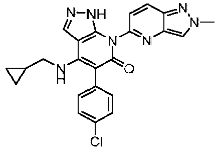 |
| 108 | 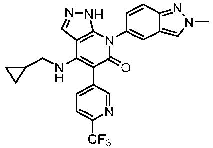 | 109 | 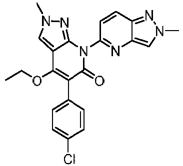 | 111 | 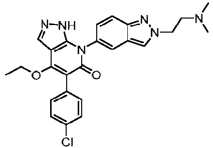 |
| 112 | 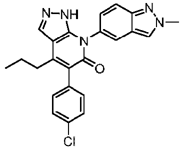 | 114 | 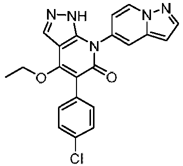 | 116 | 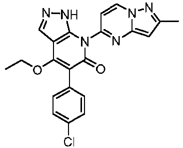 |
| 117 | 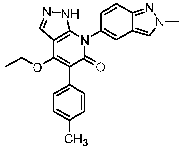 | 118 | 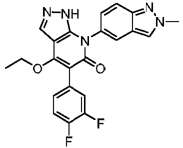 | 121 | 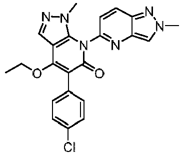 |
| 122 | 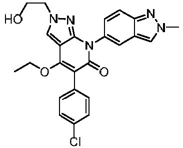 | 123 | 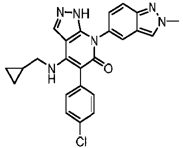 | 124 | 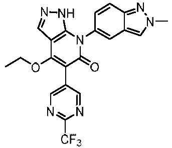 |
| 125 | 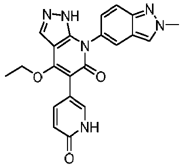 | 126 | 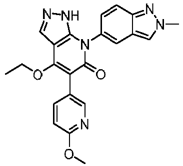 | 127 | 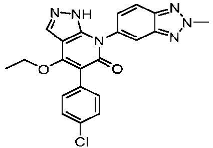 |
| 128 | 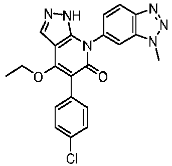 | 129 | 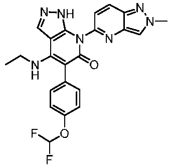 | 130 | 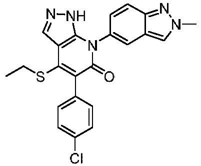 |
| 131 | 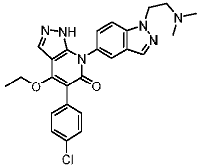 | 132 | 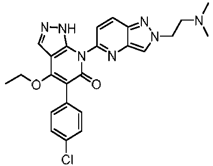 | 133 | 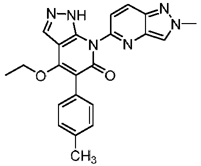 |
| 134 | 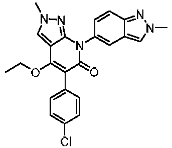 | 136 | 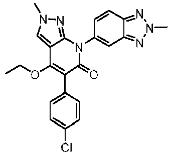 | 137 | 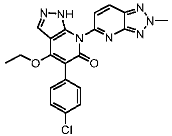 |
| 138 | 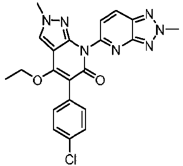 | 139 | 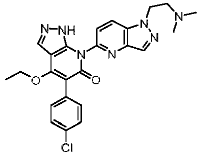 | 140 | 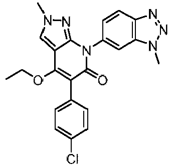 |
| 141 | 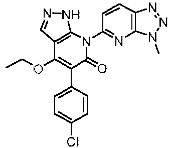 | 142 | 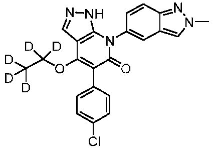 | | |

A second aspect of the present invention provides a pharmaceutical composition, which comprises a therapeutically effective dose of one or more selected from the compound represented by formula (I), a pharmaceutically acceptable salt, an enantiomer, a diastereomer, a racemate, an atropisomer, a polymorph, a solvate, or an isotope labeled compound thereof, and at least one pharmaceutically acceptable carrier. The pharmaceutical composition may also optionally comprise one or more other therapeutic agents.

In an embodiment of the present invention, the present invention provides a combination, especially a drug combination, which comprises a therapeutically effective dose of one or more selected from the compound represented by formula (I), a pharmaceutically acceptable salt, an enantiomer, a diastereomer, a racemate, an atropisomer, a polymorph, a solvate, or an isotope labeled compound thereof, and one or more other therapeutic agents.

The compound of the present invention may be used alone, in combination with other compounds of the present invention, or in combination with one or more, preferably one or two other therapeutic agents simultaneously or sequentially.

A third aspect of the present invention provides a use of the compound represented by formula (I) or a pharmaceutically acceptable salt, an enantiomer, a diastereomer, a racemate, an atropisomer, a polymorph, a solvate, or an isotope labeled compound thereof, or the pharmaceutical composition, in preparing drugs for inhibiting MAT2A activity.

A fourth aspect of the present invention provides a use of one or more of the compound represented by formula (I) or a pharmaceutically acceptable salt, an enantiomer, a diastereomer, a racemate, an atropisomer, a polymorph, a solvate, or an isotope labeled compound thereof, or the pharmaceutical composition, in preparing drugs for treating and/or preventing MTAP-related diseases, especially tumors.

Preferably, the tumors include MTAP deleted tumors; tumors with low expression of MTAP; tumors with abnormal expression of MAT2A; and other MAT2A dependent tumors.

Specifically, the tumors include breast cancer, lung cancer, glioblastoma, brain cancer and spinal cancer, head and neck cancer, skin cancer, reproductive system cancer, gastrointestinal system cancer, esophageal cancer, nasopharyngeal cancer, pancreatic cancer, rectal cancer, hepatocellular carcinoma, cholangiocarcinoma, gallbladder cancer, colon cancer, multiple myeloma, kidney and bladder cancer, bone cancer, malignant mesothelioma, sarcoma, lymphoma, adenocarcinoma, thyroid cancer, heart tumor, germ cell tumor, malignant neuroendocrine tumor, malignant rhabdoid tumor, soft tissue sarcoma, midline tract cancer and an unknown primary cancer.

In an embodiment of the present invention, the present invention provides a method for treating or preventing MAT2A related diseases, especially tumors, which includes administering an effective amount of one or more selected from the compound of the present invention, a pharmaceutically acceptable salt, an enantiomer, a diastereomer, a racemate, an atropisomer, a polymorph, a solvate, or an isotope labeled compound thereof, or the pharmaceutical composition of the present invention, to an individual in need. In some embodiments, the method further includes administering an effective amount of a second therapeutic agent to an individual in need, wherein the second therapeutic agent is one or more other therapeutic agents.

In addition, the present invention provides a product or kit, which comprises one or more selected from the compound of the present invention as defined above, a pharmaceutically acceptable salt, an enantiomer, a diastereomer, a racemate, an atropisomer, a polymorph, a solvate, or an isotope labeled compound thereof, or the pharmaceutical composition of the present invention. The kit may also comprise one or more other therapeutic agents to form a combination formulation for simultaneous, separate, or sequential use in anti-cancer therapy.

### DETAILED DESCRIPTION OF EMBODIMENTS

### Terminology

In the present invention, unless otherwise explicitly stated, the terms used in the present invention have the meanings defined below. Terms not explicitly defined in the present invention have general meanings that are generally understood by those skilled in the art.

The singular terms used in the context of the present invention (especially in the context of the claims) should be construed to cover the plural meanings unless otherwise indicated herein or clearly contradicted by the context.

A dash ("-") that is not between two letters or symbols is used to indicate a connecting site for a substituent. For example, -O(C₁-C₃ alkyl) indicates that the group is connected to the rest of the molecule through an oxygen atom. However, when the connecting site of the substituent is obvious to those skilled in the art, for example, for substituents such as halogen and hydroxyl, the "-" can be omitted.

As used herein, "heteroatoms" refer to nitrogen (N), oxygen (O), or sulfur (S) atoms, particularly nitrogen or oxygen atoms, each of which can be substituted or unsubstituted, including their oxidized forms. Examples of heteroatoms include but are not limited to -O-, -N=, -NR-, -S-, -S(O)- and -S(O)₂-, wherein R is hydrogen, C₁-C₄ alkyl or nitrogen protective groups (for example, benzyloxycarbonyl, p-methoxybenzylcarbonyl, tert-butoxycarbonyl, acetyl, benzoyl, benzyl, p-methoxy-benzyl, p-methoxy-benzyl, 3,4-dimethoxybenzyl, etc.). Any heteroatom with an unsatisfied valence bond is considered to have a hydrogen atom sufficient to satisfy the valence bond, unless otherwise indicated.

As used herein, "halogen" or "halogenated" refers to fluorine, chlorine, bromine and iodine. The preferred halogen as a substituent is fluorine and chlorine.

As used herein, "alkyl" refers to a monovalent hydrocarbon group of completely saturated straight or branched chains. Alkyl preferably contains 1-20 carbon atoms, more preferably 1-16 carbon atoms, 1-10 carbon atoms, 1-8 carbon atoms, 1-6 carbon atoms, 1-4 carbon atoms or 1-3 carbon atoms. The number before alkyl represents the number of carbon atoms. For example, "C₁-C₆ alkyl" represents an alkyl with 1-6 carbon atoms, "C₁-C₄ alkyl" represents an alkyl with 1-4 carbon atoms, and "C₁-C₃ alkyl" represents an alkyl with 1-3 carbon atoms, and so on. Representative examples of alkyl include but are not limited to methyl, ethyl, n-propyl, isopropyl, n-butyl, sec-butyl, isobutyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, n-decyl, etc. Whether the term "alkyl" appears alone or as part of other functional groups such as haloalkyl, alkoxy, etc., this definition applies.

As used herein, "alkenyl" refers to a monovalent hydrocarbon group of straight or branched chains containing at least one double bond. Alkenyl preferably contains 2-20 carbon atoms, more preferably 2-10 carbon atoms, 2-8 carbon atoms, 2-6 carbon atoms or 2-4 carbon atoms. The number before alkenyl represents the number of carbon atoms. Representative examples of alkenyl include but are not limited to vinyl, propylene, isopropenyl, butene, isobutenyl, pentenyl, isopentenyl, hexenyl, heptenenyl, octenyl, etc.

As used herein, "alkynyl" refers to a monovalent hydrocarbon group of straight or branched chains containing at least one triple bond. Alkynyl preferably contains 2-20 carbon atoms, more preferably 2-10 carbon atoms, 2-8 carbon atoms, 2-6 carbon atoms or 2-4 carbon atoms. The number before alkynyl represents the number of carbon atoms. Representative examples of alkynyl include but are not limited to acetylene, propargyl, isopropyne, butyrgyl, isobutyrgyl, pentyne, isopentyne, hexyne, heptanyne, octyne, etc.

As used herein, "alkoxy" refers to an alkyl, i.e. an alkyl-O group, connected by an oxygen bridge as defined herein. The number before alkoxy represents the number of carbon atoms. For example, "C₁-C₆ alkoxy" represents an alkoxy with 1-6 carbon atoms, i.e. -O-C₁₋₆ alkyl; "C₁-C₄ alkoxy" refers to an alkoxy with 1-4 carbon atoms, i.e. -O-C₁₋₄ alkyl; and "C₁-C₃ alkoxy" refers to an alkoxy with 1-3 carbon atoms, i.e. -O-C₁₋₃ alkyl. Representative examples of alkoxy include but are not limited to methoxy, ethoxy, propanoxy, 2-propanoxy, butoxy, tert butoxy, pentoxy, hexoxy, etc.

As used herein, "cycloalkyl" refers to saturated or partially saturated non-aromatic carbon rings, including monocyclo, bicyclo, or tricyclo, preferably with 3-12 ring carbon atoms, more preferably 3-10 ring carbon atoms, for example, 3-8, 3-7, 3-6, 4-10 or 4-8 ring carbon atoms. "C₃-C₈ cycloalkyl" is intended to include C₃, C₄, C₅, C₆, C₇ and C₈ cycloalkyl groups; "C₃-C₆ cycloalkyl" is intended to include C₃, C₄, C₅ and C₆ cycloalkyl groups. Exemplary monocyclic cycloalkyls include but are not limited to cyclopropyl, cyclobutyl, cyclopentyl, cyclopentenyl, cyclohexyl and cyclohexene. Exemplary bicyclic cycloalkyls include bornyl, tetrahydronaphthyl, decahydronaphthyl, bicyclo[2.1.1]hexyl, bicyclo[2.2.1]heptyl, bicyclo[2.2.1]heptyl, 6,6-dimethyldicyclo[3.1.1]heptyl, 2,6,6-trimethyldicyclo[3.1.1]heptyl, bicyclo[2.2.2]octyl, etc. Exemplary tricyclic cycloalkyls include diamond alkyl, etc.

As used herein, "haloalkyl" refers to an alkyl as defined herein where one or more hydrogen atoms, for example, 1, 2, 3, 4, 5, 6 or 7 hydrogen atoms, for example, 1, 2 or 3 hydrogen atoms, are replaced by a halogen, and when more than one hydrogen atom is replaced by a halogen atom, the halogen atom can be the same or different from each other. For example, "C₁-C₄ haloalkyl" is intended to include C₁, C₂, C₃ and C₄ haloalkyl groups, and "C₁-C₃ haloalkyl" is intended to include C₁, C₂ and C₃ haloalkyl groups. Examples of haloalkyl include but are not limited to fluoromethyl, difluoromethyl, trifluoromethyl, trichloromethyl, 1,1-difluoroethyl, 1,1-difluoropropyl and 1,1,1-trifluoropropyl. Examples of haloalkyl also include "fluoroalkyl", which is intended to include alkyl as defined herein where one or more hydrogen atoms are substituted by fluorine atoms. The "haloalkyl" herein is preferred that at most three hydrogen atoms in the alkyl are replaced by halogens.

As used herein, "haloalkoxy" refers to a haloalkyl as defined above with a specified number of carbon atoms connected by an oxygen bridge, where one or more hydrogen atoms, for example, 1, 2, 3, 4, 5, 6 or 7 hydrogen atoms, for example, 1, 2 or 3 hydrogen atoms, are replaced by halogens. For example, "C1-C6 haloalkoxy" or "C1 to C6 haloalkoxy" is intended to include C₁, C₂, C₃, C₄, C₅ and C₆ haloalkoxy groups. Examples of haloalkoxy include but are not limited to fluoromethoxy, difluoromethoxy, trifluoromethoxy, 2-fluoroethoxy, and 2,2,2-trifluoroethoxy. Examples of haloalkoxy also include "fluoroalkoxy".

As used herein, "aryl" consists of one or more rings fused together, with 6-20, preferably 6-14, more preferably 6-12, and most preferably 6-10. An aryl with 6-10 cyclic carbon atoms, namely C₆-C₁₀ aryl, which includes: monocyclic aryl (such as phenyl); or a fused double ring system, where one ring is an aromatic ring and the other ring is an aromatic ring (such as in naphthalene or biphenyl) or a non-aromatic ring (such as in dihydroindene or tetrahydronaphthalene). Non-limiting examples of aryl include phenyl, biphenyl, naphthyl, tetrahydronaphthyl, indenyl, dihydroindenyl or anthryl.

As used herein, "heteroaryl" refers to a 5- to 14-membered, preferably 5- to 10-membered, more preferably 5- to 7-membered, or 5- to 6-membered aromatic ring system containing 1-4, preferably 1-3 ring heteroatoms selected from N, O or S, including a single ring, a double ring, or a fused multi ring, with the remaining ring atoms being carbon atoms. Examples of heteroaryl include but are not limited to pyrrolyl, furanyl, thienyl, pyrazolyl, imidazolyl, triazolyl, thiazolyl, isothiazolyl, oxazolyl, pyridyl, pyranyl, pyrazinyl, pyridazinyl, pyrimidinyl, oxazinyl, oxadiazinyl, quinolinyl, isoquinolinyl, cinnolinyl, quinazolinyl, quinoxalinyl, benzoxazinyl, 2H-chromone, benzopyranyl, benzothienyl, indolyl, indazolyl, benzopyrazole, benzoimidazolyl, imidazopyridinyl, benzooxazolyl, benzothiazolyl, 7-azaindolyl, 6-azaindolyl, 5-azaindolyl, 4-azaindolyl, 1H-benzo[d][1,2,3]triazolyl, [1,2,4]triazolo[1,5-a]pyridinyl, [1,2,4]triazolo[4,3-a]pyridine, and pyrazolo[1,5-a]pyridine, etc.

As used herein, "heterocycloalkyl" refers to a group obtained by replacing one or more cyclic carbons of a cycloalkyl as defined in the present application with heteroatoms selected from N, O or S, the heteroatoms are, for example, -O-, -N=, -NR-, -S-, -S(O)- and -S(O)₂-, wherein R is hydrogen, C₁₋₄ alkyl or nitrogen protective groups (for example, benzyloxycarbonyl, p-methoxybenzylcarbonyl, tert-butoxycarbonyl, acetyl, benzoyl, benzyl, p-methoxy-benzyl, p-methoxy-benzyl, 3,4-dimethoxybenzyl, etc.). Preferably, heterocycloalkyl is saturated and partially unsaturated non-aromatic rings of a single, two, or three ring with 3-20 ring atoms, such as 3-12 ring atoms, such as 3-8 ring atoms, such as 3-6 ring atoms. More preferably, heterocycloalkyl preferably contains 4- to 12-membered heterocycloalkyl containing 1, 2, or 3 heteroatoms selected from N, O and S, preferably 4- to 8-membered heterocycloalkyl, more preferably 4- to 7-membered, 4- to 6-membered, or 5- to 6-membered heterocycloalkyl, wherein the heteroatoms are substituted or unsubstituted, such as being substituted by C₁-C₄ alkyl. For example, examples of heterocycloalkyl include but are not limited to oxiranyl, aziridinyl, azetidinyl, oxetanyl, azolidinyl (pyrrolidinyl), tetrahydrofuryl, tetrahydrothienyl, tetrahydrothienyl 1,1-dioxide, pyrazolidinyl, imidazolidinyl, oxazolidinyl, thiazolidinyl, isothiazolidinyl, pyrrolidyl-2-one, imidazolonyl, piperidyl (hexahydropyridine), N-methylpiperidyl, tetrahydropyranyl, oxazinanyl, 1,3-oxazinanyl, hexahydropyrimidinyl, piperazinyl, piperidinylone, 1,4-dioxa-8-aza-spiro[4.5]decane-8-yl, morpholino, thiomorpholino, sulfanomorpholino, sulfonomorpholino, octahydropyrrolo[3,2-b]pyrrolyl, etc.

As used herein, the term "oxo" refers to an oxygen atom connected to other atoms through a double bond, which can be expressed as "=O". The term "-C(=O)" refers to carbonyl, "- S(=O)" refers to sulfoxide, and "- S(=O)₂" refers to sulfonyl.

The term "optionally" used herein refers to the events described subsequently that may or may not occur, and this description includes both the situations in which the event occurred and the situations in which it did not occur. For example, "optionally substituted alkyl" includes "unsubstituted alkyl" and "substituted alkyl" as defined herein. "Optionally substituted by halogens" includes situations where "substituted by halogens" and situations where "not substituted by halogens", such as being substituted by 0-3 halogens. Those skilled in the art should understand that for any functional group containing one or more substituents, the group does not include any spatially impractical, chemically incorrect, synthetic infeasible, and/or inherently unstable substitution patterns.

The term "substituted" used herein refers to one or more hydrogen atoms on a given atom or group being replaced by one or more substituents selected from a given substituent group, provided that they do not exceed the normal valence of the given atom. When the substituent is an oxo (i.e. =O), the two hydrogen atoms on a single atom are replaced by oxygen. There are no oxygen substituents present on the aromatic portion. When a ring system (such as a carbon ring or heterocycle) is replaced by a carbonyl group or double bond, it is intended that the carbonyl group or double bond is part of the ring (i.e., within the ring). Only when the combination of substituents and/or variables leads to chemically correct and stable compounds, such combinations are allowed. A chemically correct and stable compound means that it is sufficiently stable to be separated from the reaction mixture and its chemical structure can be determined, and subsequently formulated into a preparation with at least practical utility. For example, in the absence of a clear list of substituents, the terms "substituted" used herein refer to one or more hydrogen atoms on a given atom or group being independently replaced by one or more substituents, such as 1, 2, 3 or 4 substituents. When an atom or group is replaced by multiple substituents, the substituents can be the same or different.

Unless otherwise specified, the terms "the compound of the present invention" refer to a compound comprising one or more of the formula (I) or its subforms defined herein, such as formulas (I-1), (I-2), (I-1-1), (I-2-1), (I-3-1), etc., or their pharmaceutically acceptable salts, and all isomers such as stereoisomers (including diastereoisomers, enantiomers and racemes), geometric isomers, conformational isomers (including rotamers and atropisomers), tautomers, internal addition products of isomers, prodrugs, and isomer labeled compounds (including deuterium substitution) and naturally formed parts (such as polymorphic forms, solvates, and/or hydrates). When there is a part that can form salt, it also includes salt, particularly a pharmaceutically acceptable salt. The presence of internal addition products of tautomers or isomers can be identified by those skilled in the art using tools such as NMR. The compound of formula (I) of the present invention can easily form internal addition products of tautomers and isomers as described herein.

Those skilled in the art will recognize that the compound of the present invention may contain chiral centers, allowing for the existence of different isomeric forms. As used herein, "isomers" refer to different compounds with the same molecular formula but different atomic arrangements and configurations.

As used herein, "enantiomers" are a pair of stereoisomers that are non-overlapping mirrors of each other. A 1: 1 mixture of a pair of stereoisomers is a "racemic" mixture. When appropriate, this term is used to refer to racemic mixtures. When indicating the stereochemistry of the compound of the present invention, a conventional RS system is used to specify a known relative and absolute configuration of a single stereoisomer with two chiral centers (e.g. (1S, 2S)); single stereoisomers with known relative configurations but unknown absolute configurations are marked with asterisks (e.g. (1R*, 2R*); a racemic mixture with two letters (e.g. (1RS, 2RS) is a racemic mixture of (1R, 2R) and (1S, 2S); (1RS, 2SR) is a racemic mixture of (1R, 2S) and (1S, 2R)). "Diastereomers" are stereoisomers that have at least two asymmetric atoms but are not mirror images of each other. According to the Cahn-lngold-Prelog R-S system, absolute stereochemistry is indicated. When a compound is a pure enantiomer, the stereochemistry at each chiral carbon can be explained by R or S. The split compounds with unknown absolute configurations can be specified as (+) or (-) based on the direction of polarized light (right rotation or left rotation) of rotation of plane at the sodium D-line wavelength. Alternatively, the split compounds can be defined by the respective retention times of enantiomers/diastereomers via chiral HPLC.

Some of the compounds described herein contain one or more asymmetric centers or axes, thus producing enantiomers, diastereomers, and other stereoisomers that can be defined as (R)- or (S)- in absolute stereochemistry.

When a compound contains a double bond or some other characteristic that gives the molecule a certain amount of structural rigidity, a geometric isomer can occur. If a compound contains a double bond, the substituent can be in the E or Z conformation. If a compound contains a disubstituted cycloalkyl, the cycloalkyl substituent can have a cis-configuration or trans-configuration.

A conformational isomer is an isomer that differs by the rotation of one or more valence bonds. A rotamer is a conformational isomer that differs by the rotation of only a single valence bond.

"Atropisomers" refers to a structural isomer based on axial or planar chirality generated by rotational confinement within a molecule.

Unless otherwise stated, the compound of the present invention is intended to include all such possible isomers, including racemic mixtures, optically active forms, and intermediate mixtures. (R)- and (S)- isomers with optical activity can be prepared using chiral synthons or chiral reagents, or split using conventional techniques.

The compound of the present invention can be separated into optically active or racemic forms. The compound in the optically active form can be split through racemic separation or synthesis from materials with optical activity. All methods for preparing the compound of the present invention and the intermediates prepared therein are considered to be part of the present invention. When preparing enantiomer or diastereomer products, they can be separated by conventional methods such as chromatography or stepwise crystallization.

According to the method conditions, the end product of the present invention is obtained in a free (neutral) form or a salt form. The free and salt forms of these end products are within the scope of the present invention. If desired, one form of the compound can be transformed into another form. Free bases or acids can be converted into salts; salts can be converted into free compounds or other salts; the mixture of isomeric compounds of the present invention can be separated into individual isomers.

As used herein, "pharmaceutically acceptable salts" refer to salts that maintain the biological effects and properties of the compound of the present invention, and such salts are not unexpected in terms of biology or other aspects. Non-limiting examples of the salt include non-toxic, inorganic or organic base or acid addition salts of the compound of the present invention. In many cases, the compound of the present invention can form acid salts and/or base salts due to the presence of amino and/or carboxyl or similar groups. Inorganic and organic acids can be used to form pharmaceutically acceptable acid addition salts. Inorganic acids from which salts can be derived include, for example, hydrochloric acid, hydrobromic acid, sulfuric acid, nitric acid, phosphoric acid, etc. Organic acids from which salts can be derived include, for example, acetic acid, propionic acid, hydroxyacetic acid, pyruvate, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, etc. Inorganic and organic bases can be used to form pharmaceutically acceptable base addition salts. Inorganic bases from which salts can be derived include, for example, sodium, potassium, lithium, ammonium, calcium, magnesium, iron, zinc, copper, manganese, aluminum, etc; particularly preferred are ammonium, potassium, sodium, calcium and magnesium salts. Organic bases from which salts can be derived include, for example, primary amines, secondary amines and tertiary amines, substituted amines (including naturally occurring substituted amines), cyclic amines, alkaline ion exchange resins, etc., especially such as isopropylamine, trimethylamine, diethylamine, triethylamine, tripropylamine and ethanolamine. By conventional chemical methods, the pharmaceutically acceptable salt of the present invention can be synthesized from the parent compound (alkaline portion or acidic portion). Generally speaking, the salt can be prepared by reacting the free acid form of the compound with a stoichiometric quantity of an appropriate base (such as hydroxide of Na, Ca, Mg or K, carbonate, bicarbonate, etc.) or by reacting the free base form of the compound with a stoichiometric quantity of an appropriate acid. This type of reaction is usually carried out in water, organic solvents or a mixture of both. Generally speaking, non-aqueous media such as ether, ethyl acetate, ethanol, isopropanol or acetonitrile are preferred when feasible. Other suitable salts can be found in Remington's Pharmaceutical Sciences, 20th edition, Mack Publishing Company, East, Pa., (1985), which was introduced herein as a reference.

As used herein, "pharmaceutically acceptable excipients" include any and all solvents, dispersants, coatings, surfactants, antioxidants, preservatives (such as antibacterial agents, antifungal agents), isotopes, absorption retardants, salts, preservatives, drugs, drug stabilizers, adhesives, excipients, disintegrants, lubricants, sweeteners, correctors, dyes, similar substances and their combinations, which is well-known to those skilled in the art (see, for example, Remington's Pharmaceutical Sciences, 18th edition, Mack Printing Company, 1990, pp 1289-1329, which was introduced herein as a reference). Unless any conventional carrier cannot coexist with the active ingredient, it can be considered for use in therapeutic or pharmaceutical compositions.

The arbitrary formula given herein is also intended to represent the unlabeled form of the compound and the isotopic labeled form. Isotopic labeled compounds have the structure described in the formula provided herein, except for one or more atoms replaced by atoms with the selected atomic mass or mass number. Examples of isotopes that can be incorporated into the compound of the present invention include isotopes of hydrogen, carbon, nitrogen, oxygen, phosphorus, fluorine and chlorine, such as ²H (i.e. D), ³H (i.e. T), ¹¹C, ¹³C, ¹⁴C, ¹⁵N, ¹⁸F, ³¹P, ³²P, ³⁵S, ³⁶Cl and ¹²⁵I, respectively. The present invention includes different isotopic labeled compounds as defined herein, such as those containing radioactive isotopes such as ³H, ¹³C and ¹⁴C. These isotopic labeled compounds can be used for metabolic studies (using ¹⁴C), reaction kinetics studies (such as using ²H or ³H), detection or imaging techniques, such as positron emission tomography (PET) or single photon emission computed tomography (SPECT), including drug or substrate tissue distribution measurements, or can be used for individual radiation therapy. Particularly, ¹⁸F or labeled compounds can be particularly desired for use in PET or SPECT studies. Typically, isotopic labeled compounds of the present invention can be prepared by using readily available isotope labeled reagents instead of non-isotope labeled reagents, as described in the following processes or examples and preparation examples.

Moreover, being replaced by heavier isotopes, particularly deuterium (i.e. ²H or D), can also yield certain therapeutic benefits due to greater metabolic stability, such as prolonging in vivo half-life, reducing dose requirements, or improving treatment indices. It can be understood that deuterium in the context can be regarded as a substituent of the compound of the present invention. The concentration of these heavier isotopes, particularly deuterium, can be defined by isotopic enrichment factors. "Isotope enrichment factor" represents the ratio between the isotopic abundance of a specified isotope and its natural abundance.

As used herein, the "therapeutically effective amount" of the compound of the present invention refers to the amount of the compound of the present invention that can cause individual biological or medical reactions, improve symptoms, slow or delay disease progression, or prevent disease. The "therapeutically effective amount" can be determined by participating physicians or veterinary practitioners, and will vary depending on factors such as the compound, the disease status being treated, the severity of the disease being treated, the individual's age and relevant health status, the route and form of administration, and the judgment of the attending physician or veterinary practitioner.

As used herein, "individual" refers to animals. Preferably, animals are mammals. Individuals also refer to primates (for example, humans), cows, sheep, goats, horses, dogs, cats, rabbits, rats, mice, fish, birds, etc. In a preferred embodiment, the individual is a human.

As used herein, "inhibition" refers to the alleviation or inhibition of a specific patient, symptom or disease, or a significant decrease in biological activity or process baseline activity.

As used herein, the term "treatment" or any disease or condition in an embodiment refers to the improvement of diseases or conditions (i.e., preventing or slowing down the development of the disease or at least one clinical symptom). In another embodiment, "treatment" refers to improving at least one physical parameter that may not be perceived by the patient. In another embodiment, "treatment" refers to the regulation of diseases or conditions on the body (such as stable and perceptible symptoms) or physiology (such as stable body parameters) or both.

As used herein, "prevention" refers to the administration of one or more pharmaceutical substances, particularly the compound and/or the pharmaceutically acceptable salt thereof of the present invention, to individuals with a predisposition to the disease, in order to prevent them from developing the disease.

Generally speaking, the term "about" is used herein to adjust the given value to be 20% higher or lower than that value, such as 10%, such as 5%.

The undefined technical and scientific terms used herein have the meanings commonly understood by those skilled in the art to which the present invention belongs.

### Examples

The following examples illustrate the present invention, however, they do not limit the scope of the present invention in any way. The beneficial effects of the combination of the present invention can also be determined by other test models known to those skilled in the art.

In the present invention, the source and trade name of the reagents and equipment used are all indicated at the time of first appearance. If there are no special instructions, the same reagents used thereafter are the same as those first indicated. Conventional unmarked reagents are purchased from Sinopharm Chemical Reagent Co., Ltd.

### Abbreviation meaning:

DIPEA: N,N-diisopropylethylamine; SEMCl: 2-(trimethylsilyl)ethoxymethyl chloride; TBAF: tetrabutylammonium fluoride; NBS: N-bromosuccinimide; Tf: trifluoromethanesulfonyl; FA: formic acid; BSA: bovine serum albumin; Brij 35: polyoxyethylene lauryl ether; Pd(dppf)Cl₂: [1,1 ' -bis(diphenylphosphino)ferrocene]dichloropalladium; NBS: N-bromosuccinimide; PE: petroleum ether; EA: ethyl acetate.

### Example 1: Synthesis of compound 1

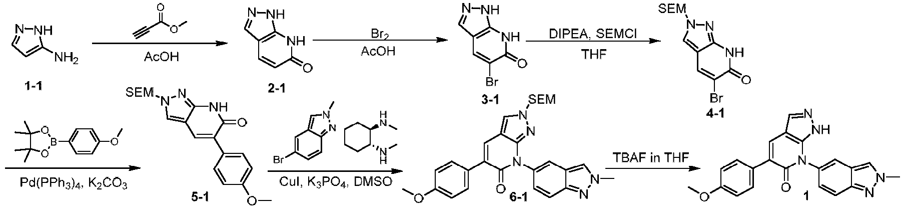

### Step 1: Synthesis of intermediate 2-1

5-aminopyrazole 1-1 (8.3 g, 100 mmol) was dissolved in glacial acetic acid (AcOH) (80 mL) in a 100 mL single-necked flask, methyl propargonate (8.4 g, 100 mmol) was added while stirring at room temperature, and performed a reflux reaction overnight. The reaction solution was concentrated to dryness, added with EA (150 ml), stirred for 1.5 h, and filtered to obtain an intermediate 2-1 (4.5 g, yield: 33.3%) as a yellow solid. LCMS (ESI) m/z = 136.2 [M+H]⁺.

### Step 2: Synthesis of intermediate 3-1

The intermediate **2-1** (3.8 g, 28.1 mmol) was dissolved in acetic acid (72.0 mL) in a 100 mL single-necked flask, and liquid bromine (18.0 g, 112.5 mmol) was added while stirring at room temperature, and the reaction was completed after reacting for 3 hours. The reaction solution was filtered, and a filter cake was collected and rinsed twice with PE to obtain an intermediate **3-1** (4.0 g, yield: 66.7%) as a pale yellow solid. ¹H NMR (400 MHz, DMSO) δ 12.14 (s, 1H), 9.82 (s, 2H), 8.29 (s, 1H), 8.03 (s, 1H).

### Step 3: Synthesis of intermediate 4-1

The intermediate **3-1** (2.14 g, 100 mmol) and DIPEA (2.84 g, 220 mmol) were dissolved in tetrahydrofuran (THF) (50 mL) in a 100 mL single-necked flask, and SEMCl (2.0 g, 120 mmol) was added dropwise while stirring at room temperature and reacted for 16 h. The reaction solution was poured into water and extracted with EA (100 mL). The organic layer was combined, dried with anhydrous sodium sulfate, and filtered. The filtrate was concentrated and purified by a silica gel rapid column (0%-30% PE/EA) to obtain an intermediate **4-1** (0.8 g, yield: 23.3%) as a waxy solid. LCMS (ESI) m/z = 346.0 [M+H]⁺.

### Step 4: Synthesis of intermediate 5-1

The intermediate **4-1** (0.8 g, 2.32 mmol), 4-methoxyphenylboronic acid pinacol ester (1.085 g, 4.64 mmol), Pd(PPh₃)₄ (536 mg, 0.464 mmol), potassium carbonate (640 mg, 4.64 mmol), 1,4-dioxane (25 ml), and water (5 ml) were sequentially added into a 50 mL single-necked flask and stirred at 110°C under N₂ protection for 16 h. The reaction solution was filtered, and the filtrate was concentrated and purified by a silica gel rapid column (0%-50% PE/EA) to obtain 800 mg of a intermediate **5-1** crude product as a white solid. 400 mg was taken and purified by pre-HPLC (H₂O/ACN, 0.1% formic acid) to obtain an intermediate **5-1** (50 mg, yield: 11.6%). LCMS (ESI) m/z = 372.1 [M+H]⁺.

### Step 5: Synthesis of intermediate 6-1

The intermediate **5-1** (40 mg, 0.10 mmol), 5-bromo-2-methyl-2H-indazole (44 mg, 0.2 mmol), trans N,N'-dimethylcyclohexanediamine (3 mg, 0.02 mmol), cuprous iodide (4 mg, 0.02 mmol), potassium phosphate (44 mg, 0.2 mmol), and dimethyl sulfoxide (DMSO) (5 ml) were sequentially added into a 50 mL single-necked flask and stirred at 110°C under nitrogen protection for 16 h. The reaction solution was concentrated to dryness and purified by a silica gel rapid column (0%-3% methanol (MEOH)/dichloromethane (DCM)) to obtain an intermediate **6-1** (21 mg, yield: 38.9%) as a white solid. LCMS (ESI) m/z = 502.4 [M+H]⁺.

### Step 6: Synthesis of compound 1

The intermediate **6-1** (21 mg, 0.057 mmol) and TBAF tetrahydrofuran solution (5 ml, 5 mmol) were sequentially added into a 25 mL single-necked flask, and stirred at 60°C under N₂ protection for 16 h. The reaction solution was concentrated to dryness and prepared by pre-TLC (DCM/THF=1/1) to obtain a compound 1 (3 mg, yield: 19.4%) as a white solid. ¹H NMR (400 MHz, DMSO) δ 12.07 (s, 1H), 8.77 (s, 1H), 8.46 (s, 1H), 8.14(s, 1H), 7.85 (s, 1H), 7.81 (d, *J =* 9.4 Hz, 1H), 7.75 (d, *J =* 9.1 Hz, 1H), 7.60(d, *J =* 8.1 Hz, 2H), 6.97 (d, *J =* 7.8 Hz, 2H), 4.20 (s, 3H), 3.79 (s, 3H). LCMS (ESI) m/z = 372.2 [M+H]⁺.

### Example 2: Synthesis of compound 2

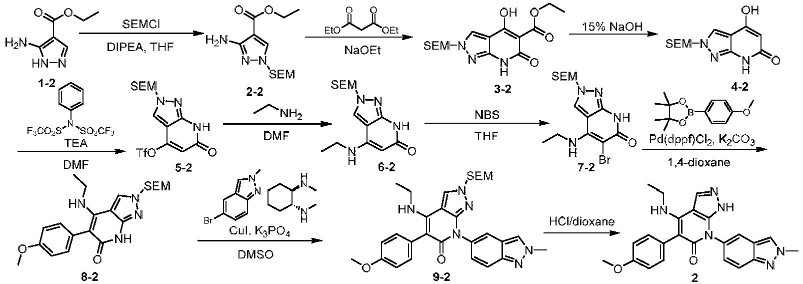

### Step 1: Synthesis of intermediate 2-2

3-amino-4-ethoxycarbonylpyrazole (40 g, 258 mmol) was dissolved in THF (500 mL) in a 100 mL single-necked flask, cooled to 0°C, and added with DIPEA (40 g, 310 mmol), stirred for 15 min. SEMCl (51.5 g, 310 mmol) was added dropwise to the reaction solution, and stirred at room temperature for 12 h after dropwise adding completion. The reaction solution was poured into 1 L of ice water mixture and extracted with EA. The organic phase was combined, washed sequentially with water and saturated saline solution once (200 mL/time), dried with anhydrous sodium sulfate, and filtered. The filtrate was concentrated and purified by a silica gel rapid column (EA/PE=1/2) to obtain an intermediate **2-2** (55 g, yield: 74.8%) as a pale yellow oily liquid. LCMS (ESI) m/z = 286.2 [M+H]⁺.

### Step 2: Synthesis of intermediate 3-2

The intermediate **2-2** (54 g, 189.5 mmol) was dissolved in ethanol (250 mL) in a 500 mL single-necked flask, and diethyl malonate (113.6 g, 709.5 mmol) was added while stirring at room temperature. After addition, the temperature was lowered to 0°C. An ethanol solution of 20% sodium ethoxide (NaOEt) (192.9 g, 567 mmol) was slowly added dropwise, heated up to 95°C under N₂ protection after dropwise adding completion, and stirred for 18 hours. The reaction solution was concentrated under reduced pressure to dryness, added with 200 mL of ice water, and extracted with methanol/DCM (1/10). The organic phase was combined, washed sequentially with water and saturated saline solution, dried with anhydrous sodium sulfate, and filtered. The filtrate was concentrated to dryness, and purified by a silica gel column (DCM/MeOH=30/1) to obtain an intermediate **3-2** (50 g, yield: 75.9%) as a pale yellow solid. LCMS (ESI) m/z = 354.2 [M+H]⁺.

### Step 3: Synthesis of intermediate 4-2

The intermediate **3-2** (50 g, 141 mmol) was dissolved in a 15% sodium hydroxide aqueous solution (350 mL) in a 500 mL single-necked flask, heated up to 105°C, and reacted for 15 hours. The reaction solution was reduced to 0°C in an ice water bath. The pH was adjusted to around 5 with 4 M dilute hydrochloric acid. The reaction solution was filtered to obtain a white solid, and purified by a silica gel rapid column (DCM/MeOH=10:1) to obtain an intermediate **4-2** (30.8 g, yield: 77.4%) as a white solid. LCMS (ESI) m/z = 282.1 [M+H]⁺.

### Step 4: Synthesis of intermediate 5-2

The intermediate **4-2** (30.8 g, 109.5 mmol) was dissolved in dimethylformamide (DMF) (200 mL) in a 100 mL single-necked flask, followed by the addition of triethylamine (TEA) (33.2 g, 328.5 mmol). Then, N-phenyl bis(trifluoromethylsulfonyl imide) (46.9 g, 131.4 mmol) was added in batches and reacted at room temperature for 16 hours. The reaction solution was poured into 1 L of ice water, and extracted with EA. The organic phase was combined, washed once with water and once with saturated saline solution (100 mL/time), dried with anhydrous sodium sulfate, and filtered. The filtrate was concentrated under reduced pressure and purified by a silica gel rapid column (PE/EA=2:1) to obtain an intermediate **5-2** (10.1 g, yield: 22.3%) as a white solid. ¹H NMR (400 MHz, DMSO) δ 12.41 (s, 1H), 8.60 (s, 1H), 6.41 (s, 1H), 5.55 (s, 2H), 3.66 - 3.55 (m, 2H), 0.93 - 0.84 (m, 2H), 0.01 - 0.02 (m, 9H).

### Step 5: Synthesis of intermediate 6-2

The intermediate **5-2** (5.0 g, 12.1 mmol) was dissolved in DMF (25 mL) in a 25 mL single-necked flask, and added with TEA (1.35 g, 13.3 mmol). Then, ethylamine tetrahydrofuran solution (13.3 ml, 26.6 mmol, 2M) was slowly added dropwise, and stirred overnight at room temperature after dropwise adding completion. The reaction solution was poured into 250 mL of ice water, and extracted with EA. The organic phase was combined, washed once with water and once with saturated saline solution (50 mL/time), dried with anhydrous sodium sulfate, and filtered. The filtrate was concentrated under reduced pressure and purified by a silica gel rapid column (DCM/MeOH=40/1) to obtain an intermediate **6-2** (1.1 g, yield: 29.5%) as a white solid. ¹H NMR (400 MHz, DMSO) δ 10.63 (s, 1H), 8.21 (s, 1H), 6.90 (t, *J =* 5.2 Hz, 1H), 5.43 (d, *J =* 4.0 Hz, 2H), 4.88 (d, *J =* 5.6 Hz, 1H), 3.57 (t, *J =* 8.2 Hz, 2H), 3.15 (d, *J =* 4.4 Hz, 2H), 1.24 (t, *J =* 7.2 Hz, 3H), 0.89 (t, *J =* 8.0 Hz, 2H), 0.02 (s, 9H).

### Step 6: Synthesis of intermediate 7-2

The intermediate **6-2** (1.1 g, 3.57 mmol) was dissolved in THF (15 mL) in a 25 mL single-necked flask, added with NBS (634 mg, 3.57 mmol), and reacted at room temperature for 2 h. The reaction solution was poured into ice water, and extracted with DCM/methanol (10/1). The organic layer was combined, washed once with water and once with saturated saline solution (20 mL/time), dried with anhydrous sodium sulfate, and filtered. The filtrate was concentrated under reduced pressure to obtain a crude product, which was purified by a silica gel rapid column (DCM/MeOH=25/1) to obtain an intermediate **7-2** (1.1 g, yield: 79.6%) as a white solid. ¹H NMR (400 MHz, DMSO) δ 11.31 (s, 1H), 8.62 (s, 1H), 6.36 (t, *J* = 6.0 Hz, 1H), 5.48 (s, 2H), 3.67 - 3.55 (m, 4H), 1.27 (t, *J =* 7.2 Hz, 3H), 0.90 (t, *J =* 8.0 Hz, 2H), 0.01 (s, 9H).

### Step 7: Synthesis of intermediate 8-2

The intermediate **7-2** (1.0 g, 2.58 mmol), 4-methoxyphenylboronic acid pinacol ester (1.2 g, 5.16 mmol), Pd(dppf)Cl₂ (209 mg, 0.258 mmol), potassium carbonate (712 mg, 5.16 mmol), 1,4-dioxane (25 ml), and water (5 ml) were sequentially added into a 50 mL single-necked flask, heated up to 100°C under N₂ protection, and stirred for 16 h. The reaction solution was concentrated to dryness, and the crude product was purified by a silica gel rapid column (DCM/methanol=30/1) to obtain an intermediate **8-2** (550 mg, yield: 51.4%) as a white solid. ¹H NMR (400 MHz, DMSO) δ 10.90 (s, 1H), 8.49 (s, 1H), 7.12 (d, *J =* 8.4 Hz, 2H), 6.98 (d, *J =* 8.4 Hz, 2H), 5.45 (s, 2H), 5.21 (t, *J =* 6.0 Hz, 1H), 3.80 (s, 3H), 3.60 (dd, *J =* 10.8, 5.3 Hz, 2H), 3.33- 3.28 (m, 2H), 1.07 (t, *J =* 7.2 Hz, 3H), 0.92 - 0.87 (m, 2H), 0.01 (s, 9H).

### Step 8: Synthesis of intermediate 9-2

The intermediate **8-2** (200 mg, 0.48 mmol), 5-bromo-2-methyl-2H-indazole (204 mg, 0.96 mmol), trans N,N'-dimethylcyclohexanediamine (7 mg, 0.048 mmol), cuprous iodide (9 mg, 0.048 mmol), potassium phosphate (204.5 mg, 1 mmol), DMSO (25 ml) were sequentially added into a 50 mL single-necked flask, heated up to 130°C under N₂ protection, and stirred for 16 h. The reaction solution was poured into ice water, and extracted with DCM/methanol (10/1). The organic layer was combined, washed once with water and once with saturated saline solution (25 mL/time), dried with anhydrous sodium sulfate, and filtered. The filtrate was concentrated to obtain a crude product, which was purified by a silica gel rapid column (DCM/MeOH=30/1) to obtain an intermediate **9-2** (100 mg, yield: 38.3%) as a white solid. LCMS (ESI) m/z = 545.2 [M+H]⁺.

### Step 9: Synthesis of compound 2

The intermediate 9-2 (100 mg, 1.83 mmol) was dissolved in 4 M HCl/1,4-dioxane solution (15 mL) in a 25 mL single-necked flask, and reacted at room temperature for 2 h. The reaction solution was poured into ice water, and the pH was adjusted to neutral with sodium bicarbonate aqueous solution. The solution was extracted with DCM/methanol (10/1). The organic phase was combined, washed once with water and once with saturated saline solution, dried with anhydrous sodium sulfate, and filtered. The filtrate was concentrated under reduced pressure, purified by prep-HPLC (H₂O/CAN, 0.1% FA), and freeze dried to obtain a compound 2 (31.77 mg, yield: 41.8%) as a white solid. ¹H NMR (400 MHz, DMSO) δ 13.03 (s, 1H), 8.35 (s, 2H), 7.60 (d, J = 13.2Hz, 2H), 7.16 (d, J = 8.8 Hz, 2H), 7.07 (dd, J = 9.2, 2.0 Hz, 1H), 6.95 (d, J = 8.8 Hz, 2H), 5.27 (s, 1H), 4.19 (s, 3H), 3.77 (s, 3H), 3.36 (m, 2H), 1.09 (t, J = 7.2 Hz, 3H). LCMS (ESI) m/z = 415.2 [M+H]⁺.

Compounds **5, 6, 7, 9, 10, 11 and 16** were prepared using the synthesis method of Example **2.**

| Compound | LCMS (ESI) *m*/*z* | ¹H NMR (400 MHz) |
|---|---|---|
| **5** | 441.2 [M+H]⁺ | (DMSO) δ 13.04 (s, 1H), 8.40 (s, 1H), 8.35 (s, 1H), 7.77 - 7.53 (m, 2H), 7.19 (d, *J =* 8.4 Hz, 2H), 7.07 (dd, *J*₁= 8.8 Hz, *J*₂= 2.0 Hz, 1H), 7.00 - 6.94(m, 2H), 5.15 (s, 1H), 4.19 (s, 3H), 3.78 (s, 3H), 3.26 (t, *J* = 6.0 Hz, 2H), 1.00 (m, 1H), 0.46 - 0.37 (m, 2H), 0.19 (m, 2H). |
| **6** | 386.1 [M+H]⁺ | (DMSO) δ 13.15 (s, 1H), 8.41 (s, 1H), 7.92 (d, *J =* 8.4 Hz, 2H), 7.60 (d, *J* = 8.4 Hz, 2H), 7.15 (d, *J* = 8.8 Hz, 2H), 6.95 (d, *J* = 8.8 Hz, 2H), 5.45 (t, *J =* 6.0 Hz, 1H), 3.77 (s, 3H), 3.37 (m, 2H), 1.08 (t, *J* = 7.2 Hz, 3H). |
| **7** | 392.2 [M+H]⁺ | (DMSO) δ 13.14 (s, 1H), 8.39 (s, 1H), 7.87 (s, 1H), 7.37 (dd, *J =* 9.6, 2.8 Hz, 1H), 7.13 (d, *J =* 8.4 Hz, 2H), 6.95 (d, *J =* 8.8 Hz, 2H), 6.42 (d, *J =* 9.6 Hz, 1H), 5.36 (m, 1H), 3.78 (s, 3H), 3.44 (s, 3H), 3.36 (m, 2H), 1.06 (t, *J =* 7.2 Hz, 3H). |
| **9** | 451.2 [M+H]⁺ | (DMSO) δ 13.06 (s, 1H), 8.37 (d, *J =* 16.8 Hz, 2H), 7.60 (d, *J* = 15.2 Hz, 2H), 7.30 (d, *J =* 8.0 Hz, 2H), 7.08,7.27,7.45(t, 1H), 7.18 (d, *J=* 8.4 Hz,2H), 7.07 (d, *J =* 8.8 Hz, 1H), 5.50 (m, 1H), 4.19 (s, 3H), 3.34 (m,2H), 1.09 (t, *J =* 6.8 Hz, 3H). |
| **10** | 391.0 [M+H]⁺ | (DMSO) δ 13.03 (s, 1H), 8.35 (s, 1H), 7.24-7.10 (m, 4H), 7.04-6.89 (m, 4H), 5.25 (s, 1H), 3.79 (s, 3H), 3.77 (s, 3H), 3.36 (m, 2H), 1.07 (t, *J =* 7.2 Hz, 3H). |
| **11** | 419.0 [M+H]⁺ | (DMSO) δ 13.04 (s, 1H), 8.36 (s, 2H), 7.70 -7.54 (m, 2H), 7.42 (d, *J* = 8.4 Hz, 2H), 7.27 (d, *J =* 8.4 Hz, 2H), 7.07 (dd, *J*₁= 9.2 Hz, *J*₂= 2.0 Hz, 1H), 5.62 (s, 1H), 4.19 (s, 3H), 3.36 (m, 2H), 1.10 (t, *J =* 7.2 Hz, 3H). |
| **16** | 419.1 [M+H]⁺ | (DMSO) δ 13.06 (s, 1H), 8.39 (s, 1H), 8.23 (s, 1H), 7.65 - 7.56 (m, 1H), 7.53 (d, *J =* 9.2 Hz, 1H), 7.45-7.35 (m, 2H), 7.31-7.25 (m, 2H), 7.16 (d, *J* = 8.0Hz, 1H), 5.60 (m, 1H), 3.88 (s, 3H), 3.34 (m, 2H), 1.10 (t, *J =* 7.2 Hz, 3H). |

### Example 3: Synthesis of compound 4

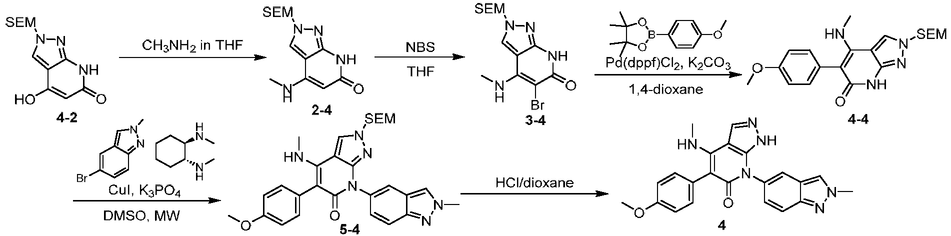

### Step 1: Synthesis of intermediate 2-4

The intermediate **4-2** (2.5 g, 8.9 mmol) was dissolved in a THF solution of methylamine (25 mL, 2 M) in a 25 mL high-pressure autoclave, heated up to 100°C, and reacted overnight. The reaction solution was concentrated directly to obtain a crude product, which was purified by a silica gel rapid column (0%-2% methanol/DCM) to obtain an intermediate **2-4** (1.0 g, yield: 38.3%) as a white solid. ¹H NMR (400 MHz, DMSO) δ 10.64 (s, 1H), 8.14 (s, 1H), 7.05 (d, *J =* 4.8 Hz, 1H), 5.44 (s, 2H), 4.86 (s, 1H), 3.61 - 3.54 (m, 2H), 2.78 (d, *J =* 4.8 Hz, 3H), 0.93 - 0.85 (m, 2H), -0.00 (s, 9H).

### Step 2: Synthesis of intermediate 3-4

The intermediate **2-4** (1.0 g, 3.4 mmol) was dissolved in THF (15 mL) in a 25 mL single-necked flask, added with NBS (604 mg, 3.4 mmol), and stirred at room temperature for 2 h. The reaction solution was concentrated to dryness to obtain a crude product, which was purified by a silica gel rapid column (0%-3% methanol/DCM) to obtain an intermediate **3-4** (760 mg, yield: 60.3 %) as a white solid. LCMS (ESI) m/z = 373.1 [M+H]⁺.

### Step 3: Synthesis of intermediate 4-4

The intermediate **3-4** (373 mg, 1.0 mmol), 4-methoxyphenylboronic acid pinacol ester (468 mg, 2 mmol), Pd(dppf)Cl₂ (81 mg, 0.1 mmol), potassium carbonate (276 mg, 2 mmol), 1,4-dioxane (15 ml), and water (3 ml) were sequentially added into a 50 mL single-necked flask, and stirred at 90°C under N₂ protection for 16 h. The reaction solution was filtered, and the filtrate was concentrated to obtain a crude product, which was purified by a silica gel rapid column (0%-3% methanol/DCM) to obtain an intermediate **4-4** (180 mg, yield: 45%) as a white solid. LCMS (ESI) m/z = 401.2 [M+H]⁺.

### Step 4: Synthesis of intermediate 5-4

The intermediate **4-4** (80 mg, 0.2 mmol), 5-bromo-2-methyl-2H-indazole (84 mg, 0.4 mmol), trans N,N'-methylcyclohexanediamine (3 mg, 0.02 mmol), cuprous iodide (8 mg, 0.04 mmol), potassium phosphate (55 mg, 0.4 mmol), DMSO (6 ml) were sequentially added into a 10 mL microwave tube and performed a microwave reaction at 110°C under nitrogen protection for 3 h. The reaction solution was poured into water, and extracted with 50 mL of methanol/DCM (1/10). The organic layer was combined, washed with water and saturated saline solution, dried with anhydrous sodium sulfate, and filtered. The filtrate was concentrated to obtain a crude product, which was purified by a silica gel rapid column (0%-2% methanol/DCM) to obtain an intermediate **5-4** (75 mg, yield: 75.5%) as a white solid. LCMS (ESI) m/z = 531.3 [M+H]⁺.

### Step 5: Synthesis of compound 4

The intermediate **5-4** (75 mg, 0.14 mmol) was dissolved in a 4 mol/L hydrogen chloride dioxane solution (5 mL) in a 25 mL single-necked flask, and stirred at room temperature for 2 h. The reaction solution was poured into 10 mL of water, and the pH was adjusted to neutral with sodium bicarbonate aqueous solution. The solution was extracted with 50 mL of DCM/methanol (10/1). The organic layer was combined, dried with anhydrous sodium sulfate, and filtered. The filtrate was concentrated under reduced pressure to dryness, purified by prep-HPLC (water/acetonitrile, 0.1% NH₃.H₂O), and freeze dried to obtain a compound **4** (6.83 mg, yield: 12.1%) as a white solid. ¹H NMR (400 MHz, DMSO) δ 13.02 (s, 1H), 8.36 (s, 2H), 7.61 (d, *J =* 12.0 Hz,2H), 7.15 (d, *J =* 8.4 Hz, 2H), 7.06 (dd, *J*₁ = 9.2 Hz, *J*₂=2.0 Hz, 1H), 6.94(d, *J =* 8.4 Hz, 2H), 5.61 (s, 1H), 4.19 (s, 3H), 3.77 (s, 3H), 2.99 (d, *J =* 4.4Hz, 3H). LCMS (ESI) m/z = 401.1 [M+H]⁺.

### Example 4: Synthesis of compound 3

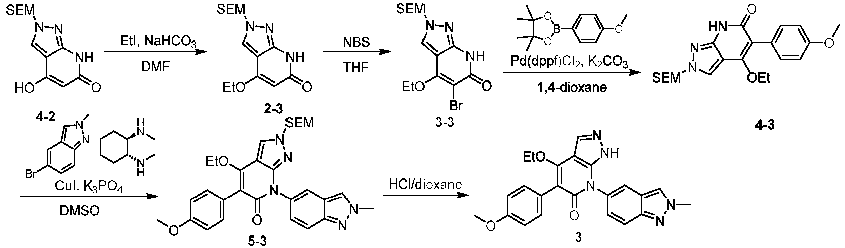

### Step 1: Synthesis of intermediate 2-3

The intermediate **4-2** (2.0 g, 7.1 mmol) was dissolved in DMF (25 mL) in a 25 mL single-necked flask, added with sodium bicarbonate (1.2 g, 14.2 mmol) and ethyl iodide (EtI) (1.66 g, 10.7 mmol), and reacted overnight at 45°C. The reaction solution was poured into ice water, and extracted with EA (100 mL). The organic layer was combined, washed once with water and once with saturated saline solution, dried with anhydrous sodium sulfate, and filtered. The filtrate was concentrated to obtain a crude product, which was purified by a silica gel rapid column (0%-80% EA/PE) to obtain an intermediate **2-3** (0.2 g, yield: 9.1%) as a white solid. ¹HNMR (400 MHz, DMSO) δ 11.37 (s, 1H), 8.30 (s, 1H), 5.52 (s, 1H), 5.44 (s, 2H), 4.17 (q, *J =* 7.2 Hz, 2H), 3.62 - 3.55 (m, 2H), 1.41 (t, *J =* 7.2 Hz, 3H), 0.91 - 0.85 (m, 2H), -0.00 (s, 9H).

### Step 2: Synthesis of intermediate 3-3

The intermediate **2-3** (0.2 g, 0.645 mmol) was dissolved in THF (15 mL) in a 25 mL single-necked flask, added with NBS (120 mg, 0.677 mmol), and stirred at room temperature for 2 h. The reaction solution was poured into water, and extracted with 50 mL of DCM/methanol (10/1). The organic layer was combined, dried with anhydrous sodium sulfate, and filtered. The filtrate was concentrated to dryness to obtain a crude product, which was purified by a silica gel rapid column (0%-4% methanol/DCM) to obtain an intermediate **3-3** (205 mg, yield: 82.3%) as a white solid. ¹HNMR (400 MHz, DMSO) δ 12.01 (s, 1H), 8.77 (s, 1H), 5.49 (s, 2H), 4.63 (q, *J =* 7.2 Hz, 2H), 3.67 - 3.59 (m, 2H), 1.44 (t, *J =* 7.2 Hz, 3H), 0.94 - 0.85 (m, 2H), 0.02 - -0.04 (m, 9H).

### Step 3: Synthesis of intermediate 4-3

The intermediate **3-3** (205 mg, 0.53 mmol), 4-methoxyphenylboronic acid pinacol ester (247 mg, 1.06 mmol), Pd(dppf)Cl₂ (43 mg, 0.053 mmol), potassium carbonate (146 mg, 1.06 mmol), 1,4-dioxane (15 ml), and water (3 ml) were sequentially added into a 50 mL single-necked flask, heated up to 100°C under N₂ protection, and stirred for 16 h. The reaction solution was filtered. The filtrate was collected, and concentrated under reduced pressure to obtain a crude product, which was purified by a silica gel rapid column (0%-2% methanol/DCM) to obtain an intermediate **4-3** (150 mg, yield: 68.5%) as a white solid. ¹H NMR (400 MHz, DMSO) 11.62 (s, 1H), 8.61 (s, 1H), 7.24 (d, *J =* 8.8Hz, 2H), 6.92 (d, *J =* 8.8 Hz, 2H), 5.47 (s, 2H), 4.32 (q, *J =* 7.2 Hz, 2H), 3.79 (s, 3H), 3.65-3.57 (m, 2H), 1.21 (t, *J* = 7.2 Hz, 3H), 0.93-0.86 (m, 2H), -0.00 (s, 9H). LCMS (ESI) m/z = 416.2 [M+H]⁺.

### Step 4: Synthesis of intermediate 5-3

The intermediate **4-3** (130 mg, 0.31 mmol), 5-bromo-2-methyl-2H-indazole (131 mg, 0.62 mmol), trans N,N'-methylcyclohexanediamine (4.4 mg, 0.03 mmol), cuprous iodide (9 mg, 0.048 mmol), potassium phosphate (131.0 mg, 0.62 mmol), DMSO (8 ml) were sequentially added into a 20 mL microwave tube and performed a microwave reaction at 110°C under nitrogen protection for 3 h. The reaction solution was poured into water, and extracted with 50 mL of methanol/DCM (1/10). The organic layer was combined, washed with water and saturated saline solution, dried with anhydrous sodium sulfate, and filtered. The filtrate was concentrated and purified by a silica gel rapid column (0%-3% methanol/DCM) to obtain an intermediate **5-3** (100 mg, yield: 58.8%) as a white solid. LCMS (ESI) m/z = 546.2 [M+H]⁺.

### Step 5: Synthesis of compound 3

The intermediate **5-3** (100 mg, 0.183 mmol) was dissolved in a 4 mol/L hydrogen chloride dioxane solution (5 mL) in a 25 mL single-necked flask, and stirred at room temperature for 2 h. The reaction solution was poured into 10 mL of water, and the pH was adjusted to neutral with sodium bicarbonate aqueous solution. The solution was extracted with 50 mL of DCM/methanol (10/1). The organic layer was combined, dried with anhydrous sodium sulfate, and filtered. The filtrate was concentrated under reduced pressure to dryness, purified by prep-HPLC (water/acetonitrile, 0.1% FA), and freeze dried to obtain a compound **3** (9.0 mg, yield: 11.84%) as a white solid. ¹H NMR (400 MHz, DMSO) δ 13.29 (s, 1H), 8.48 (s, 1H), 8.40 (s, 1H), 7.76- 7.57 (m, 2H), 7.27 (d, *J =* 8.8 Hz, 2H), 7.10 (dd, *J*₁ = 9.2 Hz, *J*₂ *=* 2.0 Hz, 1H), 6.89 (d, *J=* 8.8 Hz, 2H), 4.40 (q, *J =* 6.8Hz, 2H), 4.21 (s, 3H), 3.76 (s,3H), 1.23 (t, *J =* 7.2 Hz, 3H). LCMS (ESI) m/z = 416.1 [M+H]⁺.

Compounds **13, 14, 15, 17-26, 33-34, 37-38, 40-41, 46-48, 54, 57-58, 60, 62, 65-66, 84, 86-87, 90, 92, 101-102, 111, 114, 116-118, 124-128, 131-133, 137, 139, 141 and 142** were prepared using the synthesis method of Example 4.

| Comp -ound | LCMS (ESI) m/z | ¹H NMR (400 MHz) |
|---|---|---|
| **13** | 420.0 [M+H]⁺ | (DMSO) δ 13.38 (s, 1H), 8.57 (s, 1H), 8.39 (s, 1H), 7.89 - 7.50 (m, 2H), 7.38 (s, 4H), 7.11 (d, *J =* 7.6 Hz, 1H), 4.48 (d, *J =* 6.8 Hz, 2H), 4.21 (s, 3H), 1.25 (t, *J =* 6.8 Hz, 3H). |
| **14** | 420.0 [M+H]⁺ | (DMSO) δ 13.38 (s, 1H), 8.57 (s, 1H), 8.39 (s, 1H), 7.89 - 7.50 (m, 2H), 7.38 (s, 4H), 7.11 (d, *J =* 7.6 Hz, 1H), 4.48 (d, *J =* 6.8 Hz, 2H), 4.21 (s, 3H), 1.25 (t, *J =* 6.8 Hz, 3H). |
| **15** | 416.1 [M+H]⁺ | (DMSO) δ 13.27 (s, 1H), 8.39 (d, *J =* 76 Hz, 2H), 7.69 (d, *J =* 32 Hz, 2H), 7.30 - 7.22 (m, 2H), 7.13 (d, *J =* 8.0 Hz, 1H), 6.90 (m, 2H), 4.40 (q, *J =* 6.8 Hz, 2H), 3.84 (s, 3H), 3.76 (s, 3H), 1.24 (t, *J =* 7.2 Hz, 3H). |
| **17** | 404.1 [M+H]⁺ | (DMSO) δ 13.09 (s, 1H), 8.51 (s, 1H), 8.40 (s, 1H), 7.71 (s, 1H), 7.66 (d, *J =* 8.8 Hz, 1H), 7.38 (dd, *J*₁ = 8.8 Hz, *J*₂ = 6.0 Hz, 2H), 7.18 - 7.09 (m, 3H), 4.46 (q, *J =* 6.8Hz, 2H), 4.21 (s, 3H), 1.24 (t, *J =* 7.2 Hz, 3H). |
| **18** | 416.2 [M+H]⁺ | (DMSO) δ 13.32 (s, 1H), 8.52 (s, 1H), 8.40 (s, 1H), 7.84 - 7.51 (m, 2H), 7.24 (t, *J* = 7.6 Hz, 1H), 7.11 (d, *J* = 9.2 Hz, 1H), 6.91 (d, *J=* 7.6 Hz, 2H), 6.84 - 6.77 (m, 1H), 4.43 (dd, *J*₁= 13.6 Hz, *J*₂= 6.8 Hz, 2H), 4.21 (s, 3H), 3.74 (s, 3H), 1.24 (t, *J =* 6.8 Hz, 3H). |
| **19** | 466.1 [M+H]⁺ | (DMSO) δ 13.32 (s, 1H), 8.54 (s, 1H), 8.45 (s, 1H), 7.77 - 7.60 (m, 2H), 7.40 (d, *J =* 8.8 Hz, 2H), 7.26 (t, *J =* 76.0 Hz, 1H), 7.14 - 7.10 (m, 3H), 4.56 - 4.39 (m, 4H), 1.58 - 1.48 (m, 3H), 1.25 (t, *J =* 7.2Hz, 3H). |
| **20** | 482.15 [M+H]⁺ | (DMSO) δ 13.34 (s, 1H), 8.55 (s, 1H), 8.41 (s, 1H), 7.76-7.61 (m, 2H), 7.43-7.37 (m, 2H), 7.26 (t, *J =* 76.0 Hz, 1H), 7.14-7.10 (m, 3H), 5.01 (t, *J =* 5.4 Hz, 1H), 4.58-4.40 (m, 4H), 3.88 (q, *J* =5.4 Hz, 2H), 1.25 (t, *J =* 6.9 Hz, 3H). |
| **21** | 496.1 [M+H]⁺ | (DMSO) δ 13.33 (s, 1H), 8.54 (s, 1H), 8.42 (s, 1H), 7.67 (dd, *J*₁ = 20.8 Hz, *J*₂= 12 Hz, 2H), 7.40 (d, *J =* 8.4 Hz, 2H), 7.26 (t, *J =* 76.0 Hz,1H), 7.16-7.09 (m, 3H), 4.62 (t, *J =* 5.2 Hz, 2H), 4.47 (q, *J =* 6.8 Hz, 2H), 3.83 (dd, *J*₁ = 12.0 Hz, *J*₂ = 7.0 Hz, 2H), 3.24 (s, 3H), 1.25 (t, *J =* 6.8 Hz, 3H). |
| **22** | 452.05 [M+H]⁺ | (DMSO) δ 13.34 (s, 1H), 8.56 (s, 1H), 8.26 (s, 1H), 7.71 (d, *J =* 8.2 Hz, 1H), 7.61 (s, 1H), 7.40 (d, *J =* 8.8 Hz, 2H), 7.26 (t, *J =* 76.0 Hz, 1H), 7.13 (d, *J =* 8.6 Hz, 3H), 4.46 (q, *J =* 6.9 Hz, 2H), 3.84 (s, 3H), 1.26 (d, *J= 6.9* Hz, 3H). |
| **23** | 438.1 [M+H]⁺ | (DMSO) δ 13.45 (s, 1H), 8.78 (d, *J =* 7.4 Hz, 1H), 8.60 (s, 1H), 8.07 (d, *J =* 2.0 Hz, 1H), 7.82 (s, 1H), 7.41 (d, *J =* 8.8 Hz, 2H), 7.27 (t, *J =* 76.0 Hz 1H), 7.14 (d, *J* = 8.4 Hz, 2H), 6.88 (dd, *J*₁ = 7.2 Hz, *J*₂ = 2.0 Hz, 1H), 6.70 (s, 1H), 4.49 (q, *J =* 6.8 Hz, 2H), 1.26 (t, *J =* 6.8 Hz, 3H). |
| **24** | 438.1 [M+H]⁺ | (DMSO) δ 13.41 (s, 1H), 8.81 (s, 1H), 8.60 (s, 1H), 8.00 (s, 1H), 7.66 (d, *J =* 6.8 Hz, 2H), 7.40 (d, *J =* 8.4 Hz, 2H), 7.26 (t, *J =* 76.0 Hz, 1H), 7.23 (d, *J* = 9.2 Hz, 1H), 7.14 (d, *J =* 8.2 Hz, 2H), 4.49 (d, *J =* 6.8 Hz, 2H), 1.26 (t, *J =* 6.8 Hz, 3H). |
| **25** | 439.1 [M+H]⁺ | (DMSO) δ 13.51 (s, 1H), 9.30 (s, 1H), 8.64 (s, 1H), 8.61 (s, 1H), 7.97 (d, *J =* 9.2 Hz, 1H), 7.75 (dd, *J*₁= 9.2 Hz, *J*₂ = 2.0 Hz, 1H), 7.27 (t, *J =* 76.0 Hz ,1H), 7.44 - 7.37 (m, 2H), 7.15 (d, *J =* 8.7 Hz, 2H), 4.50 (q, *J =* 6.8 Hz, 2H), 1.27 (t, *J =* 7.2 Hz, 3H). |
| **26** | 439.10 [M+H]⁺ | (DMSO) δ 13.52 (s, 1H), 9.33 (s, 1H), 8.87 (s, 1H), 8.64 (s, 1H), 7.89 (d, *J =* 9.8 Hz, 1H), 7.44 - 7.37 (m, 3H), 7.27 (s, 1H), 7.14 (d, *J =* 8.7 Hz, 2H), 4.50 (q, *J =* 6.8 Hz, 2H), 1.26 (t, *J =* 6.9 Hz, 3H). |
| **33** | 420.00 [M+H]⁺ | (DMSO) δ 13.32 (s, 1H), 8.53 (s, 1H), 8.28 (s, 1H), 7.73 (d, *J =* 8.5 Hz, 1H), 7.63 (s, 1H), 7.38 (s, 4H), 7.14 (dd, *J*₁ = 8.5 Hz, *J*₂ = 1.8 Hz, 1H), 4.49 (q, *J =* 6.9 Hz, 2H), 3.84 (s, 3H), 1.26 (t, *J =* 7.0 Hz, 3H). |
| **34** | 434.10 [M+H]⁺ | (DMSO) δ 13.36 (s, 1H), 8.56 (s, 1H), 8.33 (s, 1H), 7.73 - 7.66 (m, 2H), 7.38 (s, 4H), 7.19 (d, *J =* 8.4 Hz, 1H), 4.48 (q, *J =* 6.8 Hz, 2H), 4.34 (q, *J =* 7.2 Hz, 2H), 1.45 (t, *J =* 7.2 Hz, 3H), 1.25 (t, *J =* 6.9 Hz, 3H). |
| **37** | 494.10 [M+H]⁺ | (DMSO) δ 13.37 (s, 1H), 8.61-8.53 (m, 2H), 7.74 (dd, *J =* 8.0, 5.2 Hz, 2H), 7.40 (d, *J =* 8.8 Hz, 2H), 7.26 (t, *J =* 76.0 Hz, 1H), 7.20-7.10 (m, 3H), 5.99-5.88 (m, 1H), 5.06 (d, *J =* 6.8 Hz, 4H), 4.47 (q, *J =* 6.9 Hz, 2H), 1.25 (t, *J =* 6.9 Hz, 3H). |
| **38** | 493.15 [M+H]⁺ | (DMSO) δ 13.38 (s, 1H), 9.25 (s, 1H), 8.93 (s, 1H), 8.58 (s, 2H), 7.76 (d, *J =* 8.8 Hz, 2H), 7.42-7.36 (m, 2H), 7.26 (s, 1H), 7.22 (dd, *J =* 9.1, 1.5 Hz, 1H), 7.13 (d, *J* = 8.6 Hz, 2H), 5.76 (p, *J* = 7.5 Hz, 1H), 4.60-4.24 (m, 6H), 1.25 (t, *J =* 6.9 Hz, 3H). |
| **40** | 452.80 [M+H]⁺ | (DMSO) δ 13.32 (s, 1H), 8.51 (s, 1H), 8.49 (s, 1H), 8.46 (d, *J =* 2.4 Hz, 1H), 8.27 (d, *J =* 2.4 Hz, 1H), 7.41 (d, *J =* 8.8 Hz, 2H), 7.26 (t, *J =* 76.0 Hz, 1H), 7.13 (d, *J =* 8.7 Hz, 2H), 4.49 (q, *J =* 7.0 Hz, 2H), 4.24 (s, 3H), 1.26 (t, *J =* 7.0 Hz, 3H). |
| **41** | 452.95 [M+H]⁺ | (DMSO) δ 13.38 (s, 1H), 8.69 (s, 1H), 8.58 (s, 1H), 8.23 (d, *J =* 8.9 Hz, 1H), 7.40 (d, *J =* 8.7 Hz, 2H), 7.27 (d, *J =* 8.9 Hz, 1H), 7.27 (t, *J =* 74.4 Hz, 1H), 7.14 (d, *J =* 8.6 Hz, 2H), 4.50 (q, *J =* 6.7 Hz, 2H), 4.26 (s, 3H), 1.26 (t, *J* = 7.0 Hz, 3H). |
| **46** | 454.85 [M+H]⁺ | (DMSO) δ 13.49 (s, 1H), 8.77 (d, J = 1.6 Hz, 1H), 8.67 (s, 1H), 8.41 (s, 1H), 8.10 (dd, J₁ = 8.2 Hz, J₂ = 1.5 Hz, 1H), 7.88 (d, J = 8.0 Hz, 1H), 7.79 - 7.57 (m, 2H), 7.13 (dd, J = 9.1, 1.8 Hz, 1H), 4.59 (t, J = 6.9 Hz, 2H), 4.21 (s, 3H), 1.30 (t, J = 6.9 Hz, 3H). |
| **47** | 426.80 [M+H]⁺ | (DMSO) δ 13.35 (s, 1H), 8.56 (s, 1H), 8.40 (s, 1H), 8.35 (d, J = 1.8 Hz, 1H), 7.78 - 7.62 (m, 2H), 7.60 (dd, J = 8.0, 2.2 Hz, 1H), 7.25 (d, J = 8.0 Hz, 1H), 7.11 (dd, J = 9.1, 1.8 Hz, 1H), 4.50 (q, J = 6.8 Hz, 2H), 4.21 (s, 3H), 2.07 (td, J = 7.7, 3.8 Hz, 1H), 1.26 (t, J = 7.0 Hz, 3H), 0.92 (t, J = 6.6 Hz, 4H). |
| **48** | 401.0 [M+H]⁺ | (DMSO) δ 13.39 (s, 1H), 8.58 (s, 1H), 8.49 - 8.35 (m, 2H), 7.70 (s, 1H), 7.64 (dd, J = 8.0, 2.4 Hz, 2H), 7.22 (d, J = 8.0 Hz, 1H), 7.11 (d, J = 8.8 Hz, 1H), 4.51 (q, J = 6.8 Hz, 2H), 4.21 (s, 3H), 2.46 (s, 3H), 1.26 (t, J = 6.8 Hz, 3H). |
| **54** | 464.05 [M+H]⁺ | (DMSO) δ 13.36 (s, 1H), 8.57 (s, 1H), 8.39 (s, 1H), 7.69 (s, 1H), 7.65 (d, J = 8.5 Hz, 1H), 7.51 (d, J =8.5 Hz, 2H), 7.32 (d, J =8.5 Hz, 2H), 7.11 (d, J = 8.3 Hz, 1H), 4.48 (d, J = 7.0 Hz, 2H), 4.21 (s, 3H), 1.25 (t, J = 6.9 Hz, 3H). |
| **57** | 406.0 [M+H]⁺ | (DMSO) δ 13.41 (s, 1H), 8.65 (s, 1H), 8.40 (s, 1H), 7.77-7.60 (m, 2H), 7.36 (q, *J* =8.8 Hz, 4H), 7.11 (d, *J =* 9.2 Hz, 1H), 4.21 (s, 3H), 4.14 (s, 3H). |
| **58** | 433.8 [M+H]⁺ | (DMSO) δ 13.37 (s, 1H), 8.57 (s, 1H), 8.40 (s, 1H), 7.70 (s, 1H), 7.65 (d, *J =* 8.8 Hz, 1H), 7.41 - 7.30 (s, 4H), 7.11 (dd, *J*₁ = 9.2 Hz, *J*₂ = 1.6 Hz, 1H), 4.39 (t, *J =* 6.0 Hz, 2H), 4.21 (s, 3H), 1.62 (dd, *J*₁ = 13.6 Hz, *J*₂ =6.4 Hz, 2H), 0.85 (t, *J =* 7.2 Hz, 3H). |
| **60** | 449.90 [M+H]⁺ | (DMSO) δ 13.35 (s, 1H), 8.55 (s, 1H), 8.40 (s, 1H), 7.71 (s, 1H), 7.66 (d, *J =* 9.0 Hz, 1H), 7.43 - 7.36 (m, 4H), 7.12 (dd, *J*₁ = 9.1 Hz, *J*₂ *=* 1.7 Hz, 1H), 4.57 - 4.51 (m, 2H), 4.21 (s, 3H), 3.59 - 3.55 (m, 2H), 3.23 (s, 3H). |
| **62** | 421.10 | (DMSO) δ 13.42 (s, 1H), 8.60 (dd, J1 = 14.9 Hz, J2 = 2.0 Hz, 2H), 8.39 |
| | [M+H]⁺ | (s, 1H), 7.89 (dd, J₁ = 8.4 Hz, J₂ = 2.5 Hz, 1H), 7.72 - 7.61 (m, 2H), 7.41 (d, J = 8.5 Hz, 1H), 7.13 - 7.07 (m, 1H), 4.42 (q, J = 6.9 Hz, 2H), 4.20 (s, 3H), 1.21 (t, J = 6.9 Hz, 3H). |
| **65** | 411.10 [M+H]⁺ | (DMSO) δ 13.44 (s, 1H), 8.62 (s, 1H), 8.40 (s, 1H), 7.78 (d, J = 8.4 Hz, 2H), 7.74 - 7.61 (m, 2H), 7.59 (d, J = 8.5 Hz, 2H), 7.12 (d, J = 8.9 Hz, 1H), 4.53 (d, J = 6.9 Hz, 2H), 4.21 (s, 3H), 1.26 (t, J = 6.9 Hz, 3H). |
| **66** | 434.1 [M+H]⁺ | (DMSO) δ 13.35 (s, 1H), 8.56 (s, 1H), 7.67 (s, 1H), 7.55 (d, J = 8.8 Hz, 1H), 7.38 (s, 4H), 7.06 (d, J = 8.8 Hz, 1H), 4.47 (d, J = 6.8 Hz, 2H), 4.08 (s, 3H), 2.60 (s, 3H), 1.25 (t, J = 6.8 Hz, 3H). |
| **84** | 421.0 [M+H]⁺ | (DMSO) δ 13.41 (s, 1H), 8.68 (s, 1H), 8.59 (s, 1H), 8.22 (d, J = 9.0 Hz, 1H), 7.41 - 7.35 (m, 4H), 7.26 (d, J = 8.9 Hz, 1H), 4.50 (q, J = 6.9 Hz, 2H), 4.26 (s, 3H), 1.26 (t, J = 7.0 Hz, 3H). |
| **86** | 402.0 [M+H]⁺ | (DMSO) δ 13.42 (s, 1H), 8.68 (s, 1H), 8.61 (s, 1H), 8.41 (d, J = 1.8 Hz, 1H), 8.23 (d, J = 8.4 Hz, 1H), 7.64 (dd, J₁ = 8.0 Hz, J₂ = 2.0 Hz, 1H), 7.27 (d, J = 8.4 Hz, 1H), 7.22 (d, J = 8.0 Hz, 1H), 4.53 (d, J = 6.8 Hz, 2H), 4.26 (s, 3H), 2.47 (s, 3H), 1.27 (t, J = 6.8 Hz, 3H). |
| **87** | 456.0 [M+H]⁺ | (DMSO) δ 13.54 (s, 1H), 8.77 (s, 1H), 8.70 (s, 2H), 8.25 (d, J = 8.8 Hz, 1H), 8.10 (dd, J₁= 8.0 Hz, J₂= 1.6 Hz, 1H), 7.89 (d, J = 8.4 Hz, 1H), 7.30 (d, J = 7.2 Hz, 1H), 4.62 (q, J = 6.8 Hz, 2H), 4.27 (s, 3H), 1.31 (t, J = 6.8 Hz, 3H). |
| **90** | 461.95 [M+H]⁺ | (DMSO) δ 13.23 (s, 1H), 8.58 (s, 1H), 8.54 (s, 1H), 7.75 (d, *J=* 9.7 Hz, 2H), 7.38 (s, 4H), 7.17 (dd, *J*₁ = 9.0 Hz, *J*₂ = 1.8 Hz, 1H), 5.94 (p, *J =* 6.9 Hz, 1H), 5.06 (d, *J =* 6.8 Hz, 4H), 4.48 (q, *J =* 6.9 Hz, 2H), 1.25 (t, *J* = 6.9 Hz, 3H). |
| **92** | 497.00 [M+H]⁺ | (DMSO) δ 13.50 (s, 1H), 8.78 (d, *J* = 1.6 Hz, 1H), 8.68 (s, 1H), 8.60 (s, 1H), 8.10 (dd, *J =* 8.1, 1.7 Hz, 1H), 7.89 (d, *J =* 8.2 Hz, 1H), 7.77 (d, *J* = 8.4 Hz, 2H), 7.19 (dd, *J =* 9.2, 1.7 Hz, 1H), 5.94 (p, *J =* 6.8 Hz, 1H), 5.06 (d, *J =* 6.8 Hz, 4H), 4.61 (q, *J =* 6.9 Hz, 2H), 1.30 (t, *J =* 6.9 Hz, 3H). |
| **101** | 415.10 [M+H]⁺ | (DMSO) δ 13.39 (s, 1H), 8.58 (s, 1H), 8.44 (s, 1H), 8.41 (d, *J* = 2.1 Hz, 1H), 7.74-7.60 (m, 3H), 7.22 (d, *J =* 8.0 Hz, 1H), 7.11 (dd, *J* = 9.1, 1.0 Hz, 1H), 4.57-4.40 (m, 4H), 2.46 (s, 3H), 1.53 (t, *J =* 7.3 Hz, 3H), 1.26 (t, *J =* 6.9 Hz, 3H). |
| **102** | 469.05 [M+H]⁺ | (DMSO) δ 13.52 (s, 1H), 8.77 (d, *J =* 1.5 Hz, 1H), 8.67 (s, 1H), 8.45 (s, 1H), 8.10 (dd, *J =* 8.1, 1.5 Hz, 1H), 7.88 (d, *J =* 8.2 Hz, 1H), 7.72 (s, 1H), 7.68 (d, *J =* 9.0 Hz, 1H), 7.17 - 7.09 (m, 1H), 4.60 (q, *J =* 6.8 Hz, 2H), 4.50 (q, *J* = 7.2 Hz, 2H), 1.53 (t, *J =* 7.3 Hz, 3H), 1.30 (t, *J =* 6.9 Hz, 3H). |
| **111** | 477.05 [M+H]⁺ | (DMSO) δ 13.35 (s, 1H), 8.54 (s, 1H), 8.44 (s, 1H), 7.75 - 7.62 (m, 2H), 7.38 (s, 4H), 7.10 (dd, *J* = 9.1, 1.8 Hz, 1H), 4.55 (t, *J* = 6.2 Hz, 2H), 4.48 (q, *J =* 6.8 Hz, 2H), 2.80 (t, *J =* 6.2 Hz, 2H), 2.19 (s, 6H), 1.25 (t, *J* = 6.9 Hz, 3H). |
| **114** | 406.05 [M+H]⁺ | (DMSO) δ 13.24 (s, 1H), 8.85 (d, J = 7.3 Hz, 1H), 8.51 (s, 1H), 8.25 (s, 1H), 8.06 (s, 1H), 7.72 (d, J = 8.4 Hz, 2H), 7.51 - 7.41 (m, 2H), 6.98 (dd, J = 7.4, 2.1 Hz, 1H), 5.73 (s, 1H), 4.22 (q, J = 7.0 Hz, 2H), 1.41 (t, J = 7.0 Hz, 3H). |
| **116** | 421.00 [M+H]⁺ | (DMSO) δ 13.30 (s, 1H), 9.25 (d, J = 7.2 Hz, 1H), 8.26 (s, 1H), 7.73 (d, J = 8.5 Hz, 2H), 7.50 (d, J = 8.5 Hz, 2H), 7.14 (d, J = 7.1 Hz, 1H), 5.71 (s, 1H), 4.23 (q, J = 7.0 Hz, 2H), 2.62 (s, 3H), 1.41 (t, J = 7.0 Hz, 3H). |
| **117** | 400.10 | (DMSO) δ 13.30 (s, 1H), 8.51 (s, 1H), 8.39 (s, 1H), 7.69 (s, 1H), 7.64 |
| | [M+H]⁺ | (d, J = 9.2 Hz, 1H), 7.22 (d, J = 8.0 Hz, 2H), 7.15-7.08 (m, 3H), 4.40 (dd, J = 13.4, 6.6 Hz, 2H), 4.20 (s, 3H), 2.31 (s, 3H), 1.22 (t, J = 6.9 Hz, 3H). |
| **118** | 422.0 [M+H]⁺ | (DMSO) δ 13.38 (s, 1H), 8.59 (s, 1H), 8.40 (s, 1H), 7.79 - 7.59 (m, 2H), 7.44 - 7.33 (m, 2H), 7.22 (m, 1H), 7.11 (dd, J = 9.2, 2.0 Hz, 1H), 4.51 (q, J = 7.2 Hz, 2H), 4.21 (s, 3H), 1.27 (t, J = 6.8 Hz, 3H). |
| **124** | 456.1 [M+H]⁺ | (DMSO) δ 13.53 (s, 1H), 9.13 (s, 2H), 8.70 (s, 1H), 8.42 (s, 1H), 7.74 (s, 1H), 7.68 (d, J = 9.2 Hz, 1H), 7.14 (dd, J = 9.2, 2.0 Hz, 1H), 4.66 (q, J = 6.8 Hz, 2H), 4.21 (s, 3H), 1.34 (t, J = 6.8 Hz, 3H). |
| **125** | 403.1 [M+H]⁺ | (DMSO) δ 13.36 (s, 1H), 11.55 (s, 1H), 8.53 (s, 1H), 8.40 (s, 1H), 7.73 - 7.61 (m, 2H), 7.47 (dd, J = 9.6, 2.6 Hz, 1H), 7.34 (d, J = 2.4 Hz, 1H), 7.09 (dd, J = 9.2, 2.0 Hz, 1H), 6.29 (d, J = 9.6 Hz, 1H), 4.51 (q, J = 6.8 Hz, 2H), 4.21 (s, 3H), 1.31 (t, J = 6.8 Hz, 3H). |
| **126** | 417.05 [M+H]⁺ | (DMSO) δ 13.35 (s, 1H), 8.56 (s, 1H), 8.40 (s, 1H), 8.13 (d, J = 2.2 Hz, 1H), 7.67 (dt, J = 9.5, 6.0 Hz, 3H), 7.11 (dd, J = 9.1, 1.6 Hz, 1H), 6.79 (d, J = 8.5 Hz, 1H), 4.50 (q, J = 6.7 Hz, 2H), 4.21 (s, 3H), 3.86 (s, 3H), 1.27 (t, J = 6.9 Hz, 3H). |
| **127** | 421.00 [M+H]⁺ | (DMSO) δ 13.42 (s, 1H), 8.60 (s, 1H), 7.99 (d, *J =* 8.9 Hz, 1H), 7.97 (s, 1H), 7.39 (s, 4H), 7.35 (s, 1H), 4.54 (s, 3H), 4.49 (dd, *J =* 13.6, 6.7 Hz, 2H), 1.26 (t, *J =* 7.0 Hz, 3H). |
| **128** | 421.00 [M+H]⁺ | (DMSO) δ 13.42 (s, 1H), 8.60 (s, 1H), 8.13 (d, *J =* 8.8 Hz, 1H), 8.01 (s, 1H), 7.39 (s, 4H), 7.37 (dd, *J =* 8.9, 1.7 Hz, 1H), 4.50 (q, *J =* 6.9 Hz, 2H), 4.32 (s, 3H), 1.26 (t, *J =* 7.0 Hz, 3H). |
| **131** | 477.05 [M+H]⁺ | (DMSO) δ 13.35 (s, 1H), 8.54 (s, 1H), 8.11 (s, 1H), 7.81 - 7.71 (m, 2H), 7.38 (s, 4H), 7.30 (dd, *J* = 8.8, 1.8 Hz, 1H), 4.56 (t, *J* = 6.3 Hz, 2H), 4.49 (q, *J =* 6.9 Hz, 2H), 2.80 (s, 2H), 2.23 (s, 6H), 1.25 (t, *J =* 7.0 Hz, 3H). |
| **132** | 478.10 [M+H]⁺ | (DMSO) δ 13.32 (s, 1H), 8.72 (s, 1H), 8.55 (s, 1H), 8.23 (d, *J* = 9.0 Hz, 1H), 7.38 (s, 4H), 7.27 (d, *J* = 9.2 Hz, 1H), 4.60 (t, *J* = 6.2 Hz, 2H), 4.51 (q, *J* = 7.0 Hz, 2H), 2.83 (t, *J* = 6.2 Hz, 2H), 2.19 (s, 6H), 1.28 - 1.21 (m, 3H). |
| **133** | 401.1 [M+H]⁺ | (DMSO) δ 13.33 (s, 1H), 8.68 (s, 1H), 8.53 (s, 1H), 8.22 (d, *J =* 8.8 Hz, 1H), 7.22 (m, 3H), 7.14 (d, *J =* 8.0 Hz, 2H), 4.43 (t, *J =* 6.4 Hz, 2H), 4.26 (s, 3H), 2.31 (s, 3H), 1.24 (t, *J =* 7.2 Hz, 3H). |
| **137** | 421.95 [M+H]⁺ | (DMSO) δ 13.38 (s, 1H), 8.63 (d, *J =* 8.8 Hz, 1H), 8.58 (s, 1H), 7.57 (d, *J* = 8.9 Hz, 1H), 7.39 (s, 4H), 4.60 (s, 3H), 4.53 (q, *J* = 6.9 Hz, 2H), 1.27 (t, *J =* 7.0 Hz, 3H). |
| **139** | 478.10 [M+H]⁺ | (DMSO) δ 13.33 (s, 1H), 8.55 (s, 1H), 8.38 (d, *J =* 8.9 Hz, 1H), 8.30 (s, 1H), 7.45 (d, *J* = 8.7 Hz, 1H), 7.38 (s, 4H), 4.59 (t, *J* = 6.1 Hz, 2H), 4.51 (q, *J =* 7.0 Hz, 2H), 2.75 (t, *J =* 6.2 Hz, 2H), 2.18 (s, 6H), 1.26 (t, *J* = 7.0 Hz, 3H). |
| **141** | 421.95 [M+H]⁺ | (DMSO) δ 13.31 (s, 1H), 8.77 (d, *J =* 8.6 Hz, 1H), 8.58 (s, 1H), 7.61 (d, *J =* 8.5 Hz, 1H), 7.39 (s, 4H), 4.62 - 4.46 (m, 2H), 4.30 (s, 3H), 1.27 (t, *J* = 7.0 Hz, 3H). |
| **142** | 425.2 [M+H]⁺ | (DMSO) δ 13.36 (s, 1H), 8.56 (s, 1H), 8.39 (s, 1H), 7.70 (s, 1H), 7.65 (d, *J =* 9.2 Hz, 1H), 7.38 (s, 4H), 7.11 (dd, *J =* 9.2, 1.6 Hz, 1H), 4.20 (s, 3H). |

### Trifluoroacetate of compound 35:

Using the synthesis method of Example 4, the 4-methoxyphenylboronic acid pinacol ester in the step 3 was replaced with 4-chlorophenylboronic acid pinacol ester, the 5-bromo-2-methyl-2H-indazole in the step 4 was replaced with 1-(azetine-3-yl)-6-iodo-1H-benzo[d]imidazole, and the mobile phase FA purified by prep-HPLC in the step 5 was replaced with trifluoroacetic acid to obtain a trifluoroacetate of compound **35.** ¹H NMR (400 MHz, MeOD) δ 8.83 (s, 1H), 8.46 (d, J = 9.1 Hz, 1H), 7.92 - 7.82 (dd, J = 23.1, 14.3 Hz, 2H), 7.46 - 7.25 (m, 5H), 5.90 - 5.63 (m, 1H), 4.81 - 4.58 (m, 4H), 4.51 (q, J = 7.0 Hz, 2H), 1.33 (t, J = 7.0 Hz, 3H). LCMS (ESI) m/z = 460.85 [M+H]⁺.

### Example 5: Synthesis of compound 12

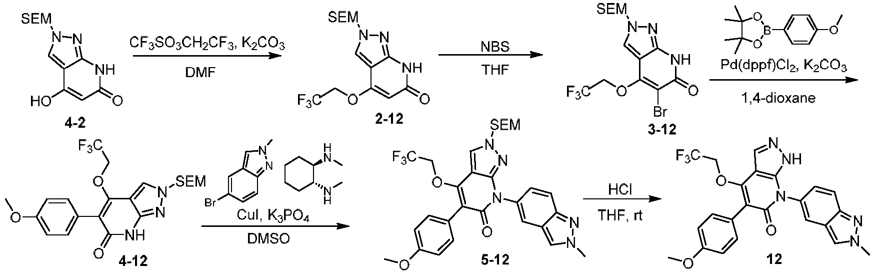

### Step 1: Synthesis of intermediate 2-12

The intermediate **4-2** (2.0 g, 7.1 mmol) and potassium carbonate (1.96 g, 14.2 mmol) were dissolved in DMF (25 mL) in a 25 mL single-necked flask. 2,2,2-trifluoroethyl trifluoromethanesulfonate solution (3.3 g, 14.2 mmol) was added to the reaction solution and stirred at room temperature for 6 h. The reaction solution was poured into water, and extracted with EA (100 mL). The organic layer was combined, washed once with water and once with saturated saline solution, dried with anhydrous sodium sulfate, and filtered. The filtrate was concentrated and purified by a silica gel rapid column (0%-60% EA) to obtain an intermediate **2-12** (0.6 g, yield: 51.9%) as a white solid. ¹HNMR (400 MHz, DMSO) δ 11.53 (s, 1H), 8.35 (s, 1H), 5.70 (s, 1H), 5.41 (s, 2H), 4.91 (q, *J =* 8.8 Hz, 2H), 3.54 (t, *J =* 8.0 Hz, 2H), 0.83 (t, *J =* 8.0 Hz, 2H), -0.05 (s, 9H). LCMS (ESI) m/z = 363.9 [M+H]⁺.

### Step 2: Synthesis of intermediate 3-12

The intermediate **2-12** (0.6 g, 1.36 mmol) was dissolved in THF (15 mL) in a 25 mL single-necked flask, added with NBS (0.24 g, 1.36 mmol), and stirred at room temperature for 2 h. The reaction solution was slowly added dropwise to ice water. There were a large amount of solids to separate out, and suction filtration was performed. A filter cake was purified by a silica gel rapid column (0%-4% methanol/DCM) to obtain an intermediate **3-12** (600 mg, yield: 93.7%) as a white solid. ¹HNMR (400 MHz, DMSO) δ 12.23 (s, 1H), 8.76 (s, 1H), 5.50 (s, 2H), 5.26 (q, *J =* 8.5 Hz, 2H), 3.68 - 3.57 (m, 2H), 0.89 (dd, *J*₁ = 10.6 Hz, *J*₂ =5.6 Hz, 2H), -0.00 (s, 9H). LCMS (ESI) m/z = 441.9 [M+H]⁺.

### Step 3: Synthesis of intermediate 4-12

The intermediate **3-12** (200 mg, 0.426 mmol), 4-methoxyphenylboronic acid pinacol ester (200 mg, 0.852 mmol), Pd(dppf)Cl₂ (34.5 mg, 0.043 mmol), potassium carbonate (117 mg, 0.852 mmol), 1,4-dioxane (15 ml), and water (3 ml) were sequentially added into a 50 mL single-necked flask, and stirred at 100°C under N₂ protection for 16 h. The mixture was filtered. The filtrate was concentrated and purified by a silica gel rapid column (0%-3% methanol/DCM) to obtain an intermediate **4-12** (95 mg, yield: 47.5%) as a white solid. LCMS (ESI) m/z = 470.0 [M+H]⁺.

### Step 4: Synthesis of intermediate 5-12

The intermediate **4-12** (95 mg, 0.2 mmol), 5-bromo-2-methyl-2H-indazole (85 mg, 0.4 mmol), trans N,N'-dimethylcyclohexanediamine (6 mg, 0.04 mmol), cuprous iodide (8 mg, 0.04 mmol), potassium phosphate (85 mg, 0.4 mmol), and DMSO (8 ml) were sequentially added into a 20 mL microwave tube and performed a microwave reaction at 110° C under nitrogen protection for 3 h. The reaction solution was poured into ice water, and extracted with 50 mL of methanol/DCM (1/10). The organic layer was combined, washed once with water and once with saturated saline solution, dried with anhydrous sodium sulfate, and filtered. The filtrate was collected and purified by a silica gel rapid column (0%-3% methanol/DCM) to obtain an intermediate **5-12** (80 mg, yield: 65.8%) as a white solid. LCMS (ESI) m/z = 600.2 [M+H]⁺.

### Step 5: Synthesis of compound 12

The intermediate **5-12** (80 mg, 0.133 mmol) was dissolved in 4 M hydrogen chloride 1,4-dioxane (6 mL) in a 25 mL single-necked flask, and stirred at room temperature for 4 h. The reaction solution was poured into water, and the pH was adjusted to neutral with sodium bicarbonate aqueous solution. The solution was extracted with 50 mL of DCM/methanol (10/1). The organic layer was combined, dried with anhydrous sodium sulfate, and filtered. The filtrate was collected, concentrated under reduced pressure to dryness, purified by prep-HPLC (water/acetonitrile, 0.1% FA), and freeze dried to obtain a compound **12** (31.53 mg, yield: 50.4%) as a white solid. ¹H NMR (400 MHz, DMSO) δ 13.38 (s, 1H), 8.51 (s, 1H), 8.41 (s, 1H), 7.72 (s, 1H), 7.67 (d, *J =* 9.2 Hz, 1H), 7.28 (d, *J =* 8.8 Hz, 2H), 7.11 (dd, *J*₁ *=* 9.2 Hz, *J*₂ = 2.0 Hz, 1H), 6.91 (d, *J =* 8.8 Hz, 2H), 4.98 (q, *J =* 8.8 Hz, 2H), 4.21 (s, 3H), 3.77 (s, 3H). LCMS (ESI) m/z = 470.1 [M+H]⁺.

Compounds **27-29, 46-47, 49, 52-54, 89, 93 and 99** were prepared using the synthesis method of Example **5.**

| Compound | LCMS (ESI) m/z | ¹H NMR (400 MHz) |
|---|---|---|
| **27** | 506.10 [M+H]⁺ | (DMSO) δ 13.46 (s, 1H), 8.59 (s, 1H), 8.40 (s, 1H), 7.72 (s, 1H), 7.66 (d, *J =* 9.1 Hz, 1H), 7.43 - 7.40 (m, 1H), 7.40 (d, *J =* 2.0 Hz, 1H), 7.28 (t, *J =* 76.0 Hz, 1H), 7.16 (s, 1H), 7.14 (s, 1H), 7.12 (d, *J* = 9.3 Hz, 1H), 5.07 (q, *J =* 8.7 Hz, 2H), 4.21 (s, 3H). |
| **28** | 473.80 [M+H]⁺ | (DMSO) δ 13.48 (s, 1H), 8.61 (s, 1H), 8.41 (s, 1H), 7.71 (s, 1H), 7.66 (d, *J =* 9.0 Hz, 1H), 7.44 - 7.39 (m, 2H), 7.39 - 7.35 (m, 2H), 7.12 (d, *J* = 9.0 Hz, 1H), 5.09 (q, *J =* 8.5 Hz, 2H), 4.21 (s, 3H). |
| **29** | 471.00 [M+H]⁺ | (DMSO) δ 13.41 (s, 1H), 8.56 (s, 1H), 8.41 (s, 1H), 8.14 (d, *J* = 2.3 Hz, 1H), 7.73 (s, 1H), 7.68 (dd, *J =* 8.6, 2.2 Hz, 2H), 7.12 (dd, *J =* 9.0, 1.8 Hz, 1H), 6.81 (d, *J =* 8.6 Hz, 1H), 5.12 (q, *J =* 8.6 Hz, 2H), 4.21 (s, 3H), 3.86 (s, 3H). |
| **36** | 474.05 [M+H]⁺ | (DMSO) δ 13.48 (s, 1H), 8.61 (s, 1H), 8.28 (s, 1H), 7.74 (d, *J =* 8.5 Hz, 1H), 7.63 (s, 1H), 7.39 (q, *J =* 8.7 Hz, 4H), 7.14 (d, *J =* 8.4 Hz, 1H), 5.10 (d, *J =* 8.5 Hz, 2H), 3.84 (s, 3H). |
| **46** | 454.85 [M+H]⁺ | (DMSO) δ 13.49 (s, 1H), 8.77 (d, *J* = 1.6 Hz, 1H), 8.67 (s, 1H), 8.41 (s, 1H), 8.10 (dd, *J* = 8.2, 1.5 Hz, 1H), 7.88 (d, *J =* 8.0 Hz, 1H), 7.79 - 7.57 (m, 2H), 7.13 (dd, *J =* 9.1, 1.8 Hz, 1H), 4.59 (t, *J =* 6.9 Hz, 2H), 4.21 (s, 3H), 1.30 (t, *J =* 6.9 Hz, 3H). |
| **47** | 426.80 [M+H]⁺ | (DMSO) δ 13.35 (s, 1H), 8.56 (s, 1H), 8.40 (s, 1H), 8.35 (d, *J=* 1.8 Hz, 1H), 7.78 - 7.62 (m, 2H), 7.60 (dd, *J =* 8.0, 2.2 Hz, 1H), 7.25 (d, *J* = 8.0 Hz, 1H), 7.11 (dd, *J =* 9.1, 1.8 Hz, 1H), 4.50 (q, *J =* 6.8 Hz, 2H), 4.21 (s, 3H), 2.07 (td, *J =* 7.7, 3.8 Hz, 1H), 1.26 (t, *J =* 7.0 Hz, 3H), 0.92 (t, *J =* 6.6 Hz, 4H). |
| **49** | 458.10 [M+H]⁺ | (DMSO) δ 13.45 (s, 1H), 8.59 (s, 1H), 8.41 (s, 1H), 7.81 - 7.55 (m, 2H), 7.38 (dd, *J* = 8.7, 5.8 Hz, 2H), 7.17 (t, *J =* 9.0 Hz, 2H), 7.12 (dd, *J* = 9.0, 1.3 Hz, 1H), 5.06 (q, *J =* 8.8 Hz, 2H), 4.21 (s, 3H). |
| **52** | 455.10 [M+H]⁺ | (DMSO) δ 13.48 (s, 1H), 8.62 (s, 1H), 8.41 (d, *J =* 1.6 Hz, 2H), 7.78 - 7.59 (m, 3H), 7.24 (d, *J =* 8.0 Hz, 1H), 7.12 (dd, *J =* 8.9, 1.2 Hz, 1H), 5.14 (q, *J =* 8.4 Hz, 2H), 4.21 (s, 3H), 2.47 (s, 3H). |
| **53** | 481.15 [M+H]⁺ | (DMSO) δ 13.48 (s, 1H), 8.61 (s, 1H), 8.51-8.26 (m, 2H), 7.75- 7.64 (m, 2H), 7.60 (dd, *J* = 8.1, 2.2 Hz, 1H), 7.27 (d, *J* = 7.9 Hz, 1H), 7.12 (dd, *J =* 9.1, 1.6 Hz, 1H), 5.12 (q, *J =* 8.4 Hz, 2H), 4.21 (s, 3H), 2.09 (tt, *J* = 7.9, 5.1 Hz, 1H), 0.93 (ddd, *J =* 7.5, 4.4, 2.5 Hz, 4H). |
| **54** | 464.05 [M+H]⁺ | (DMSO) δ 13.36 (s, 1H), 8.57 (s, 1H), 8.39 (s, 1H), 7.69 (s, 1H), 7.65 (d, *J =* 8.5 Hz, 1H), 7.51 (d, *J =* 8.5 Hz, 2H), 7.32 (d, *J =* 8.5 Hz, 2H), 7.11 (d, *J =* 8.3 Hz, 1H), 4.48 (d, *J =* 7.0 Hz, 2H), 4.21 (s, 3H), 1.25 (t, *J =* 6.9 Hz, 3H). |
| **89** | 456.05 [M+H]⁺ | (DMSO) δ 13.52 (s, 1H), 8.70 (s, 1H), 8.62 (s, 1H), 8.41 (d, *J* = 1.8 Hz, 1H), 8.26 (d, *J =* 9.1 Hz, 1H), 7.64 (dd, *J =* 8.0, 2.2 Hz, 1H), 7.30 (d, *J =* 8.9 Hz, 1H), 7.25 (d, *J =* 8.0 Hz, 1H), 5.17 (q, *J =* 8.6 Hz, 2H), 4.27 (s, 3H), 2.48 (s, 3H). |
| **93** | 515.95 [M+H]⁺ | (DMSO) δ 13.46 (s, 1H), 8.59 (s, 2H), 7.77 (d, *J* = 9.2 Hz, 2H), 7.44 - 7.36 (m, 4H), 7.18 (dd, *J* = 9.0, 1.9 Hz, 1H), 5.94 (p, *J* = 6.8 Hz, 1H), 5.16 - 5.08 (m, 2H), 5.06 (d, *J =* 6.8 Hz, 4H). |
| **99** | 474.95 [M+H]⁺ | (DMSO) δ 13.51 (s, 1H), 8.66 (d, *J =* 25.4 Hz, 2H), 8.24 (d, *J =* 8.9 Hz, 1H), 7.44 - 7.39 (m, 2H), 7.39 - 7.35 (m, 2H), 7.28 (d, *J =* 8.9 Hz, 1H), 5.11 (q, *J =* 8.6 Hz, 2H), 4.27 (s, 3H). |

### Example 6: Synthesis of compounds 31 and 45

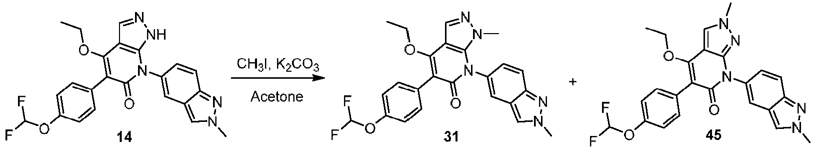

The compound **14** (50 mg, 0.011 mmol) and cesium carbonate (7 mg, 0.022 mmol) were dissolved in 3 mL of acetone in a 25 mL single-necked flask, added with iodomethane (4 mg, 0.03 mmol), and stirred at room temperature for 1 h after adding completion. The reaction solution was concentrated to dryness to obtain a crude product, which was purified by pre-TLC (DCM/MeOH=20/1), and freeze dried to obtain a compound **31** (15 mg, yield: 29.1%) as a white solid; and a compound **45** (35 mg, yield: 67.9%) as a white solid.

Compound **31:** ¹H NMR (400 MHz, DMSO) δ 8.49 (s, 1H), 8.14 (s, 1H), 7.86 (d, *J* = 1.3 Hz, 1H), 7.74 (d, *J =* 9.0 Hz, 1H), 7.40 (d, *J =* 8.8 Hz, 2H), 7.26 (t, *J =* 76.0 Hz, 1H), 7.24 (dd, *J =* 9.0, 2.0 Hz, 1H), 7.13 (d, *J =* 8.7 Hz, 2H), 4.46 (q, *J =* 7.0 Hz, 2H), 4.22 (s, 3H), 3.05 (s, 3H), 1.25 (t, *J =* 7.0 Hz, 3H). LCMS (ESI) m/z = 466.0 [M+H]⁺.

Compound **45:** ¹H NMR (400 MHz, DMSO) δ 8.57 (s, 1H), 8.40 (s, 1H), 7.68 (d, *J* = 1.4 Hz, 1H), 7.65 (d, *J =* 9.0 Hz, 1H), 7.40 (d, *J =* 8.7 Hz, 2H), 7.26 (t, *J =* 76.0 Hz, 1H), 7.13 (d, *J =* 8.6 Hz, 2H), 7.10 (d, *J =* 1.9 Hz, 1H), 4.42 (d, *J =* 7.0 Hz, 2H), 4.20 (s, 3H), 3.83 (s, 3H), 1.25 (t, *J =* 7.0 Hz, 3H). LCMS (ESI) m/z = 466.0 [M+H]⁺.

### Example 7: Synthesis of compound 50

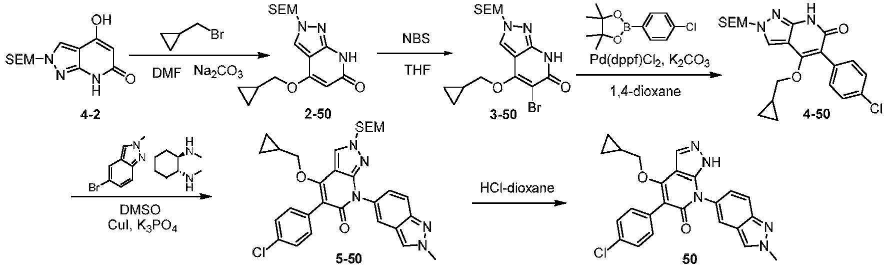

### Step 1: Synthesis of intermediate 2-50

The intermediate 4-2 (4 g, 14.2 mol) was dissolved in DMF (50 mL) in a 250 mL single-necked flask, added with sodium carbonate (3 g, 28.4 mol) and bromomethyl cyclopropane (6.72 g, 49.7 mol), heated up to 90°C, and stirred for 3 h. At the end of the reaction, the reaction solution was poured into water to precipitate a solid, and performed suction filtration to obtain a crude product, which was purified by a silica gel Flash column (0%-2% methanol) to obtain an intermediate 2-50 (1.5 g, yield: 31.5%) as a white solid. LCMS (ESI) m/z = 336.1 [M+H]⁺.

### Step 2: Synthesis of intermediate 3-50

The intermediate 2-50 (1.5 g, 4.47 mmol) was dissolved in THF (20 mL) in a 50 mL single-necked flask. NBS (0.84 g, 4.7 mmol) was added in batches at room temperature, and reacted at room temperature for 2 h. The reaction solution was poured into ice water, and extracted with EA (50 mL). The organic layer was combined, dried with anhydrous sodium sulfate, and filtered. The filtrate was concentrated to obtain a crude product, which was purified by a silica gel Flash column (0%-100% EA) to obtain an intermediate 3-50 (1.35 g, yield: 73.6%) as a pale yellow solid. LCMS (ESI) m/z = 414.0 [M+H]⁺.

### Step 3: Synthesis of intermediate 4-50

The intermediate 3-50 (1.25 g, 3 mmol), 4-chlorophenylboronic acid pinacol ester (1.43 g, 11.6 mmol), Pd(dppf)Cl₂ (0.24 g, 0.58 mmol), potassium carbonate (0.83 g, 11.6 mmol), 1,4-dioxane (15 mL), and water (3 ml) were sequentially added into a 100 mL single-necked flask, heated up to 100°C under N₂ protection, and stirred for 16 h. The reaction solution was concentrated and purified by a silica gel Flash column (PE:EA=1:1) to obtain an intermediate 4-50 (0.55 g, yield: 41%) as a pale yellow solid. LCMS (ESI) m/z = 446.1 [M+H]⁺.

### Step 4: Synthesis of intermediate 5-50

The intermediate 4-50 (550 mg, 1.23 mmol), 5-bromo-2-methyl-2H-indazole (516 mg, 2.46 mmol), trans N,N'-dimethylcyclohexanediamine (20 mg, 0.12 mmol), cuprous iodide (47 mg, 0.246 mmol), potassium phosphate (524 mg, 2.46 mmol), DMSO (10 ml) were sequentially added into a 50 mL single-necked flask, heated up to 130°C under N₂ protection, and reacted overnight. The reaction solution was concentrated to dryness and concentrated to obtain a crude product, which was purified by a silica gel Flash column (3% methanol/DCM) to obtain an intermediate 5-50 (420 mg, yield: 59.1 %) as a white solid. LCMS (ESI) m/z = 576.2 [M+H]⁺.

### Step 5: Synthesis of compound 50

The intermediate 5-50 (420 mg, 0.73 mmol) was dissolved in 4 M hydrochloric acid/1,4-dioxane (10 mL) in a 50 mL single-necked flask, and reacted at room temperature for 4 h. The reaction solution was poured into water, and the pH was adjusted to alkaline with ammonia solution. The solution was extracted with 50 mL of DCM/methanol (10/1). The organic layer was combined, dried with anhydrous sodium sulfate, and filtered. The filtrate was concentrated under reduced pressure to dryness, purified by prep-HPLC (water/acetonitrile, 0.1% NH₃.H₂O), and freeze dried to obtain an end product compound 50 (164.39 mg, yield: 50.5%) as a white solid. ¹H NMR (400 MHz, DMSO) δ 13.33 (s, 1H), 8.52 (s, 1H), 8.40 (s, 1H), 7.74 - 7.61 (m, 2H), 7.45 - 7.33 (m, 4H), 7.11 (d, J = 8.9 Hz, 1H), 4.28 (d, J = 6.6 Hz, 2H), 4.20 (s, 3H), 1.18 - 1.04 (m, 1H), 0.50 (d, J = 7.3 Hz, 2H), 0.29 (d, J = 4.0 Hz, 2H). LCMS (ESI) m/z = 446.0 [M+H]⁺.

Compounds **32, 39, 51, 95, 96 and 104** were prepared using the synthesis method of Example **7.**

| Comp -ound | LCMS (ESI) m/z | ¹H NMR (400 MHz) |
|---|---|---|
| **32** | 478.1 [M+H]⁺ | (DMSO) δ 13.33 (s, 1H), 8.55 (s, 1H), 8.27 (s, 1H), 7.72 (d, *J =* 8.0 Hz, 1H), 7.62 (s, 1H), 7.43 (d, *J =* 8.8 Hz, 2H), 7.27 (t, *J =* 74.4 Hz, 1H), 7.14 (d, *J* = 8.7 Hz, 3H), 4.25 (t, *J =* 7.9 Hz, 2H), 3.83 (d, *J =* 7.5 Hz, 3H), 1.11 (s, 1H), 0.54 - 0.46 (m, 2H), 0.29 (t, *J =* 5.0 Hz, 2H). |
| **39** | 478.1 [M+H]⁺ | (DMSO) δ 13.34 (s, 1H), 8.54 (s, 1H), 8.39 (s, 1H), 7.69 (s, 1H), 7.64 (d, J = 8.8 Hz, 1H), 7.43 (d, J = 8.8 Hz, 2H), 7.27 (t, J = 74.4 Hz, 1H), 7.14 (d, J = 8.7 Hz, 2H), 7.11 (d, J = 9.4 Hz, 1H), 4.25 (d, J = 6.9 Hz, 2H), 4.20 (s, 3H), 1.11 (s, 1H), 0.55-0.44 (m, 2H), 0.28 (d, J = 4.4 Hz, 2H). |
| **51** | 430.1 [M+H]⁺ | (DMSO) δ 13.33 (s, 1H), 8.53 (s, 1H), 8.39 (s, 1H), 7.72-7.62 (m, 2H), 7.44-7.36 (m, 2H), 7.20-7.07 (m, 3H), 4.25 (d, *J =* 6.7 Hz, 2H), 4.21 (s, 3H), 1.10 (s, 1H), 0.52 - 0.44 (m, 2H), 0.27 (d, *J* = 4.7 Hz, 2H). |
| **95** | 446.0 [M+H]⁺ | (DMSO) δ 13.33 (s, 1H), 8.54 (s, 1H), 8.26 (s, 1H), 7.65 (d, *J =* 8.0 Hz, 1H), 7.59 (s, 1H), 7.40 (q, *J =* 8.8 Hz, 4H), 7.20 (d, *J* = 8.4 Hz, 1H), 4.28 (d, *J =* 6.4 Hz, 2H), 3.89 (s, 3H), 1.11 (s, 1H), 0.54 - 0.45 (m, 2H), 0.29 (m, 2H). |
| **96** | 447.00 [M+H]⁺ | (DMSO) δ 13.37 (s, 1H), 8.68 (s, 1H), 8.57 (s, 1H), 8.23 (d, *J =* 8.9 Hz, 1H), 7.44-7.34 (m, 4H), 7.27 (d, *J*=7.1 Hz, 1H), 4.30 (d, *J=* 6.8 Hz, 2H), 4.26 (s, 3H), 1.11 (d, *J =* 7.6 Hz, 1H), 0.55 - 0.46 (m, 2H), 0.30 (t, *J =* 5.1 Hz, 2H). |
| **104** | 481.05 [M+H]⁺ | (DMSO) δ 13.48 (s, 1H), 8.80 (d, *J =* 1.8 Hz, 1H), 8.65 (s, 1H), 8.41 (s, 1H), 8.12 (dd, *J =* 8.1, 1.6 Hz, 1H), 7.89 (d, *J =* 7.9 Hz, 1H), 7.73 (s, 1H), 7.67 (d, *J =* 9.0 Hz, 1H), 7.13 (dd, *J =* 9.1, 1.9 Hz, 1H), 4.40 *(d, J=7.0* Hz, 2H), 4.21 (s, 3H), 1.15 (qd, *J*=7.5, 3.9 Hz, 1H), 0.59 - 0.46 (m, 2H), 0.38-0.27 (m, 2H). |

### Example 8: Synthesis of compound 59

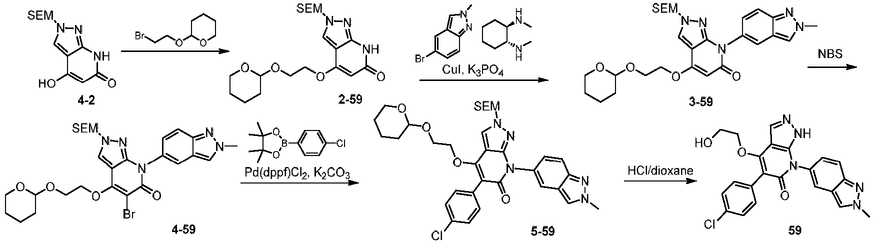

### Step 1: Synthesis of intermediate 2-59

The intermediate 4-2 (1.2 g, 4.27 mmol), 2-(2-bromoethoxy)tetrahydropyran (1.0 g, 4.7 mmol), and sodium carbonate (0.9 g, 8.54 mmol) were dissolved in DMF (15 mL) in a 50 mL single-necked flask, and reacted at 90°C for 2 h. Water (50 mL) was added to quench the reaction. The mixture was extracted with EA, washed with saturated saline solution, and dried with anhydrous sodium sulfate. The mixture was concentrated to obtain a crude product, which was separated by silica gel Flash (PE/EA=1/4) to obtain an intermediate 2-59 (640 mg, yield: 36.64%) as a yellow solid. LCMS (ESI) m/z = 410.2 [M+H]⁺.

### Step 2: Synthesis of intermediate 3-59

The intermediate 2-59 (600 mg, 1.46 mmol), 5-bromo-2-methyl-2H-indazole (462 mg, 2.20 mmol), (1R,2R)-dimethylcyclohexane-1,2-diamine (207 mg, 1.46 mmol), cuprous iodide (277 mg, 1.46 mmol), and potassium phosphate (403 mg, 2.92 mmol) were dissolved in DMSO (10 mL) in a 25 mL single-necked flask, and reacted at 110°C under nitrogen protection for 16 h. Water was added to quench the reaction. The mixture was extracted with EA, washed with saturated saline solution, and dried with anhydrous sodium sulfate. The mixture was concentrated and separated by silica gel Flash (EA/PE=9/1) to obtain an intermediate 3-59 (600 mg, yield: 76.24%) as a yellow solid. LCMS (ESI) m/z = 540.2 [M+H]⁺.

### Step 3: Synthesis of intermediate 4-59

The intermediate 3-59 (500 mg, 0.928 mmol) was dissolved in THF (20 mL) in a 50 mL single-necked flask, then added with NBS (173 mmol, 0.974 mmol) in batches, and reacted at room temperature for 2 h. A saturated sodium thiosulfate solution was added to quench the reaction. The mixture was extracted with EA, washed with saturated saline solution, and dried with anhydrous sodium sulfate. The mixture was concentrated to obtain a crude product, which was purified by silica gel Flash (EA/PE=90/10) to obtain an intermediate 4-59 (400 mg, yield: 69.82%) as a pale yellow solid. LCMS (ESI) m/z = 618.0 [M+H]⁺.

### Step 4: Synthesis of intermediate 5-59

The intermediate 4-59 (100 mg, 0.162 mmol), 4-chlorophenylboronic acid pinacol ester (77.1 mg, 0.324 mmol), Pd(dppf)Cl₂ (11.8 mg, 0.016 mmol), potassium carbonate (44.7 mg, 0.324 mmol), and 1,4-dioxane/water=5/1 (2 mL) were sequentially added into a 25 mL single-necked flask, and reacted at 100°C under nitrogen protection for 16 h. Water (10 mL) was added to quench the reaction. The mixture was extracted with DCM, washed with saturated saline solution, and dried with anhydrous sodium sulfate. The mixture was concentrated to obtain a crude product, which was purified by silica gel Flash (methanol/DCM=2/98) to obtain an intermediate 5-59 (94 mg, yield: 89.40%) as a pale yellow solid. LCMS (ESI) m/z = 650.1 [M+H]⁺.

### Step 5: Synthesis of compound 59

The intermediate 5-59 (80 mg, 0.123 mmol) was dissolved in hydrogen chloride/1,4-dioxane solution (4 M, 2 mL) in a 25 mL single-necked flask, and reacted at room temperature for 4 h. Ice water (10 mL) was added to quench the reaction. The pH of the reaction solution was adjusted to 8-9 with ammonia water. The reaction solution was extracted with DCM/methanol=10/1, washed with saturated saline solution, and dried with anhydrous sodium sulfate. The mixture was concentrated to obtain a crude product, which was separated by prep-HPLC (ACN/water-0.1% NH₃H₂O), and freeze dried to obtain an end product 59 (16.68 mg, yield: 31.17%) as a white solid. ¹H NMR (400 MHz, DMSO) δ 13.35 (s, 1H), 8.55 (s, 1H), 8.39 (s, 1H), 7.70 (s, 1H), 7.65 (d, J = 8.8 Hz, 1H), 7.46 - 7.41 (m, 2H), 7.39 - 7.34 (m, 2H), 7.11 (d, J = 8.8 Hz, 1H), 4.84 (t, J = 5.2 Hz, 1H), 4.41 (s, 2H), 4.21 (s, 3H), 3.64 (dd, J = 9.6, 4.8 Hz, 2H). LCMS (ESI) m/z = 436.0[M+H]⁺.

### Example 9: Synthesis of compound 67

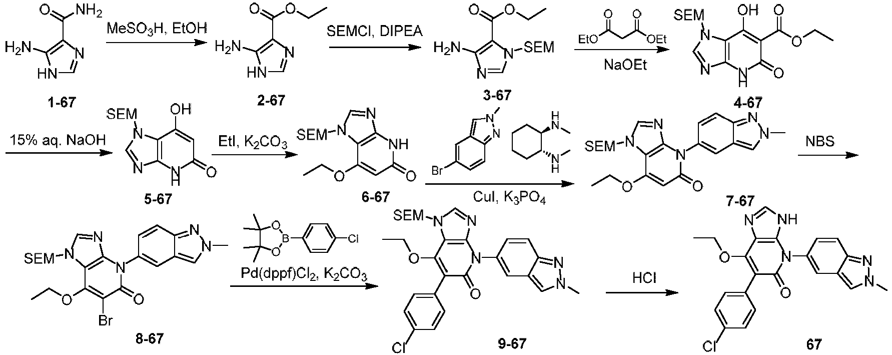

### Step 1: Synthesis of intermediate 2-67

4-amino-5-imidazolecarboxamide 1-67 (10 g, 79 mmol), methanesulfonic acid (20 mL), and ethanol (100 mL) were sequentially added into a 200 mL reaction vessel. After sealing, the system was allowed to react at 120°C for 16 h. The reaction solution was neutralized with ammonia water to pH 8-9, extracted with methanol/DCM=1/10 (50 mL×5), and concentrated to obtain a crude product, which was separated by silica gel Flash (methanol/DCM=20/80) to obtain an intermediate 2-67 (6.4 g, yield: 52.03%) as a white solid. LCMS (ESI) m/z = 156.0 [M+H]⁺.

### Step 2: Synthesis of intermediate 3-67

The intermediate 2-67 (6.0 g, 38.7 mmol) and diisopropylethylamine (7.5 g, 58.1 mmol) were dissolved in THF (100 mL) in a 250 mL three-necked flask. 2-(trimethylsilyl)ethoxymethyl chloride (9.6 g, 58.1 mmol) was slowly adddd dropwise, and reacted at room temperature for 2 h after adding completion. The solvent was removed, the rest was diluted with water, extracted with EA, washed with saturated saline solution, and dried with anhydrous sodium sulfate. The mixture was concentrated to obtain a crude product, which was separated by silica gel Flash (EA/PE=1/1) to obtain an intermediate 3-67 crude product (3.0 g, yield: 27.3%) as a colorless viscous oil. LCMS (ESI) m/z = 285.9 [M+H]⁺.

### Step 3: Synthesis of intermediate 4-67

The intermediate 3-67 crude product (3.0 g, 10.5 mmol) and diethyl malonate (9.2 g, 57.7 mmol) were dissolved in ethanol (20 mL) in a 100 mL three-necked flask. Sodium ethoxide (20%) (17.8 g, 52.5 mmol) was added dropwise in an ice water bath, and reacted at 95°C for 16 h after dropwise adding completion. Most of the solvent was removed. Ice water (100 mL) was added. The mixture was extracted with EA, washed with saturated saline solution (200 mL), and dried with anhydrous sodium sulfate. The mixture was concentrated to obtain a crude product, which was separated by silica gel Flash (methanol/DCM=15/85) to obtain an intermediate 4-67 (3.2 g, yield: 86.3%) as a white solid. LCMS (ESI) m/z = 354.0 [M+H]⁺.

### Step 4: Synthesis of intermediate 5-67

The intermediate 4-67 (3.2 g, 9.1 mmol) was dissolved in a 15% sodium hydroxide aqueous solution (30 mL) in a 100 mL single-necked flask, and reacted at 105°C for 5 h. The system was cooled to 0°C, adjusted to pH 5 with 4 M hydrochloric acid, and filtered to obtain an intermediate 5-67 (1.5 g, yield: 58.68%) as a white solid. LCMS (ESI) m/z = 282.1 [M+H]⁺.

### Step 5: Synthesis of intermediate 6-67

The intermediate 5-67 (1.0 g, 3.56 mmol) and potassium carbonate (982 mg, 7.12 mmol) were dissolved in DMF (5 mL) in a 50 mL single-necked flask. Iodoethane (833 mg, 5.34 mmol) was added dropwise and reacted at 40°C for 16 h after adding completion. Water (20 mL) was added to quench the reaction. The mixture was extracted with EA, washed with saturated saline solution (50 mL), and dried with anhydrous sodium sulfate. The mixture was concentrated to obtain a crude product, which was separated by silica gel Flash (methanol/DCM=7/93) to obtain an intermediate 6-67 (310 mg, yield: 28.18%). ₁H NMR (400 MHz, DMSO) δ 11.72 (s, 1H), 8.14 (s, 1H), 5.71 (s, 1H), 5.59 (s, 2H), 4.21 (q, J = 6.8 Hz, 2H), 3.59 (t, J = 7.8 Hz, 2H), 1.46 (t, J = 6.8 Hz, 3H), 0.91 (t, J = 8.0 Hz, 2H), -0.00 (s, 9H).

### Step 6: Synthesis of intermediate 7-67

The intermediate 6-67 (280 mg, 0.906 mmol), 5-bromo-2-methylindazole (285 mg, 1.35 mmol), (1R,2R)-dimethylcyclohexane-1,2-diamine (128.6 mg, 0.906 mmol), cuprous iodide (172 mg, 0.906 mmol), and potassium phosphate (384 mg, 1.81 mmol) were dissolved in DMSO (5 mL) in a 100 mL single-necked flask, and reacted at 110°C under nitrogen protection for 16 h. Water was added to quench the reaction. The mixture was extracted with EA, washed with saturated saline solution, and dried with anhydrous sodium sulfate. The mixture was concentrated, and purified by silica gel Flash (methanol/DCM=6/94) to obtain an intermediate 7-67 (190 mg, yield: 47.77%) as a pale yellow solid. LCMS (ESI) m/z = 440.1 [M+H]⁺.

### Step 7: Synthesis of intermediate 8-67

The intermediate 7-67 (170 mg, 0.36 mmol) was dissolved in THF (3 mL) in a 25 mL single-necked flask, then added with NBS (61.6 mmol, 0.36 mmol) in batches, and reacted at room temperature for 1 h. A saturated sodium thiosulfate solution was added to quench the reaction. The mixture was extracted with EA, washed with saturated saline solution, and dried with anhydrous sodium sulfate. The mixture was concentrated to obtain a crude product, which was purified by silica gel Flash (methanol/DCM=5/95) to obtain an intermediate 8-67 (120 mg, yield: 64.44%) as a pale yellow solid. LCMS (ESI) m/z = 518.0 [M+H]⁺.

### Step 8: Synthesis of intermediate 9-67

The intermediate 8-67 (50 mg, 0.0967 mmol), 4-chlorophenylboronic acid pinacol ester (46 mg, 0.193 mmol), Pd(dppf)Cl₂ (8.1 mg, 0.00967 mmol), potassium carbonate (26.6 mg, 0.193 mmol), and 1,4-dioxane/water=5/1 (2 mL) were sequentially added into a 25 mL single-necked flask, and reacted at 100°C under nitrogen protection for 16 h. Water was added to quench the reaction. The mixture was extracted with DCM, washed with saturated saline solution, and dried with anhydrous sodium sulfate. The mixture was concentrated to obtain a crude product, which was purified by pre-TLC (methanol/DCM=1/10) to obtain an intermediate 9-67 (50 mg, yield: 94.18%) as a white solid. LCMS (ESI) m/z = 550.1 [M+H]⁺.

### Step 9: Synthesis of compound 67

The intermediate 9-67 (50 mg, 0.091 mmol) was dissolved in hydrogen chloride/1,4-dioxane solution (4 M, 3 mL) in a 50 mL single-necked flask, and reacted at room temperature for 4 h. Ice water (10 mL) was added to quench the reaction. The pH of the reaction solution was adjusted to 8-9 with ammonia water. The reaction solution was extracted with DCM/methanol=10/1, washed with saturated saline solution, and dried with anhydrous sodium sulfate. The mixture was concentrated to obtain a crude product, which was separated by pre-TLC (methanol/DCM=1/10), and concentrated and freeze-dried to obtain a compound 67 (12.70 mg, yield 33.30%) as a white solid. ¹H NMR (400 MHz, DMSO) δ 13.06 (s, 1H), 8.42 (s, 1H), 7.92 (s, 1H), 7.73 (s, 1H), 7.67 (d, J = 8.4 Hz, 1H), 7.40 (s, 4H), 7.14 (d, J = 8.4 Hz, 1H), 4.21 (s, 5H), 1.23 (m, 3H). LCMS (ESI) m/z = 420.0 [M+H]⁺.

A compound 44 was prepared using the synthesis method of Example 9.

| Compound | LCMS (ESI) m/z | ¹H NMR (400 MHz) |
|---|---|---|
| **44** | 452.0 [M+H]⁺ | (MeOD) δ 8.32 (s, 1H), 7.85 (s, 1H), 7.81-7.69 (m, 2H), 7.46 (d, *J =* 8.8 Hz, 2H), 7.25 (dd, *J =* 9.2, 2.0 Hz, 1H), 7.16 (d, *J =* 8.8 Hz, 2H), 6.84 (t, *J =* 74.0 Hz, 1H), 4.17 (m, 5H), 1.26 (t, *J =* 7.2 Hz, 3H). |

### Example 10: Synthesis of compound 61

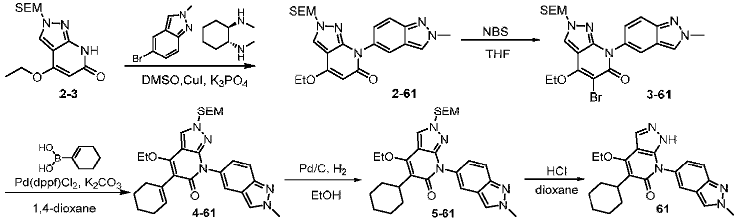

### Step 1: Synthesis of intermediate 2-61

The intermediate 2-3 (6.0 g, 19.4 mmol), 5-bromo-2-methyl-2H-indazole (8.2 g, 38.8 mmol), trans N,N'-dimethylcyclohexanediamine (551 mg, 3.88 mmol), cuprous iodide (739 mg, 3.88 mmol), potassium phosphate (8.2 g, 38.8 mmol), DMSO (80 ml) were sequentially added into a 250 mL three-necked flask, heated up to 120°C under N₂ protection, and reacted overnight. The reaction solution was concentrated to dryness, added with ice water, and extracted with 50 mL (methanol/DCM=1/10). The organic layer was combined, washed sequentially with water and saturated saline solution, dried with anhydrous sodium sulfate, and filtered. The filtrate was concentrated to obtain a crude product, which was purified by a silica gel Flash column (0%-3% methanol/DCM) to obtain an intermediate compound 2-61 (7.0 g, yield: 82.0%) as a white solid. ¹H NMR (400 MHz, DMSO) δ 8.47 (d, J = 3.2 Hz, 2H), 7.75 (d, J = 9.6 Hz, 2H), 7.15 (dd, J₁ = 8.8 Hz, J₂ = 1.6 Hz, 1H), 5.81 (s, 1H), 5.41 (s, 2H), 4.36 -4.22 (m, 5H), 3.58 (t, J = 8.0 Hz, 2H), 1.49 (t, J = 7.2 Hz, 3H), 0.91 - 0.80 (m, 2H), -0.00 (s, 9H). LCMS (ESI) m/z = 440.2 [M+H]⁺.

### Step 2: Synthesis of intermediate 3-61

The intermediate 2-61 (4.0 g, 9.1 mmol) was dissolved in THF (120 mL) in a 25 mL single-necked flask, NBS (1.46 g, 8.2 mmol) was added in batches while stirring at room temperature, and reacted at room temperature for 2 h. The reaction solution was poured into ice water, and extracted with EA (50 mL). The organic layer was combined, dried with anhydrous sodium sulfate, and filtered. The filtrate was concentrated to obtain a crude product, which was purified with a silica gel Flash column (0-100% EA) to obtain an intermediate 3-61 (3.5 g, yield: 74.2%) as a pale yellow solid. LCMS (ESI) m/z = 518 [M+H]⁺.

### Step 3: Synthesis of intermediate 4-61

The intermediate 3-61 (100 mg, 0.19 mmol), cyclohexyl-1-en-1-ylboronic acid (49 mg, 0.38 mmol), Pd(dppf)Cl₂ (30 mg, 0.04 mmol), potassium carbonate (53 mg, 0.38 mmol), and dioxane:water=5:1 (5 mL) were added into a 50 mL single-necked flask, and stirred at 100°C under N₂ protection for 16 h. The reaction solution was filtered and concentrated to obtain a crude product, which was purified by Flash (DCM/MeOH=98/2) to obtain an intermediate 4-61 (80 mg, yield: 79.2%) as a yellow solid. LCMS (ESI) m/z = 520.3 [M+H]⁺.

### Step 4: Synthesis of intermediate 5-61

The intermediate 4-61 (80 mg, 0.15 mmol), Pd/C (10 mg), and EtOH (3 ml) were sequentially added into a 50 ml single-necked flask, and stirred at room temperature under a hydrogen atmosphere for 72 h. The reaction solution was concentrated to obtain an intermediate 5-61 crude product (70 mg, yield: 87.5%) as a yellow solid. LCMS (ESI) m/z = 522.2 [M+H]⁺.

### Step 5: Synthesis of compound 61

The intermediate 5-61 (120 mg, 0.13 mmol) was dissolved in 4 M HCl/1,4-dioxane solution (5 mL) in a 25 mL single-necked flask, and reacted at room temperature for 3 h. The reaction solution was poured into ice water, and the pH was adjusted to 9-10 with ammonia solution. The mixture was extracted with DCM/methanol (10/1). The organic phase was washed once with water and once with saturated saline solution, dried with anhydrous sodium sulfate, and filtered. The filtrate was concentrated under reduced pressure to dryness, purified by prep-HPLC (H₂O/ACN, 0.1%NH₃.H₂O), and freeze dried to obtain a compound 61 (10 mg, yield: 19.2%) as a white solid. ¹H NMR (400 MHz, DMSO) δ 13.14 (s, 1H), 8.38 (s, 2H), 7.63 (d, J = 3.2 Hz, 2H), 7.04 (dd, J₁ = 9.1 Hz, J₂ = 1.7 Hz, 1H), 4.47 (d, J = 6.9 Hz, 2H), 4.21 (s, 3H), 3.09 (t, J = 12.0 Hz, 1H), 2.11 - 2.00 (m, 2H), 1.74 (d, J = 12.3 Hz, 2H), 1.66 (d, J = 11.1 Hz, 1H), 1.47 (d, J = 11.9 Hz, 2H), 1.42 (t, J = 7.0 Hz, 3H), 1.22 (td, J = 25.0, 12.6 Hz, 3H). LCMS (ESI) m/z = 392.0 [M+H]⁺.

### Example 11: Synthesis of compound 63

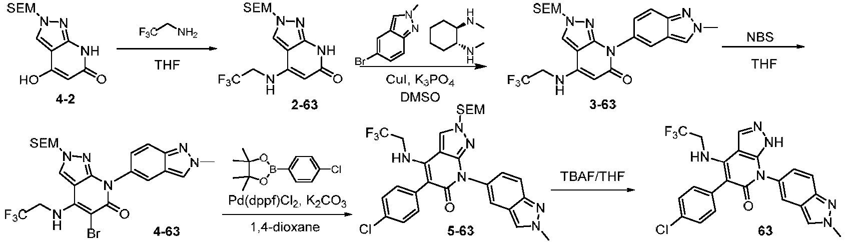

### Step 1: Synthesis of intermediate 2-63

The intermediate 4-2 (1.05 g, 3.39 mmol), 2,2,2-trifluoroethane-1-amine (2 mL), and THF (5 mL) were added into a 50 mL reaction vessel, heated up to 110°C and reacted for 16 h. The reaction solution was concentrated to obtain a crude product, which was purified by a silica gel Flash column (DCM/MeOH=97/3) to obtain an intermediate 2-63 (175 mg, yield: 24.72%) as a yellow solid. LCMS (ESI) m/z = 363.10 [M+H]⁺.

### Step 2: Synthesis of intermediate 3-63

The intermediate 2-63 (175 mg, 0.48 mmol), 5-bromo-2-methylindazole (203 mg, 0.97 mmol), trans N,N'-dimethylcyclohexanediamine (69 mg, 0.48 mmol), cuprous iodide (92 mg, 0.48 mmol), potassium phosphate (205 mg, 0.97 mmol), DMSO (3 ml) were sequentially added into a 25 mL single-necked flask, and reacted at 110°C under N₂ protection for 16 h. The reaction solution was concentrated to obtain a crude product, which was purified by a silica gel Flash column (DCM/MeOH=97/3) to obtain an intermediate 3-63 (200 mg, yield: 84.03%) as a black solid. LCMS (ESI) m/z = 493.10 [M+H]⁺.

### Step 3: Synthesis of intermediate 4-63

The intermediate 3-63 (200 mg, 0.41 mmol) was dissolved in THF (5 mL) in a 25 mL single-necked flask, then added with NBS (72 mmol, 0.41 mmol) in batches after dissolution, and reacted at room temperature for 2 h. The reaction solution was poured into a saturated sodium sulfite solution, and extracted with DCM. The organic phase was concentrated. The crude product was purified by a silica gel Flash column (DCM/MeOH=96/4) to obtain an intermediate 4-63 (100 mg, yield: 43.10%) as a yellow solid. LCMS (ESI) m/z = 571.00 [M+H]⁺.

### Step 4: Synthesis of intermediate 5-63

The intermediate 4-63 (100 mg, 0.18 mmol), 4-chlorophenylboronic acid pinacol ester (83 mg, 0.35 mmol), Pd(dppf)Cl₂ (14 mg, 0.02 mmol), potassium carbonate (48 mg, 0.35 mmol), 1,4-dioxane (3 ml), and water (0.6 ml) were sequentially added into a 25 mL single-necked flask, and reacted at 110°C under N₂ protection for 16 h. The reaction solution was filtered. The filtrate was collected, and concentrated to obtain a crude product, which was purified by a silica gel Flash column (0%-3% methanol/DCM) to obtain a yellow solid intermediate 5-63 (40 mg, yield: 37.74%). LCMS (ESI) m/z = 603.15 [M+H]⁺.

### Step 5: Synthesis of compound 63

The intermediate 5-63 (40 mg, 0.07 mmol) was dissolved in TBAF tetrahydrofuran solution (6 mL) in a 25 mL single-necked flask, and performed a reflux reaction at 60°C for 2 h. The reaction solution was concentrated, EA was added, and TBAF was removed by washing with water. The organic phase was concentrated to obtain a crude product, which was purified by pre-TLC (DCM/MeOH=12/1), and freeze dried to obtain an end product compound 63 (7.25 mg, yield: 23.39%) as a white solid. ¹H NMR (400 MHz, DMSO) δ 13.18 (s, 1H), 8.52 (s, 1H), 8.37 (s, 1H), 7.66 (s, 1H), 7.61 (d, J = 8.9 Hz, 1H), 7.47 (d, J = 8.5 Hz, 2H), 7.25 (d, J = 8.5 Hz, 2H), 7.09 (dd, J = 9.1, 1.7 Hz, 1H), 5.98 (t, J = 7.5 Hz, 1H), 4.20 (s, 3H), 4.17 - 4.05 (m, 2H). LCMS (ESI) m/z = 473.05 [M+H]⁺.

Compounds **105-108, 123 and 129** were prepared using the synthesis method of Example 11.

| Compound | LCMS (ESI) m/z | ¹H NMR (400 MHz) |
|---|---|---|
| **105** | 419.95 [M+H]⁺ | (DMSO) δ 13.10 (s, 1H), 8.63 (s, 1H), 8.41 (s, 1H), 8.15 (d, *J* = 9.1 Hz, 1H), 7.43 (d, *J =* 8.4 Hz, 2H), 7.27 (d, *J =* 8.4 Hz, 2H), 7.18 (d, *J* = 8.9 Hz, 1H), 5.72 (t, *J =* 6.1 Hz, 1H), 4.25 (s, 3H), 3.37 (dd, *J* = 14.0, 7.3 Hz, 2H), 1.10 (t, *J =* 7.0 Hz, 3H). |
| **106** | 454.10 [M+H]⁺ | (DMSO) δ 13.17 (s, 1H), 8.62 (d, *J =* 1.4 Hz, 1H), 8.41 (d, *J =* 37.3 Hz, 2H), 7.95 (dd, *J* = 8.0, 1.6 Hz, 1H), 7.87 (d, *J* = 8.1 Hz, 1H), 7.66-7.57 (m, 2H), 7.08 (dd, *J =* 9.0, 1.8 Hz, 1H), 6.28 (s, 1H), 4.19 (s, 3H), 3.48 - 3.34 (m, 2H), 1.14 (t, *J =* 7.0 Hz, 3H). |
| **107** | 446.20 [M+H]⁺ | (DMSO) δ 13.05 (s, 1H), 8.64 (s, 1H), 8.39 (s, 1H), 8.16 (d, *J =* 8.8 Hz, 1H), 7.48-7.42 (m, 2H), 7.32-7.28 (m, 2H), 7.22 (d, *J =* 8.8 Hz, 1H), 5.61 (s, 1H), 4.25 (s, 3H), 3.27 (t, *J* = 6.0 Hz, 2H), 1.03-0.95 (m, 1H), 0.46 - 0.40 (m, 2H), 0.23-0.18 (m, 2H). |
| **108** | 480.20 [M+H]⁺ | (DMSO) δ 13.17 (s, 1H), 8.64 (s, 1H), 8.48 (s, 1H), 8.36 (s, 1H), 7.97 (d, *J* = 9.6 Hz, 1H), 7.89 (d, *J* = 8.1 Hz, 1H), 7.69-7.55 (m, 2H),7.09 (dd, *J* = 9.1, 1.8 Hz, 1H), 6.22 (s, 1H), 4.19 (s, 3H), 3.29-3.24 (m, 2H), 1.13-0.99 (m, 1H), 0.49-0.40 (m, 2H), 0.26-0.18 (m, 2H). |
| **123** | 445.1 [M+H]⁺ | (DMSO) δ 13.09 (s, 1H), 8.42 (s, 1H), 8.36 (s, 1H), 7.68 - 7.56 (m, 2H), 7.47-7.41 (m, 2H), 7.33-7.27 (m, 2H), 7.07 (dd, *J =* 9.2, 2.0 Hz, 1H), 5.47 (s, 1H), 4.19 (s, 3H), 3.25 (t, *J =* 6.0 Hz, 2H), 1.08 - 0.97 (m, 1H), 0.46 - 0.39 (m, 2H), 0.23 - 0.17 (m, 2H). |
| **129** | 452.05 [M+H]⁺ | (DMSO) δ 13.09 (s, 1H), 8.64 (s, 1H), 8.41 (s, 1H), 8.16 (d, *J=* 9.1 Hz, 1H), 7.33- 7.28 (m, 2H), 7.27 (t, *J* = 76.0 Hz, 1H), 7.18 (d, *J* = 8.6 Hz, 3H), 5.61 (s, 1H), 4.25 (s, 3H), 3.36 (q, *J =* 7.5 Hz, 2H), 1.10 (t, *J =* 7.0 Hz, 3H). |

### Example 12: Synthesis of compound 64

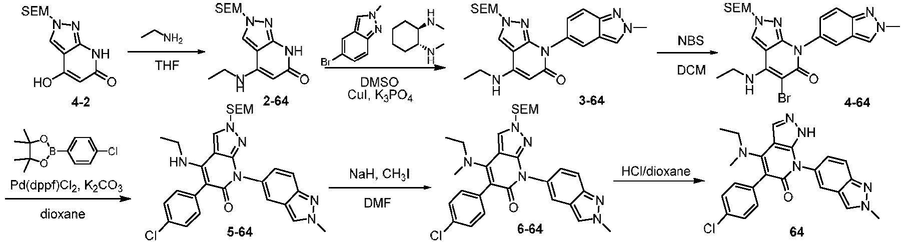

### Step 1: Synthesis of intermediate 2-64

The intermediate 4-2 (1.0 g, 3.55 mmol) was dissolved in tetrahydrofuran solution of ethylamine (12 mL) in a 50 mL reactor, and reacted at 110°C for 16 h. The reaction solution was added with water, and extracted with EA. The organic phase was concentrated to obtain a crude product, which was purified by a silica gel Flash column (DCM/MeOH=40/1) to obtain an intermediate 2-64 (550 mg, yield: 50.0%) as a white solid. ¹H NMR (400 MHz, DMSO) δ 10.64 (s, 1H), 8.21 (s, 1H), 6.90 (t, J = 5.0 Hz, 1H), 5.44 (s, 2H), 4.89 (s, 1H), 3.64 - 3.50 (m, 2H), 3.16 (dd, J = 7.1, 5.2 Hz, 2H), 1.24 (t, J = 7.2 Hz, 3H), 0.94 - 0.83 (m, 2H), -0.00 (s, 9H).

### Step 2: Synthesis of intermediate 3-64

The intermediate 2-64 (550 mg, 1.78 mmol), 5-bromo-2-methylindazole (753 mg, 3.57 mmol), trans N,N'-dimethylcyclohexanediamine (254 mg, 1.78 mmol), cuprous iodide (340 mg, 1.78 mmol), potassium phosphate (757 mg, 3.57 mmol), DMSO (4 ml) were sequentially added into a 25 mL single-necked flask, and reacted at 110°C under N₂ protection for 16 h. The reaction solution was concentrated to obtain a crude product, which was purified by a silica gel Flash column (DCM/MeOH=97/3) to obtain an intermediate 3-64 (800 mg, yield: 100 %) as a black solid. LCMS (ESI) m/z = 439.10 [M+H]⁺.

### Step 3: Synthesis of intermediate 4-64

The intermediate 3-64 (800 mg, 1.82 mmol) was dissolved in THF (10 mL) in a 50 mL single-necked flask, then added with NBS (292 mg, 1.64 mmol) in batches after dissolution, and reacted at room temperature for 2 h. The reaction solution was poured into a saturated sodium sulfite solution, and extracted with DCM. The organic phase was concentrated. The crude product was purified by a silica gel Flash column (DCM/MeOH=96/4) to obtain an intermediate 4-64 (700 mg, yield: 74.23 %) as a yellow solid. LCMS (ESI) m/z = 518.05 [M+H]⁺.

### Step 4: Synthesis of intermediate 5-64

The intermediate 4-64 (350 mg, 0.68 mmol), 4-chlorophenylboronic acid pinacol ester (323 mg, 1.35 mmol), Pd(dppf)Cl₂ (56 mg, 0.07 mmol), potassium carbonate (189 mg, 1.35 mmol), 1,4-dioxane (5 ml), and water (1 ml) were sequentially added into a 25 mL single-necked flask, and reacted at 100°C under N₂ protection for 16 h. The reaction solution was filtered, and the filtrate was concentrated to obtain a crude product, which was purified by a silica gel Flash column (DCM/MeOH=97/3) to obtain an intermediate 5-64 (260 mg, yield: 70.08%) as a yellow solid. ¹H NMR (400 MHz, DMSO) δ 8.68 (s, 1H), 8.41 (s, 1H), 7.76 - 7.56 (m, 2H), 7.49 (dd, J = 8.8, 2.1 Hz, 2H), 7.40 - 7.24 (m, 2H), 7.11 (dd, J = 9.0, 2.0 Hz, 1H), 5.80 (t, J = 6.2 Hz, 1H), 5.43 (s, 2H), 4.25 (s, 3H), 3.76 - 3.52 (m, 2H), 1.17 (t, J = 7.0 Hz, 3H), 0.93 - 0.84 (m, 2H), 0.06 (s, 2H), 0.01 (s, 9H).

### Step 5: Synthesis of intermediate 6-64

The intermediate 5-64 (109 mg, 0.20 mmol) and solvent DMF (5 mL) were added into a 25 mL three-necked flask, cooled to 0°C under N₂ protection, added with NaH (24 mg, 0.60 mmol), stirred for about 10 min, added with CH₃I (56 mg, 0.40 mmol), and stirred for 20 min. The reaction solution was quenched with water, extracted with EA, and the organic phase was concentrated to obtain an intermediate 6-64 crude product (109 mg, purity: 83%, yield: 80.78%) as a gray solid. LCMS (ESI) m/z = 562.95 [M+H]⁺.

### Step 6: Synthesis of compound 64

The intermediate 9 (109 mg, 0.19 mmol) was dissolved in HCl/1,4-dioxane solution (10 mL) in a 25 mL single-necked flask, and reacted at room temperature for 2 h. The reaction solution was poured into ice water, and the pH was adjusted to 9-10 with ammonia solution. The mixture was extracted with DCM/methanol (10/1). The organic phase was washed once with water and once with saturated saline solution, dried with anhydrous sodium sulfate, and filtered. The filtrate was concentrated under reduced pressure to obtain a crude product, which was purified by silica gel Flash (DCM/MeOH=97/3), and freeze dried to obtain a compound 64 (14.95 mg, yield: 16.65%) as a white solid. ¹H NMR (400 MHz, DMSO) δ 13.11 (s, 1H), 8.36 (s, 1H), 8.32 (s, 1H), 7.68 - 7.56 (m, 2H), 7.41 - 7.34 (m, 2H), 7.33 - 7.27 (m, 2H), 7.07 (dd, J = 9.1, 1.5 Hz, 1H), 4.19 (s, 3H), 3.16 (q, J = 6.6 Hz, 2H), 2.71 (s, 3H), 1.00 (t, J = 7.0 Hz, 3H).LCMS (ESI) m/z = 433.10 [M+H]⁺.

### Example 13: Synthesis of compound 112

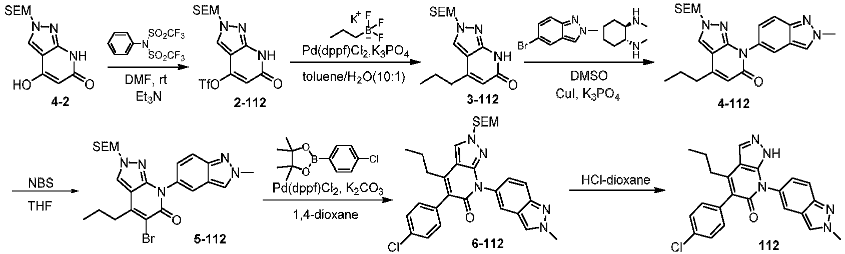

### Step 1: Synthesis of intermediate 2-112

The intermediate 4-2 (9.0 g, 32.0 mmol), N-phenyl-bis(trifluoromethanesulfonimide) (13.7 g, 38.4 mmol), and TEA (9.7 g, 96 mmol) were dissolved in DMF (50 mL) in a 100 mL single-necked flask. The mixture was reacted at room temperature under nitrogen protection for 16 h. Ice water (50 mL) was added to quench the reaction. The mixture was extracted with EA, washed with saturated saline solution, and dried with anhydrous sodium sulfate. The mixture was concentrated to obtain a crude product, which was purified by silica gel Flash (PE/EA=2/1) to obtain an intermediate 2-112 (2.6 g, yield 19.66%) as a white solid. LCMS (ESI) m/z = 414.0 [M+H]⁺.

### Step 2: Synthesis of intermediate 3-112

The intermediate 2-112 (500 mg, 1.2 mmol), potassium n-propyltrifluoroborate (259 mg, 2.4 mmol), Pd(dppf)Cl₂ (101 mg, 0.12 mmol), and potassium phosphate (770 mg, 3.6 mmol) were sequentially added in a 100 mL single-necked flask. A toluene/water mixed solvent (10 mL, 10/1) was added, and reacted at 90°C under nitrogen protection for 16 h. Water (20 mL) was added to quench the reaction. The mixture was extracted with DCM, washed with saturated saline solution, and dried with anhydrous sodium sulfate. The mixture was concentrated to obtain a crude product, which was purified by silica gel Flash (DCM/methanol=98/2) to obtain a product intermediate 3-112 (180 mg, yield: 48.45%) as a white solid. LCMS (ESI) m/z = 308.0 [M+H]⁺.

### Step 3: Synthesis of intermediate 4-112

The intermediate 3-112 (160 mg, 0.52 mmol), 5-bromo-2-methylindazole (163 mg, 0.78 mmol), (1R,2R)-dimethylcyclohexane-1,2-diamine (73.8 mg, 0.52 mmol), cuprous iodide (99 mg, 0.52 mmol), and potassium phosphate (220 mg, 1.04 mmol) were sequentially added in a 100 mL single-necked flask. Dimethyl sulfoxide (4 mL) was added and reacted at 110°C under nitrogen protection for 16 h. Water (20 mL) was added to quench the reaction. The mixture was extracted with EA (30 mL/time), washed with saturated saline solution, and dried with anhydrous sodium sulfate. The mixture was concentrated to obtain a crude product, which was separated and purified by silica gel Flash (methanol/DCM=3/97) to obtain an intermediate 4-112 (160 mg, yield: 70.41%) as a black solid. LCMS (ESI) m/z = 438.1 [M+H]⁺.

### Step 4: Synthesis of intermediate 5-112

The intermediate 4-112 (140 mg, 0.32 mmol) was dissolved in THF (4 mL) in a 100 mL three-necked flask, NBS (62.7 mmol, 0.35 mmol) was added in batches under nitrogen protection, and reacted at room temperature for 2 h. A saturated sodium thiosulfate solution was added to quench the reaction (10 mL). The mixture was extracted with EA, washed with saturated saline solution, and dried with anhydrous sodium sulfate. The mixture was concentrated to obtain a crude product, which was purified by silica gel Flash (PE/EA=65/35) to obtain a product intermediate 5-112 (120 mg, yield: 72.81%) as a colorless viscous solid. LCMS (ESI) m/z = 516.1 [M+H]⁺.

### Step 5: Synthesis of intermediate 6-112

The intermediate 5-112 (100 mg, 0.19 mmol), 2-(4-chlorophenyl)-4,4,5,5-tetramethyl-1,3,2-dioxacycloborane (92 mg, 0.39 mmol), Pd(dppf)Cl₂ (16 mg, 0.019 mmol), potassium carbonate (53 mg, 0.39 mmol), and 1,4-dioxane/water=5/1 (2 mL) were sequentially added into a 25 mL single-necked flask, and reacted at 100°C under nitrogen protection for 16 h. Water was added to quench the reaction. The mixture was extracted with DCM, washed with saturated saline solution, and dried with anhydrous sodium sulfate. The mixture was concentrated to obtain a crude product, which was purified by silica gel Flash (methanol/DCM=3/97) to obtain an intermediate 6-112 (120 mg, yield: 94.15%) as a white solid. LCMS (ESI) m/z = 548.1 [M+H]⁺.

### Step 6: Synthesis of compound 112

The intermediate 6-112 (110 mg, 0.20 mmol) was added into a 25 mL single-necked flask, hydrochloric acid-1,4-dioxacyclo solution (4 M, 4 mL) was added under nitrogen protection, and reacted at room temperature for 4 h. Ice water (10 mL) was added to quench the reaction. The reaction solution was adjusted to pH=8-9 with ammonia water, extracted with DCM/methanol=10/1, washed with saturated saline solution, and dried with anhydrous sodium sulfate. The mixture was concentrated to obtain a crude product, which was separated and purified by prep-HPLC (water/acetonitrile, 0.1% NH₃H₂O) to obtain an end product 112 (41.73 mg, yield: 50.03%) as a white solid. ¹H NMR (400 MHz, DMSO) δ 13.14 (s, 1H), 8.38 (d, J = 5.6 Hz, 2H), 7.73 (s, 1H), 7.65 (d, J = 9.2 Hz, 1H), 7.46 (d, J = 8.4 Hz, 2H), 7.27 (d, J = 8.4 Hz, 2H), 7.14 (dd, J = 9.2, 2.0 Hz, 1H), 4.20 (s, 3H), 2.53 (m, 2H), 1.64 - 1.51 (m, 2H), 0.84 (t, J = 7.2 Hz, 3H). LCMS (ESI) m/z = 418.1 [M+H]⁺.

### Example 14: Synthesis of compound 122

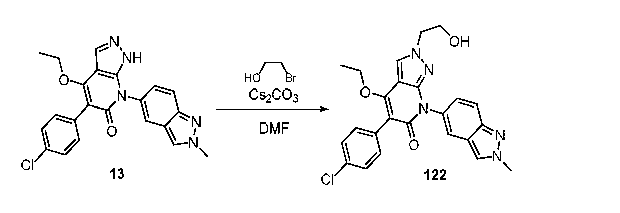

The compound 13 (110 mg, 0.26 mmol), cesium carbonate (169 mg, 0.52 mmol), and 2-bromo-1-ol (40 mg, 0.31 mmol) were dissolved in DMF (3 mL) in a 50 mL single-necked flask, and react at 80°C for 3 h. The reaction solution was filtered, purified by prep-HPLC (H₂O/ACN, 0.1%FA), and freeze-dried to obtain a compound 122 (21 mg, yield: 17.4%) as a white solid. ¹H NMR (400 MHz, DMSO) δ 8.54 (s, 1H), 8.39 (s, 1H), 7.69 (d, J = 1.3 Hz, 1H), 7.66 (d, J = 9.1 Hz, 1H), 7.38 (s, 4H), 7.10 (dd, J = 9.1, 1.9 Hz, 1H), 4.92 (s, 1H), 4.43 (q, J = 6.9 Hz, 2H), 4.20 (s, 3H), 4.12 (t, J = 5.3 Hz, 2H), 3.67 (t, J = 5.1 Hz, 2H), 1.25 (t, J = 7.0 Hz, 3H). LCMS (ESI) m/z = 464.05 [M+H]⁺.

Compounds **134, 136, 138 and 140** were prepared using the synthesis method of Example 14.

| Compound | LCMS (ESI) m/z | ¹H NMR (400 MHz) |
|---|---|---|
| **134** | 434.0 [M+H]⁺ | (DMSO) δ 8.58 (s, 1H), 8.40 (s, 1H), 7.67 (dd, *J =* 12.0, 5.2 Hz, 2H), 7.38 (s, 4H), 7.09 (dd, *J =* 9.2, 2.0 Hz, 1H), 4.43 (q, *J =* 7.2 Hz, 2H), 4.20 (s, 3H), 3.83 (s, 3H), 1.25 (t, *J =* 7.2 Hz, 3H). |
| **136** | 435.10 [M+H]⁺ | (DMSO) δ 8.61 (s, 1H), 8.00 (d, *J =* 9.0 Hz, 1H), 7.96 (s, 1H), 7.39 (s, 4H), 7.35 (dd, *J =* 9.0, 1.8 Hz, 1H), 4.54 (s, 3H), 4.44 (q, *J =* 7.0 Hz, 2H), 3.84 (s, 3H), 1.26 (t, *J =* 7.0 Hz, 3H). |
| **138** | 436.10 [M+H]⁺ | (DMSO) δ 8.63 (d, *J =* 8.1 Hz, 2H), 7.56 (d, *J =* 8.8 Hz, 1H), 7.39 (s, 4H), 4.60 (s, 3H), 4.47 (q, *J* =6.9 Hz, 2H), 3.84 (s, 3H), 1.27 (t, *J* =7.0 Hz, 3H). |
| **140** | 435.10 [M+H]⁺ | (DMSO) δ 8.63 (s, 1H), 8.14 (d, *J =* 8.8 Hz, 1H), 7.99 (d, *J =* 1.2 Hz, 1H), 7.38 (d, *J =* 2.0 Hz, 4H), 7.35 (dd, *J*= 8.8,1.8 Hz, 1H), 4.45 (q, *J* = 7.0 Hz, 2H), 4.31 (d, *J =* 2.1 Hz, 3H), 3.84 (s, 3H), 1.26 (dd, *J* = 8.0, 5.9 Hz, 3H). |

### Example 15: Synthesis of compounds 109 and 121

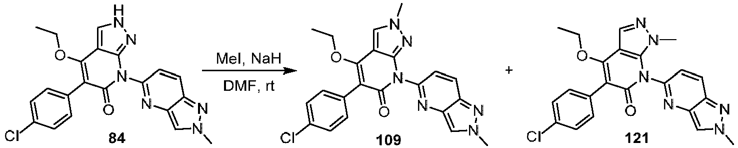

The compound 84 (120 mg, 0.28 mmol) was dissolved in 5 mL of DMF in a 25 mL single-necked flask, NaH (18 mg, 0.42 mmol) was added in an ice bath, stirred for 10 min, then iodomethane (48 mg, 0.34 mmol) was added, and stirred for 1 h. The reaction solution was poured into water, and extracted with EA. The organic phase was washed with water, washed with saturated saline solution, and concentrated to obtain a crude product, which was purified by pre-HPLC (water/acetonitrile, 0.1% FA), and freeze dried to obtain a compound 109 (34.96 mg, yield: 36.9%) as a white solid, and a compound 121 (16.86 mg, yield: 17.8%) as a white solid.

Compound **109:** ¹H NMR (400 MHz, DMSO) δ 8.68 (s, 1H), 8.60 (s, 1H), 8.23 (dd, *J =* 8.8, 0.8 Hz, 1H), 7.41 - 7.35 (m, 4H), 7.25 (d, *J =* 8.9 Hz, 1H), 4.45 (q, *J =* 7.2 Hz, 2H), 4.26 (s, 3H), 3.83 (s, 3H), 1.26 (t, *J =* 7.2 Hz, 3H). LCMS (ESI) m/z = 435.1 [M+H]⁺.

Compound **121:** ¹H NMR (400 MHz, DMSO) δ 8.79 (s, 1H), 8.33 (d, *J* = 8.8 Hz, 1H), 8.19 (s, 1H), 7.51 (d, *J =* 8.8 Hz, 1H), 7.41 - 7.35 (m, 4H), 4.51 (q, *J =* 6.8 Hz, 2H), 4.28 (s, 3H), 2.97 (s, 3H), 1.26 (t, *J =* 6.8 Hz, 3H). LCMS (ESI) m/z = 435.1 [M+H]⁺.

### Example 16: Synthesis of compound 130

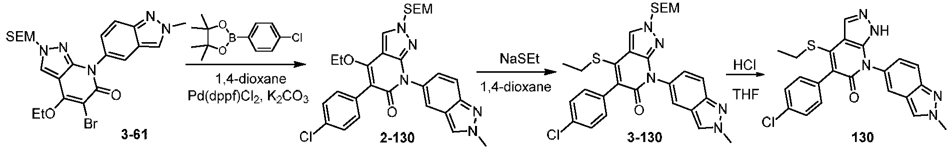

### Step 1: Synthesis of intermediate 2-130

The compound 3-61 (3.0 g, 5.8 mmol), 4-chlorophenylboronic acid pinacol ester (2.8 g, 11.6 mmol), Pd(dppf)Cl₂ (0.47 g, 0.58 mmol), potassium carbonate (1.6 g, 11.6 mmol), 1,4-dioxane (45 mL), and water (9 ml) were sequentially added into a 100 mL single-necked flask, and reacted overnight at 110°C under N₂ protection. The reaction solution was filtered, and the filter cake was rinsed with DCM. The filtrate was combined, and concentrated to obtain a crude product, which was purified by a silica gel Flash column (0%-2% methanol/DCM) to obtain an intermediate 2-130 (3.0 g, yield: 94.1%) as a pale yellow solid. LCMS (ESI) m/z = 550.1 [M+H]⁺.

### Step 2: Synthesis of intermediate 3-130

The intermediate 2-130 (550 mg, 1.0 mmol), sodium ethanethiol (84 mg, 10 mmol), and 1,4-dioxane (25 mL) were sequentially added into a 50 mL three-necked flask, and reacted at 70°C under N₂ protection for 16 h. The reaction solution was poured into water (50 mL), and extracted with EA. The organic layer was combined, dried with anhydrous sodium sulfate, and filtered. The filtrate was concentrated to obtain a crude product, which was purified by silica gel column chromatography (0%-2% methanol/DCM) to obtain an intermediate 3-130 (350 mg, yield: 61.8%) as a pale yellow solid. LCMS (ESI) m/z =566.2 [M+H]⁺.

### Step 3: Synthesis of compound 130

The intermediate 3-130 (350 mg, 0.62 mmol) was dissolved in 4 M HCl/1,4-dioxane (16 mL) in a 50 mL three-necked flask, and reacted at room temperature for 4 h. The reaction solution was poured into ice water (50 mL), adjusted to pH 9-10 with ammonia water, stirred at room temperature for 1 h, and extracted with EA. The organic layer was combined, dried with anhydrous sodium sulfate, and filtered. The filtrate was concentrated to obtain a crude product, which was purified by prep-HPLC (water/acetonitrile, 0.1% NH₃H₂O) to obtain a product compound 130 (33.90 mg, yield: 12.7%) as a white solid. ¹HNMR (400 MHz, DMSO) δ 13.25 (s, 1H), 8.40 (s, 2H), 7.75 (s, 1H), 7.66 (d, J = 9.2 Hz, 1H), 7.47 - 7.41 (m, 2H), 7.34 - 7.26 (m, 2H), 7.15 (dd, J₁ =9.2 Hz, J₂ = 2.0 Hz, 1H), 4.20 (s, 3H), 2.99 (q, J = 7.2 Hz, 2H), 1.12 (t, J = 7.2 Hz, 3H). LCMS (ESI) m/z = 436.1[M+H]⁺.

### Example 17: Synthesis of compound 55

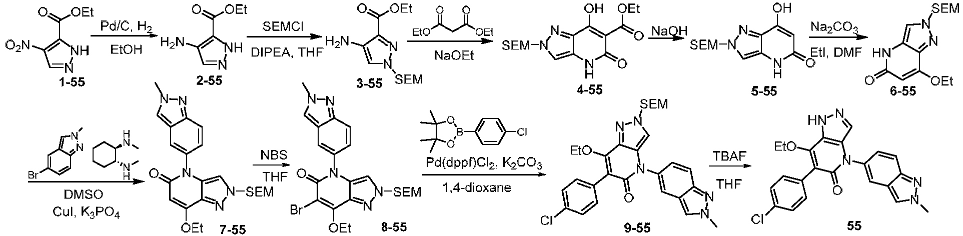

### Step 1: Synthesis of intermediate 2-55

4-nitropyrazole-5-ethyl carboxylate 1-55 (10 g, 54.05 mmol) was dissolved in ethanol (100 mL) in a 250 mL single-necked flask, added with 1 g of wet palladium carbon, and stirred overnight at room temperature under a hydrogen atmosphere. The reaction solution was filtered, and the filtrate was concentrated to obtain an intermediate 2-55 (8.7 g, yield>100%, including a solvent) as a purple solid. ¹H NMR (400 MHz, DMSO) δ 12.80 (s, 1H), 7.09 (s, 1H), 4.78 (s, 2H), 4.25 (q, J = 7.1 Hz, 2H), 1.29 (t, J = 7.1 Hz, 3H).

### Step 2: Synthesis of intermediate 3-55

The intermediate 2-55 (8.66 g, 55.9 mmol) was dissolved in THF (200 mL) in a 500 mL single-necked flask, cooled to 0°C in an ice bath, and added with DIPEA (14.42 g, 111.8 mmol). Then, SEMCl (10.27 g, 61.49 mmol) was added dropwise, and stirred overnight at room temperature after dropwise adding completion. SEMCl (5 g, 30 mmol) was added, and stirred at room temperature for 16 h after adding completion. The reaction solution was poured into water, extracted with EA, dried with anhydrous sodium sulfate, and filtered. The filtrate was concentrated to obtain 16.3 g of an intermediate 3-55 crude product as a pale yellow oil, which did not require purification to proceed directly to the next step. LCMS (ESI) m/z = 285.9 [M+H]⁺.

### Step 3: Synthesis of intermediate 4-55

The intermediate 3-55 (16.16 g, 56.7 mmol) was dissolved in ethanol (250 mL) in a 500 mL single-necked flask, diethyl malonate (54.4 g, 340 mmol) and an ethanol solution of 20% sodium ethoxide (115.7 g, 340 mmol) were added, and refluxed at 85°C for 18 h after adding completion. The reaction solution was concentrated under reduced pressure, added with water, extracted with EA, dried with anhydrous sodium sulfate, and filtered. The filtrate was concentrated to obtain 8 g of an intermediate 4-55 crude product as a yellow oil, which did not require purification to proceed directly to the next step. LCMS (ESI) m/z = 354.1 [M+H]⁺.

### Step 4: Synthesis of intermediate 5-55

The intermediate 4-55 (7.5 g, 21.22 mmol) was added to a 15% NaOH solution in a 250 mL single-necked flask, heated up to 105°C, and performed a reflux reaction for 6 h. The reaction solution was adjusted to pH around 4 with 2 M hydrochloric acid, and a solid was precipitated. The mixture was filtered to obtain an intermediate 5-55 (6.1 g, yield: 95.31%) as a yellow solid. LCMS (ESI) m/z = 282.10 [M+H]⁺.

### Step 5: Synthesis of intermediate 6-55

The intermediate 5-55 (3.3 g, 11.73 mmol) was dissolved in DMF (60 mL) in a 100 mL single-necked flask, Na₂CO₃ (2.49 g, 23.45 mmol) was added, iodoethane (3.66 g, 23.45 mmol) was added dropwise, heated up to 40°C after dropwise adding completion, and stirred for 3 h. The reaction solution was poured into water, and extracted with EA. The organic phase was washed with water, washed with saturated saline solution, dried, and filtered. The filtrate was concentrated to obtain a crude product, which was purified by silica gel Flash (DCM/MeOH=96/4) to obtain an intermediate 6-55 (1 g, yield: 27.55%) as a white solid. LCMS (ESI) m/z = 310.15 [M+H]⁺.

### Step 6: Synthesis of intermediate 7-55

The intermediate 6-55 (1.05 g, 3.39 mmol), 5-bromo-2-methylindazole (1.43 g, 6.79 mmol), trans N,N'-dimethylcyclohexanediamine (483 mg, 3.39 mmol), cuprous iodide (646 mg, 6.79 mmol), potassium phosphate (1.44 g, 3.39 mmol), and DMSO (12 ml) were added into a 100 mL single-necked flask, and reacted at 110°C under N₂ protection for 16 h. The reaction solution was concentrated to obtain a crude product, which was purified by a silica gel Flash column (DCM/MeOH=96/4) to obtain an intermediate 7-55 (1.2 g, yield: 80.54 %) as a black oil. LCMS (ESI) m/z = 440.15 [M+H]⁺.

### Step 7: Synthesis of intermediate 8-55

The intermediate 7-55 (1.2 g, 2.72 mmol) was dissolved in THF in a 25 mL single-necked flask, then added with NBS (437 mg, 2.46 mmol) in batches after dissolution, and reacted at room temperature for 10 h. The reaction solution was poured into a sodium sulfite solution, and extracted with DCM. The organic phase was concentrated, and purified by a silica gel Flash column (DCM/MeOH=97/3) to obtain an intermediate 8-55 (400 mg, yield: 28.5%) as a yellow solid. LCMS (ESI) m/z = 518.05 [M+H]⁺.

### Step 8: Synthesis of intermediate 9-55

The intermediate 8-55 (250 mg, 0.48 mmol), 4-chlorophenylboronic acid pinacol ester (230 mg, 0.96 mmol), Pd(dppf)Cl₂ (39 mg, 0.05 mmol), potassium carbonate (133 mg, 0.96 mmol), 1,4-dioxane (5 ml), and water (1 ml) were sequentially added into a 50 mL single-necked flask, and reacted at 100°C under N₂ protection for 16 h. The mixture was filtered. The filtrate was concentrated and purified by a silica gel Flash column (0%-3% methanol/DCM) to obtain an intermediate 9-55 (150 mg, yield: 56.60 %) as a yellow solid. LCMS (ESI) m/z = 550.10 [M+H]⁺.

### Step 9: Synthesis of compound 55

The intermediate 9-55 (150 mg, 0.27 mmol) was dissolved in TBAF (10 mL) in a 25 mL single-necked flask, heated up to 60°C, and performed a reflux reaction for 1 h. The reaction solution was concentrated, washed with water to remove TBAF, extracted with EA. The organic phase was concentrated, purified by prep-HPLC (water/acetonitrile, 0.1% FA), and freeze-dried to obtain a compound 55 (77.75 mg, yield: 68.2%) as a white solid. ¹H NMR (400 MHz, DMSO) δ 13.71 (s, 1H), 8.43 (s, 1H), 7.79 (d, J = 1.3 Hz, 1H), 7.70 (d, J = 9.1 Hz, 1H), 7.47 - 7.34 (m, 5H), 7.18 (dd, J = 9.1, 2.0 Hz, 1H), 4.81 (s, 2H), 4.21 (s, 3H), 1.25 (t, J = 7.0 Hz, 3H). LCMS (ESI) m/z = 420.05 [M+H]⁺.

### Example 18: Synthesis of compound 56

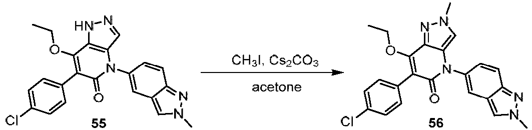

The compound 55 (50 mg, 0.12 mmol) was dissolved in acetone (3 mL) in a 25 mL single-necked flask, Cs₂CO₃ (78 mg, 0.24 mmol) was added, then CH₃I (34 mg, 0.24 mmol) was added, and stirred at room temperature for 1 h. The reaction solution was filtered, concentrated, purified by prep-HPLC (water/acetonitrile, 0.1% TFA), and freeze-dried to obtain a compound 56 (13.40 mg, yield: 25.8%) as a white solid. ¹H NMR (400 MHz, DMSO) δ 8.43 (s, 1H), 7.79 (d, J = 1.2 Hz, 1H), 7.71 (d, J = 9.1 Hz, 1H), 7.46 (s, 1H), 7.43 - 7.36 (m, 4H), 7.17 (dd, J = 9.1, 2.0 Hz, 1H), 4.78 (q, J = 7.0 Hz, 2H), 4.21 (s, 3H), 3.94 (s, 3H), 1.25 (t, J = 7.0 Hz, 3H). LCMS (ESI) m/z = 434.05 [M+H]⁺.

### Example 19: Synthesis of compound 100

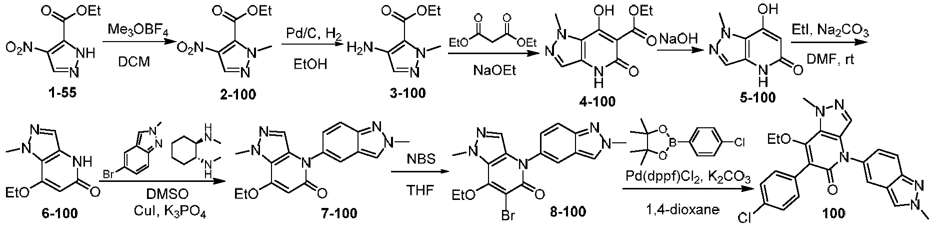

### Step 1: Synthesis of intermediate 2-100

4-nitropyrazole-5-ethyl carboxylate 1-55 (5.0 g, 27.0 mmol) was dissolved in 50 mL of DCM in a 100 mL single-necked flask, added with Me₃OBF₄ (6.0 g, 40.5 mmol) slowly, and reacted at room temperature for 3 h. The reaction solution was concentrated to obtain a crude product, which was purified by a silica gel Flash column (0%-1% methanol/DCM) to obtain an intermediate 2-100 (2.5 g, yield: 47%) as a pale yellow liquid. LCMS (ESI) m/z = 200.1 [M+H]⁺.

### Step 2: Synthesis of intermediate 3-100

The intermediate 2-100 (2.5 g, 12.56 mmol) was dissolved in 30 mL of ethanol in a 50 mL single-necked flask, added with Pd/C (0.5 g), and reacted overnight at room temperature under a hydrogen atmosphere. Pd/C was filtered through diatomaceous earth. The filtrate was concentrated to obtain an intermediate 3-100 (2.2 g, yield: 103%) as a yellow liquid. LCMS (ESI) m/z = 170.1 [M+H]⁺.

### Step 3: Synthesis of intermediate 4-100

The intermediate 3-100 (2.2 g, 13.0 mmol) was dissolved in 100 mL of ethanol in a 250 mL single-necked flask. Diethyl malonate (6.25 g, 39.0 mmol) and NaOEt (20% EtOH, 4.42 g, 39.0 mmol) were added sequentially, heated up to 80°C, and reacted overnight. The reaction solution was concentrated, dissolved in EA (20 mL), added with 50 mL of HCl (dioxane), stirred for 1 h, filtered to collect the solid to obtain an intermediate 4-100 (3.0 g, yield: 97%) as a yellow solid. LCMS (ESI) m/z = 238.2 [M+H]⁺.

### Step 4: Synthesis of intermediate 5-100

The intermediate 4-100 was dissolved in 30 mL of 15% NaOH aqueous solution in a 100 mL single-necked flask, heated up to 100°C, and reacted overnight. The mixture was diluted with water, adjusted the pH to 3-4 with dilute hydrochloric acid to precipitate a white solid, and filtered to collect the white solid to obtain an intermediate 5-100 (1.2g, yield: 57%) as a white solid. LCMS (ESI) m/z = 166.2 [M+H]⁺.

### Step 5: Synthesis of intermediate 6-100

The intermediate 5-100 (1.0 g, 6.1 mmol) was dissolved in 20 mL of DMF in a 100 mL single necked flask, added with Na₂CO₃ (1.9 g, 18.2 mmol) and EtI (0.94 g, 9.1 mmol), and stirred overnight at room temperature. The reaction solution was poured into water, and extracted with EA. The organic phase was washed with water, washed with saturated saline solution, and concentrated to obtain a crude product, which was purified by a silica gel Flash column (0%-2% methanol/DCM) to obtain an intermediate 6-100 (300 mg, yield: 26%) as a white solid. LCMS (ESI) m/z = 194.2 [M+H]⁺.

### Step 6: Synthesis of intermediate 7-100

The intermediate 6-100 (300 mg, 1.55 mmol), 5-bromo-2-methyl-2H-indazole (492 mg, 2.33 mmol), trans N,N'-dimethylcyclohexanediamine (220 mg, 1.55 mmol), cuprous iodide (295 mg, 1.55 mmol), potassium phosphate (660 mg, 3.10 mmol), DMSO (10 ml) were sequentially added into a 50 mL single-necked flask, and reacted overnight at 110°C under N₂ protection. The reaction solution was concentrated to dryness to obtain a crude product, which was purified by a silica gel Flash column (0%-3% methanol/DCM) to obtain an intermediate 7-100 (300 mg, yield: 60%) as a pale yellow solid. LCMS (ESI) m/z = 324.0 [M+H]⁺.

### Step 7: Synthesis of intermediate 8-100

The reaction was performed in a 25 mL single-necked flask. The intermediate 7-100 (300 mg, 0.99 mmol) was dissolved in 10 mL of DCM, added with NBS (211 mg, 1.19 mmol), and stirred at room temperature for 2 h. The reaction solution was concentrated to dryness, and purified by a silica gel Flash column (0%-3% methanol/DCM) to obtain an intermediate 8-100 (300 mg, yield: 73 %) as a yellow solid. LCMS (ESI) m/z = 402.0 [M+H]⁺.

### Step 8: Synthesis of compound 100

The intermediate 8-100 (80 mg, 0.2 mmol), 4-chlorophenylboronic acid pinacol ester (95 mg, 0.4 mmol), Pd(dppf)Cl₂ (29 mg, 0.02 mmol), potassium carbonate (55 mg, 0.4 mmol), 1,4-dioxane (5 mL), and water (1 ml) were sequentially added into a 25 mL single-necked flask, and reacted at 100°C under N₂ protection for 16 h. The reaction solution was concentrated to obtain a crude product, which was purified by a silica gel Flash column (0%-2% methanol/DCM) to obtain a crude product, then purified by prep-HPLC (water/acetonitrile, 0.1% FA), and freeze dried to obtain a compound 100 (16.26 mg, yield: 19%) as a white solid. ¹H NMR (400 MHz, DMSO) δ 8.44 (s, 1H), 7.79 (d, J = 1.6 Hz, 1H), 7.72 (d, J = 9.2 Hz, 1H), 7.53 - 7.44 (m, 4H), 7.17 (dd, J = 9.2, 2.0 Hz, 1H), 7.06 (s, 1H), 4.21 (s, 3H), 4.14 (s, 3H), 3.69 (q, J = 7.2 Hz, 2H), 1.15 (t, J = 7.2 Hz, 3H). LCMS (ESI) m/z = 434.2 [M+H]⁺.

### Test example 1: Biochemical test

Experimental principle: MAT2A can catalyze the conversion of L-methionine and ATP into SAM, inorganic phosphate, and inorganic diphosphate. By adding a chromogenic reagent to the enzymatic reaction system, the content of inorganic phosphates in a sample can be quantitatively detected, thereby characterizing the enzymatic activity of MAT2A.

Experimental material: Tris (Life Science #0497); BSA (Sigma #); MAT2A his-tag (BPS #71401-1); 384 well plate (Corning #3765); L-methionine (Admas #1100469); ATP (Sigma #A7699); MgCl₂ (Sigma #M8266); KCl (Sigma #7447-40-7); Brij35 (Sigma #B4184); EDTA (Sigma #E1644)

### Test method:

1. 1xAssaybuffer (buffer composition: Tris, KCl, MgCl₂, BSA, Brij35, and EDTA; the solvent was ultrapure water) was prepared. MAT2A his-tag enzyme solution (containing 1.3 µL of MAT2A enzyme and 998.7 µL of 1xAssaybuffer per 1000 µL) and substrate mixed solution (containing 5 µL of ATP, 1.3 µL of L-methionine, and 993.7 µL of 1xAssaybuffer per 1000 µL) were prepared using 1xAssayBuffer.
2. Preparation of compound concentration gradient: The test compound was tested with a starting concentration of 10 µM, and 10 equimolar concentrations were set according to a 3-fold dilution. Specifically, firstly, the compound was diluted into 10 different concentrations of series solutions in a 384 well plate (with final detection concentrations of 10, 3.33, 1.11, 0.37, 0.123, 0.041, 0.0137, 0.0046, 0.0015, and 0.0005 µM), and then 250 nL of the above series solutions was transferred to the 384 well reaction plate using an ultrasonic pipetting system Echo550 for later use. 250 nL of 100% DMSO was added into a negative control well and a positive control well, respectively. Double well test.
3. 15 µL of MAT2A his-tag enzyme solution was added into both the compound well and the positive control well; and 15 µL of 1xAssaybuffer was added into the negative control well.
4. The 384 well plate reaction plate was centrifuged at 1000 rpm for 60 seconds, shaken, mixed well, and incubated for 15 minutes. 10 µL of substrate mixed solution was added into each experimental well of the 384 well reaction plate to initiate the reaction.
5. The 384 well reaction plate was centrifuged at 1000 rpm for 60 seconds, shaken, mixed well, and incubated for 150 minutes.
6. 50 µL of enzyme reaction termination solution Biomol was added into all experimental wells of the 384 well reaction plate to terminate the reaction, centrifuged at 1000 rpm for 60 seconds, and incubated for 15 minutes. OD620 was read and data was processed.

Data analysis: the inhibition rate (%) of the compound was calculated and fitted to obtain the IC₅₀ of the tested compound. Calculation method for compound inhibition rate: Compound inhibition rate (%) = (OD620_max - OD620_stample)/(OD620_max - OD620_min)X100, wherein OD620_stample was the absorbance value of the sample well; OD620_min was the absorbance value of the positive control well, indicating the reading of the wells without enzyme activity; and OD620_max was the absorbance value of the negative control well, indicating the reading of the well without compound inhibition.

Experimental results: Under the conditions of this experiment, the inhibitory effect of the tested compound on MAT2A enzyme activity could be expressed as the IC₅₀ value of the inhibition of phosphate production level during the enzymatic reaction process. The specific MAT2A inhibitory activity of the tested compound is shown in Table 1. The results showed that, compared with compound 1 with similar structure, the compound of the present invention exhibited significant MAT2A enzyme inhibition effect.

**Table 1. Inhibition of MAT2A enzyme activity**

| Compo und | IC₅₀ (nM) | Compo und | IC₅₀ (nM) | Compo und | IC₅₀ (nM) | Compo und | IC₅₀ (nM) |
|---|---|---|---|---|---|---|---|
| **1** | >10000 | **15** | 15.3 | **28** | 15.0 | **41** | 12.7 |
| **2** | 17.5 | **16** | >10000 | **29** | 12.3 | **45** | 10.5 |
| **3** | 19.4 | **17** | 13.9 | **31** | 120.5 | **46** | 15.3 |
| **4** | 62.0 | **18** | 22.9 | **28** | 15.0 | **47** | 14.8 |
| 5 | 17.7 | **19** | 16.6 | **32** | 14.6 | **48** | 16.3 |
| **6** | 707.7 | **20** | 9.8 | **33** | 14.9 | **49** | 17.9 |
| **7** | 35.3 | **21** | 8.3 | **34** | 22.1 | **50** | 13.6 |
| **9** | 15.3 | **22** | 16.0 | **35** | 60.8 | **51** | 15.7 |
| **10** | 20.6 | **23** | 9.8 | **36** | 17.0 | **52** | 17.6 |
| **11** | 14.2 | **24** | 15.9 | **37** | 11.0 | **53** | 16.0 |
| **12** | 12.1 | **25** | 14.0 | **38** | 18.6 | **54** | 12.9 |
| **13** | 15.1 | **26** | 13.5 | **39** | 15.0 | **55** | 16.5 |
| **14** | 9.2 | **27** | 17.1 | **40** | 55.8 | **56** | 24.4 |
| **58** | 14.8 | **59** | 20.7 | **60** | 14.2 | **61** | 14.2 |
| **62** | 18.0 | **63** | 18.2 | **64** | 18.5 | **65** | 16.0 |
| **66** | 13.7 | **67** | 17.8 | **84** | 20.7 | **86** | 20.3 |
| **87** | 28.3 | **89** | 16.0 | **90** | 18.2 | **92** | 19.1 |
| **93** | 16.2 | **96** | 17.6 | **99** | 16.1 | **100** | 62.4 |
| **101** | 17.4 | **102** | 20.6 | **104** | 15.7 | **105** | 19.6 |
| **106** | 28.8 | **107** | 16.7 | **108** | 17.3 | **109** | 23.2 |
| **111** | 17.5 | **112** | 18.3 | **114** | 67.3 | **116** | 335.3 |
| **117** | 17.2 | **118** | 16.0 | **121** | 1117.0 | **122** | 27.7 |
| **123** | 11.7 | **124** | 18.0 | **125** | 45.3 | **126** | 12.6 |
| **127** | 12.4 | **128** | 8.6 | **129** | 17.8 | **130** | 11.2 |
| **131** | 36.2 | **132** | 16.5 | **133** | 27.4 | **134** | 19.1 |
| **136** | 17.9 | **137** | 16.8 | **138** | 50.7 | **139** | 376.4 |
| **140** | 13.0 | **141** | 16.7 | **142** | 10.3 | | |

### Test example 2. Human pancreatic cancer KP-4 cell activity inhibition test

Experimental principle: after co-incubating the MAT2A inhibitor to be tested with KP-4 cells for a period of time, a cell viability measurement method based on ATP content is used to characterize the effect of the tested compound on cell viability.

Experimental material: KP-4 cells (JCRB # JCRB0182); IMDM (Gibco #12440061); fetal bovine serum (EXCELL #FND500); penicillin-streptomycin (Gibco #15140-122); 0.25% Typsin-EDTA (Gibco #25200-072); DMSO (Sigma #D2650); 96 well plate (Corning #3610); CellTiter-Glo (Promega #G7571)

### Test method:

1. KP-4 cells were cultured in IMDM culture medium containing 10% fetal bovine serum and 1% penicillin-streptomycin at 37°C and 5% CO₂ in a cell culture incubator. Only cells in logarithmic growth phase were used for subsequent experiments.
2. The cells in logarithmic growth phase were inoculated into a 96 well plate, with a seeding quantity of 500 cells per well, and incubated overnight in a cell culture incubator at 37°C and 5% CO₂.
3. The compound was dissolved with DMSO, diluted with culture medium to 10 different concentrations (approximately 10000, 3333.3, 1111.1, 370, 123, 41.2, 13.7, 4.6, 1.5, and 0.5 nM) before adding it to the cell plate, and cultured at 37°C and 5% CO₂ for 5 days.
4. CellTiter-Glo reagent was added, and an enzyme-linked immunosorbent assay reader was used to detect cell viability.

Data analysis: the inhibition rate (%) of the compound was calculated and fitted to obtain the IC₅₀ of the tested compound. Calculation method for compound inhibition rate: Compound inhibition rate (%) = (Signal_max - Signal_sample)/(Signal_max - Signal_min)×100, wherein Signal_sample was the sample well reading, indicating the cell activity of the compound inhibition well; Signal_min was the positive control well reading, indicating the background activity without cells; and Signal_max was the negative control well reading, indicating the cell activity of the well without compound inhibition.

Experimental results: Under the conditions of this experiment, the IC₅₀ values of the tested compound for inhibiting KP-4 cell activity are shown in Table 2. The results showed that compared to compound 1, the typical compound of the present invention has significant anti-tumor activity.

**Table 2. Human pancreatic cancer KP-4 cell activity inhibition test**

| Compound | IC₅₀ (nM) | Compound | IC₅₀ (nM) | Compound | IC₅₀ (nM) | Compound | IC₅₀ (nM) |
|---|---|---|---|---|---|---|---|
| **1** | >10000 | **11** | 4.9 | **21** | 18.9 | **29** | 13.5 |
| **2** | 70.8 | **12** | 11.7 | **22** | 28.1 | **33** | 41.5 |
| **3** | 64.0 | **13** | 7.4 | **23** | 89.2 | **36** | 39.2 |
| **4** | 28.0 | **14** | 8.1 | **24** | 256 | **37** | 36.0 |
| **5** | 28.0 | **15** | 158.6 | **25** | 274.1 | **38** | 148.9 |
| **7** | >10000 | **17** | 18.0 | **26** | 659.0 | **41** | 11.4 |
| **9** | 24.7 | **19** | 7.4 | **27** | 6.8 | **45** | 89.1 |
| **10** | 617.6 | **20** | 223.8 | **28** | 5.2 | **46** | 60.9 |
| **47** | 6.6 | **49** | 57.8 | **50** | 21.4 | **51** | 35.7 |
| **52** | 9.0 | **53** | 19.5 | **54** | 19.2 | **55** | 2.5 |
| **58** | 3.5 | **62** | 180.5 | **63** | 2.4 | **66** | 12.7 |
| **84** | 25.0 | **90** | 32.0 | **92** | 279.4 | **93** | 1.5 |
| **96** | 32.0 | **99** | 87.8 | **102** | 41.1 | **104** | 8.3 |
| **105** | 59.0 | **106** | 234.5 | **107** | 73.2 | **108** | 66.7 |
| **109** | 127.8 | **111** | 62.8 | **117** | 23.7 | **118** | 136.4 |
| **123** | 3.7 | **124** | 236.4 | **126** | 38.0 | **127** | 8.9 |
| **128** | 1.2 | **129** | 23.3 | **132** | 160.0 | **134** | 6.1 |
| **137** | 427.4 | **141** | 145.5 | | | | |

### Test example 3. In vivo anti-tumor efficacy study of compound 13

The in vivo anti-tumor efficacy of compound 13 and AG-270 MAT2A inhibitor, 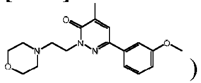 were evaluated using a human B-cell lymphoma DOHH-2 cell subcutaneous xenograft tumor model.

The female CB-17 SCID mice used were purchased from Beijing Vital River Laboratory Animal Technology Co., Ltd. Tumor cell culture and subcutaneous inoculation: Human B-cell lymphoma DOHH-2 cells (DSMZ) were cultured in vitro in RPMI-1640 medium containing 10% fetal bovine serum and 1% bispecific antibody, and incubated in a 5% CO₂ incubator at 37°C. When the required number of cells was reached, the cells were collected, and counted. 0.2 mL (10×10⁶) of DOHH-2 cells (with matrix gel added, volume ratio of 1:1) were subcutaneously inoculated into the right back of each mouse. When the average tumor volume reached 100-150 mm³, random grouping would begin, with 5 mice in each group. The administration group (compound 13 or compound AG-270) was performed intragastric administration twice a day (dosage shown in Table 3), while the negative control group was performed intragastric administration using the same volume of blank solvent per day.

### Experimental indicator and data analysis

The experimental indicator was to examine whether tumor growth was inhibited, delayed, or cured. The tumor diameter was measured twice a week using a vernier caliper. The formula for calculating tumor volume was: V = 0.5a × b², wherein a and b indicated the long and short diameters of tumors, respectively.

The anti-tumor efficacy of the compound was evaluated using TGI (%) or relative tumor proliferation rate T/C (%).

TGI (%) reflects the tumor growth inhibition rate. Calculation of TGI (%): TGI (%)=[(1-(average tumor volume at the end of administration in the treatment group-average tumor volume at the beginning of administration in the treatment group))/(average tumor volume at the end of treatment in the negative control group-average tumor volume at the beginning of treatment in the negative control group)] × 100%.

Relative tumor proliferation rate T/C (%): The calculation formula was as follows: T/C%=TRTV/CRTV×100% (TRTV: administration group RTV; CRTV: negative control group RTV). The calculation formula of the relative tumor volume (RTV) was RTV=Vt/V₀, wherein V₀ was the average tumor volume measured at the time of group administration (i.e. d₀), Vt was the average tumor volume measured at a certain measurement, and TRTV and CRTV took the same day's data.

Experimental results: The TGI and T/C calculated by each group based on the tumor volume growth curve are shown in Table 3, indicating that compound 13 of the present invention exhibits dose-dependent in vivo efficacy.

**Table 3. Evaluation of antitumor efficacy of compound 13 on human B-cell lymphoma DOHH-2 xenograft tumor model**

| Group | Dosage of administration | Number of animals | TGI (%) | T/C (%) |
|---|---|---|---|---|
| Compound 13 | 5 mg/kg, BID | 5 | 68.82 | 35.54 |
| Compound 13 | 10 mg/kg, BID | 5 | 78.48 | 26.46 |
| Compound 13 | 20 mg/kg, BID | 5 | 89.67 | 15.98 |
| AG-270 | 80 mg/kg, QD | 5 | 82.92 | 22.32 |

## Claims

1. A bicyclic compound represented by formula (I) or a pharmaceutically acceptable salt, an enantiomer, a diastereomer, a racemate, an atropisomer, a polymorph, a solvate, or an isotope labeled compound thereof, 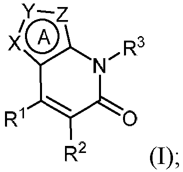 wherein,
R¹ is selected from halogen, C₁-C₆ alkyl, C₂-C₆ alkenyl, C₂-C₆ alkynyl, C₁-C₆ alkylsulfonyl, C₃-C₇ cycloalkyl, 3- to 6-membered heterocycloalkyl, cyano, nitro, carboxyl, -NR^{a}R^{a2}, -NHCOR^{a}, -OR^{a}, and -SR^{a}, the C₁-C₆ alkyl, C₂-C₆ alkenyl, C₂-C₆ alkynyl, C₃-C₇ cycloalkyl, and 3- to 6-membered heterocycloalkyl are unsubstituted or optionally substituted with one or more substituents selected from D and halogen;
R^{a} and R^{a2} are each independently selected from H, C₁-C₁₀ alkyl, C₃-C₁₀ cycloalkyl, C₆-C₁₀ aryl, 5- to 10-membered heteroaryl, 3- to 6-membered heterocycloalkyl, C₁-C₁₀ alkyl substituted with one or more substituents selected from group A, and C₃-C₁₀ cycloalkyl substituted with one or more substituents selected from the group A; the group A substituents include D, halogen, C₁-C₃ alkoxy, hydroxyl, C₆-C₁₀ aryl, 5- to 10-membered heteroaryl, and C₃-C₁₀ cycloalkyl that is unsubstituted or substituted with one or more substituents selected from group A2; the group A2 substituents include D, halogen, hydroxyl, C₁-C₆ alkyl, and C₁-C₁₀ alkoxy;
R² and R³ are each independently selected from unsubstituted or substituted C₃-C₁₀ cycloalkyl, unsubstituted or substituted C₆-C₁₀ aryl, unsubstituted or substituted 4- to 6-membered heterocycloalkyl, and unsubstituted or substituted 5- to 10-membered heteroaryl; wherein the substitution refers to being substituted by one or more substituents selected from group B, the group B substituents include halogen, cyano (-CN), hydroxyl (-OH), oxo (=O), mercapto (-SH), amino (-NH₂), nitro (-NO₂), 4- to 6-membered heterocycloalkyl, C₁-C₄ alkyl that is unsubstituted or substituted with one or more substituents selected from group C, C₃-C₇ cycloalkyl that is unsubstituted or substituted with halogen, C₁-C₄ alkoxy that is unsubstituted or substituted with halogen, -COOH, -CONHR^{b}, -NHCOR^{b}, and -NHSO₂R;
the group C substituents include D, halogen, hydroxyl, C₃-C₆ cycloalkyl, 4- to 6-membered heterocycloalkyl, and C₁-C₄ alkoxy;
R^{b} is selected from H, C₁-C₄ alkyl, C₃-C₁₀ cycloalkyl, C₁-C₁₀ alkoxy, and C₆-C₁₀ aryl, wherein the C₁-C₄ alkyl, C₃-C₁₀ cycloalkyl, C₁-C₁₀ alkoxy, and C₆-C₁₀ aryl in R^{b} are unsubstituted or substituted with one or more groups selected from halogen, hydroxyl, and cyano;
ring A is a five-membered heteroaromatic ring, wherein at most one of X, Y, and Z is CR⁴, and the rest are each independently selected from N, NR⁵, O, and S; and
R⁴ and R⁵ are each independently selected from H, D, halogen, amino, C₁-C₆ alkyl, and C₃-C₆ cycloalkyl, the C₁-C₆ alkyl, and C₃-C₆ cycloalkyl are unsubstituted or substituted with hydroxyl.

2. The bicyclic compound or a pharmaceutically acceptable salt, an enantiomer, a diastereomer, a racemate, an atropisomer, a polymorph, a solvate, or an isotope labeled compound thereof according to claim 1, wherein
R¹ is selected from C₁-C₆ alkyl, C₃-C₇ cycloalkyl, 3- to 6-membered heterocycloalkyl, -OR^{a}, -SR^{a}, and NR^{a}R^{a2}; the C₁-C₆ alkyl, C₃-C₇ cycloalkyl, and 3- to 6-membered heterocycloalkyl are unsubstituted or substituted with one or more substituents selected from D and halogen; R^{a} and R^{a2} are each independently selected from H, C₃-C₇ cycloalkyl, and C₁-C₆ alkyl that is unsubstituted or substituted with one or more substituents selected from group A, the group A substituents include D, halogen, C₁-C₃ alkoxy, hydroxyl, C₆-C₁₀ aryl, 5-to 10-membered heteroaryl, and C₃-C₁₀ cycloalkyl that is unsubstituted or substituted with one or more substituents selected from group A2; the group A2 substituents include D, halogen, hydroxyl, C₁-C₆ alkyl, and C₁-C₆ alkoxy; and
R² and R³ are each independently selected from unsubstituted or substituted C₃-C₁₀ cycloalkyl, unsubstituted or substituted C₆-C₁₀ aryl, and unsubstituted or substituted 5- to 10-membered heteroaryl, the substitution refers to being substituted by one or more substituents selected from group B; the group B substituents include halogen, cyano, hydroxyl, mercapto, nitro, amino, oxo, unsubstituted or substituted 4- to 6-membered heterocycloalkyl, C₁-C₄ alkyl that is unsubstituted or substituted with one or more substituents selected from group C, C₃-C₇ cycloalkyl that is unsubstituted or substituted with halogen, and C₁-C₄ alkoxy that is unsubstituted or substituted with halogen; the group C substituents include D, halogen, hydroxyl, C₃-C₆ cycloalkyl, 4- to 6-membered heterocycloalkyl, and C₁-C₄ alkoxy; particularly, the 5- to 10-membered heteroaryl is selected from benzene-fused 5-membered heteroaryl, benzene-fused 6-membered heteroaryl, 6-membered heteroaryl-fused 5-membered heteroaryl, 6-membered heteroaryl-fused 6-membered heteroaryl, and

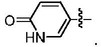

3. The bicyclic compound or a pharmaceutically acceptable salt, an enantiomer, a diastereomer, a racemate, an atropisomer, a polymorph, a solvate, or an isotope labeled compound thereof according to claim 1 or 2, wherein
R¹ is selected from C₂-C₆ alkyl, C₃-C₆ cycloalkyl, -OR^{a}, -SR^{a}, and NR^{a}R^{a2}; the C₂-C₆ alkyl and C₃-C₆ cycloalkyl are unsubstituted or substituted with one or more substituents selected from D and halogen; R^{a} and R^{a2} are each independently selected from H, C₃-C₆ cycloalkyl, and C₂-C₆ alkyl that is unsubstituted or substituted with one or more substituents selected from group A, the group A substituents include **D,** halogen, methoxy, hydroxyl, and C₃-C₆ cycloalkyl;
R² and R³ are each independently selected from unsubstituted or substituted cyclohexyl, unsubstituted or substituted phenyl, and unsubstituted or substituted 5- to 10-membered heteroaryl, the 5- to 10-membered heteroaryl is selected from the following structures: 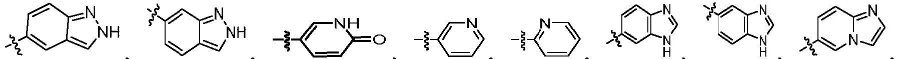 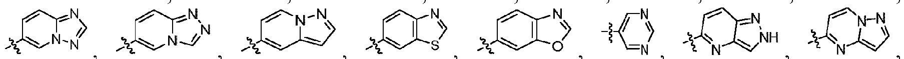 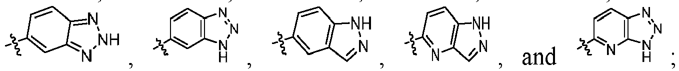 the substitution in the unsubstituted or substituted phenyl, and unsubstituted or substituted 5- to 10-membered heteroaryl refers to being substituted by one or more substituents selected from group B, the group B substituents include halogen, cyano, hydroxyl, mercapto, nitro, amino, 4- to 6-membered heterocycloalkyl, C₁-C₄ alkyl that is unsubstituted or substituted with one or more substituents selected from group C, C₃-C₇ cycloalkyl that is unsubstituted or substituted with halogen, and C₁-C₄ alkoxy that is unsubstituted or substituted with halogen; the group C substituents include D, halogen, C₃-C₆ cycloalkyl, hydroxyl, 4- to 6-membered heterocycloalkyl, and C₁-C₄ alkoxy; and
R⁵ is independently H, D, C₁-C₃ alkyl, or C₃-C₆ cycloalkyl at each occurrence, the C₁-C₃ alkyl and C₃-C₆ cycloalkyl are unsubstituted or substituted with hydroxyl.

4. The bicyclic compound or a pharmaceutically acceptable salt, an enantiomer, a diastereomer, a racemate, an atropisomer, a polymorph, a solvate, or an isotope labeled compound thereof according to any one of claims 1-3, wherein
R¹ is selected from C₂-C₆ alkyl, -OR^{a}, and NHR^{a}; R^{a} is selected from C₃-C₆ cycloalkyl, C₂-C₆ alkyl that is unsubstituted or substituted with one or more substituents selected from group A; the group A substituents include D, halogen, methoxy, hydroxyl, and C₃-C₆ cycloalkyl;
R² and R³ are each independently selected from: 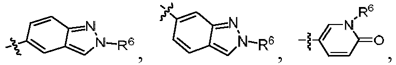 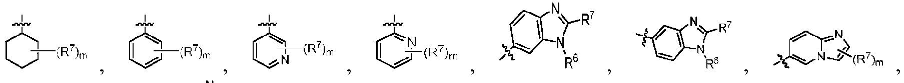 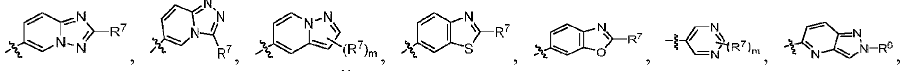 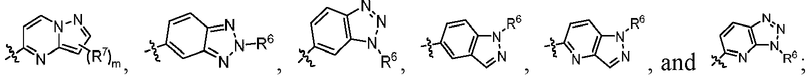
wherein (R⁷)ₘ represents m identical or different R⁷ substituents on the ring where it is located; m is 1, 2 or 3; R⁶ and each R⁷ are each independently selected from H, halogen, cyano, hydroxyl, mercapto, nitro, amino, 4- to 6-membered heterocycloalkyl, C₁-C₄ alkyl that is unsubstituted or substituted with one or more substituents selected from group C, C₃-C₇ cycloalkyl that is unsubstituted or substituted with halogen, and C₁-C₄ alkoxy that is unsubstituted or substituted with halogen; the group C substituents include D, halogen, C₃-C₆ cycloalkyl, hydroxyl, 4- to 6-membered heterocycloalkyl, and C₁-C₄ alkoxy; preferably, m is 1 or 2; R⁶ and each R⁷ are each independently selected from H, halogen, cyano, hydroxyl, mercapto, nitro, amino, 4- to 6-membered heterocycloalkyl, C₁-C₂ alkyl that is unsubstituted or substituted with one or more substituents selected from group C, cyclopropyl that is unsubstituted or substituted with halogen, and C₁-C₂ alkoxy that is unsubstituted or substituted with halogen; the group C substituents include D, halogen, C₃-C₆ cycloalkyl, hydroxyl, 4- to 6-membered heterocycloalkyl, and C₁-C₄ alkoxy; and
R⁵ is independently H, methyl, or hydroxyethyl at each occurrence.

5. The bicyclic compound or a pharmaceutically acceptable salt, an enantiomer, a diastereomer, a racemate, an atropisomer, a polymorph, a solvate, or an isotope labeled compound thereof according to any one of claims 1-4, wherein
R¹ is selected from C₂-C₆ alkyl, -OR^{a}, -SR^{a}, and NHR^{a}; R^{a} is selected from C₃-C₆ cycloalkyl, unsubstituted C₂-C₄ alkyl, C₂-C₄ alkyl substituted with halogen, C₂-C₄ alkyl substituted with cyclopropyl, C₂-C₄ alkyl substituted with methoxy, and C₂-C₄ alkyl substituted with hydroxyl;
R² and R³ are each independently selected from: 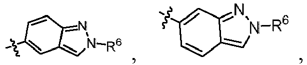 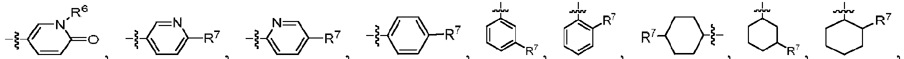 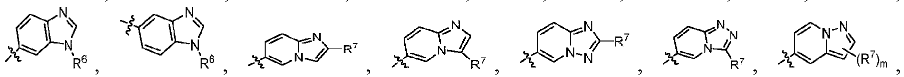 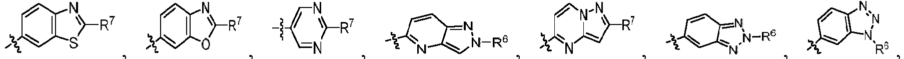 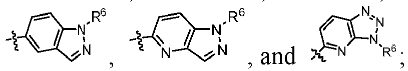
wherein m is 1, 2, or 3; R⁶ and R⁷ are each independently selected from H, halogen, cyano, hydroxyl, mercapto, nitro, amino, 4- to 6-membered heterocycloalkyl, C₁-C₄ alkyl that is unsubstituted or substituted with one or more substituents selected from group C, C₃-C₇ cycloalkyl that is unsubstituted or substituted with halogen, and C₁-C₄ alkoxy that is unsubstituted or substituted with halogen; the group C substituents include D, halogen, C₃-C₆ cycloalkyl, hydroxyl, 4- to 6-membered heterocycloalkyl, and C₁-C₄ alkoxy;
preferably, R⁶ and R⁷ are each independently selected from H, halogen, cyano, hydroxyl, mercapto, nitro, amino, 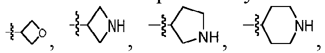 C₁-C₃ alkyl that is unsubstituted or substituted with one or more substituents selected from group C, cyclopropyl that is unsubstituted or substituted with halogen, and C₁-C₂ alkoxy that is unsubstituted or substituted with halogen; the group C substituents include D, halogen, C₂-C₆ cycloalkyl, hydroxyl, methoxy, 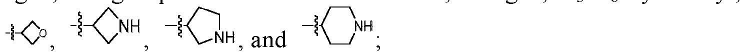
preferably,
R¹ is selected from propyl, -OC₂H₅, -SC₂H₅, -OCH₂CF₃, -NHCH₃, -NHC₂H₅, 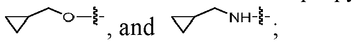
R² is selected from 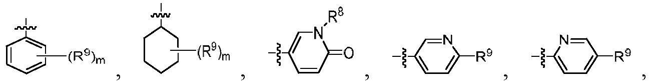 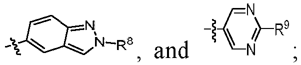 wherein m is 1, 2, or 3; R⁸ and R⁹ are each independently selected from H, halogen, CN, methoxy, methyl substituted with one or more halogens, methoxy substituted with one or more halogens, and cyclopropyl; preferably, R² is selected from 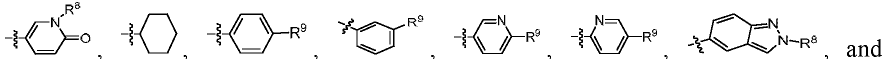 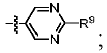 wherein R⁸ and R⁹ are each independently selected from H, halogen, cyano, methoxy, methyl substituted with one or more halogens, methoxy substituted with one or more halogens, and cyclopropyl; and
R³ is selected from 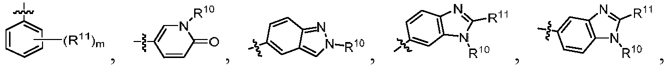 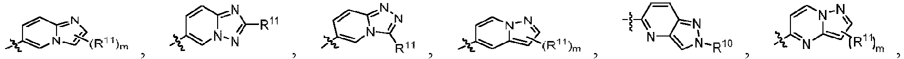 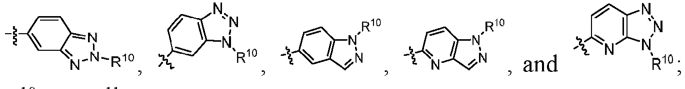 wherein m is 1, 2, or 3; R¹⁰ and R¹¹ are each independently selected from hydrogen, methyl, ethyl, isopropyl, cyano, methoxy, ethyl substituted with hydroxyl, ethyl substituted with methoxy, cyclopropyl,

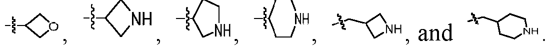

6. The bicyclic compound or a pharmaceutically acceptable salt, an enantiomer, a diastereomer, a racemate, an atropisomer, a polymorph, a solvate, or an isotope labeled compound thereof according to any one of claims 1-5, wherein the bicyclic compound is selected from the following structures: 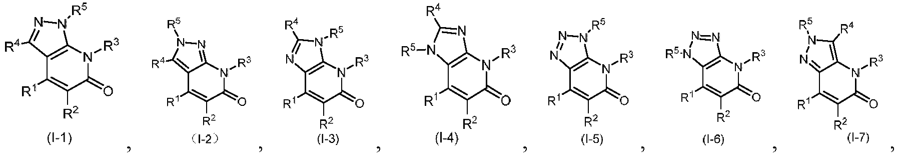 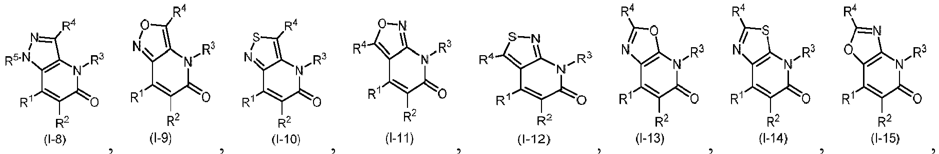 and 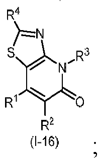
preferably, the bicyclic compound is selected from the following structures: 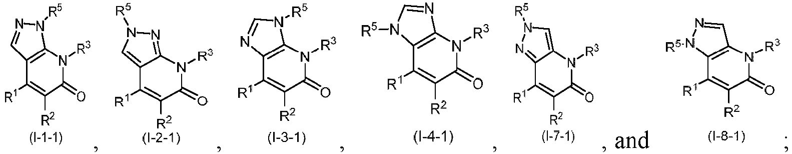
wherein R¹, R², R³, and R⁵ have the same definitions as the corresponding claims.

7. The bicyclic compound or a pharmaceutically acceptable salt, an enantiomer, a diastereomer, a racemate, an atropisomer, a polymorph, a solvate, or an isotope labeled compound thereof according to claim 1, wherein the bicyclic compound is selected from the following structures:
| No. | Structure | No. | Structure | No. | Structure |
|---|---|---|---|---|---|
| 2 | 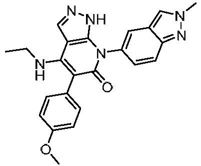 | 3 | 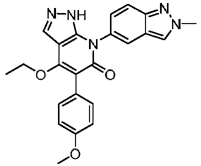 | 4 | 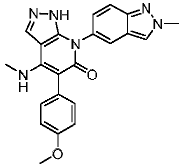 |
| 5 | 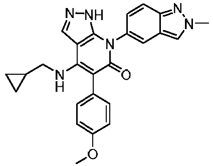 | 6 | 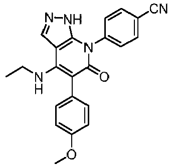 | 7 | 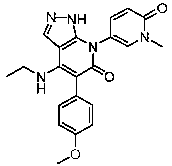 |
| 8 | 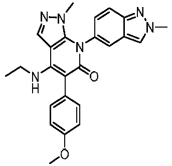 | 9 | 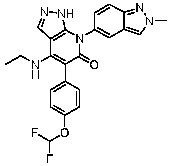 | 10 | 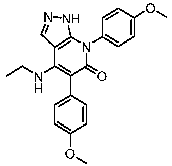 |
| 11 | 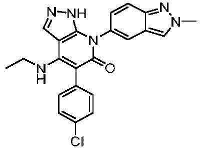 | 12 | 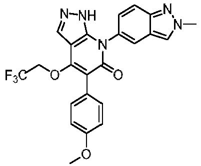 | 13 | 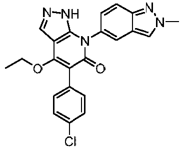 |
| 14 | 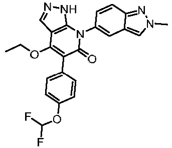 | 15 | 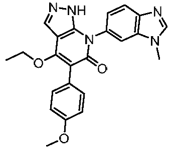 | 16 | 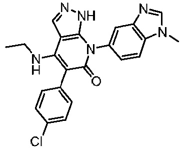 |
| 17 | 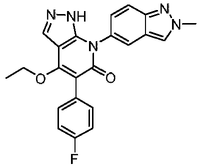 | 18 | 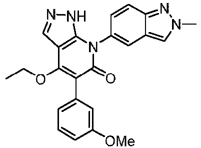 | 19 | 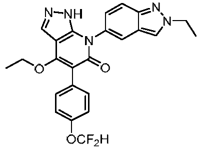 |
| 20 | 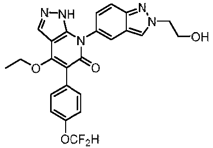 | 21 | 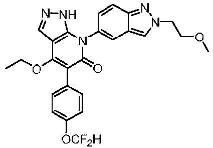 | 22 | 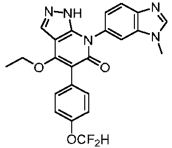 |
| 23 | 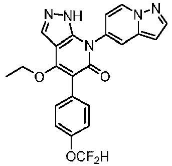 | 24 | 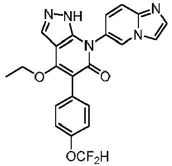 | 25 | 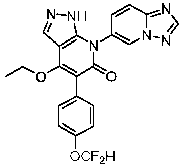 |
| 26 | 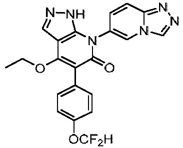 | 27 | 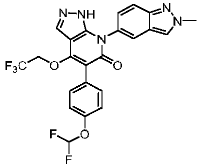 | 28 | 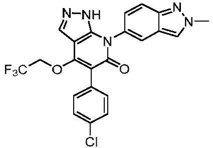 |
| 29 | 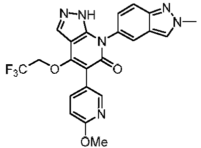 | 30 | 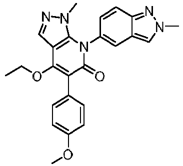 | 31 | 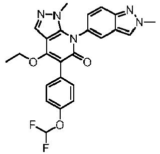 |
| 32 | 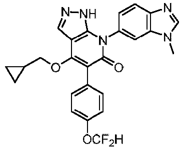 | 33 | 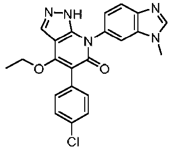 | 34 | 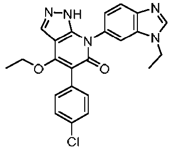 |
| 35 | 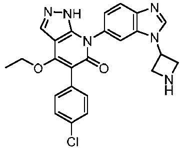 | 36 | 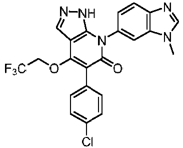 | 37 | 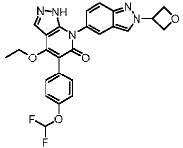 |
| 38 | 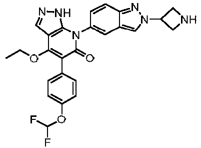 | 39 | 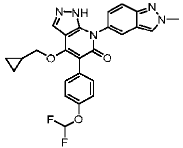 | 40 | 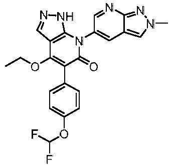 |
| 41 | 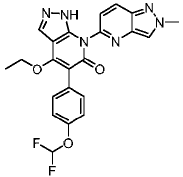 | 42 | 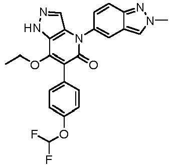 | 43 | 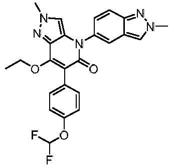 |
| 44 | 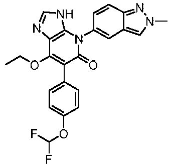 | 45 | 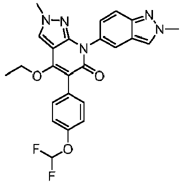 | 46 | 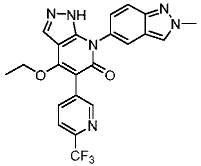 |
| 47 | 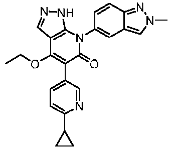 | 48 | 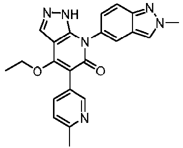 | 49 | 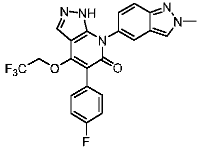 |
| 50 | 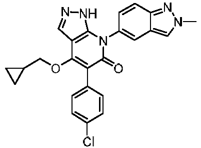 | 51 | 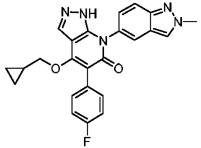 | 52 | 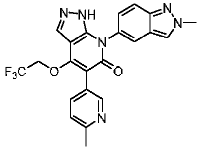 |
| 53 | 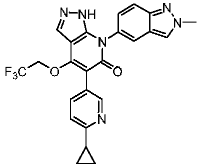 | 54 | 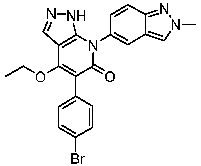 | 55 | 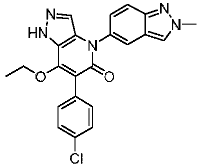 |
| 56 | 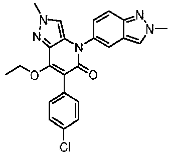 | 57 | 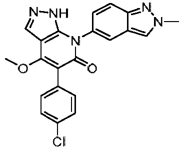 | 58 | 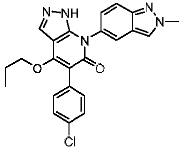 |
| 59 | 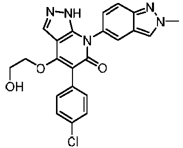 | 60 | 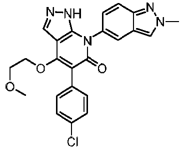 | 61 | 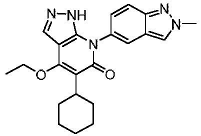 |
| 62 | 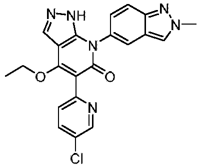 | 63 | 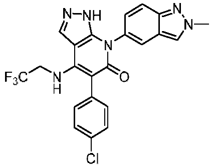 | 64 | 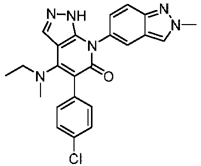 |
| 65 | 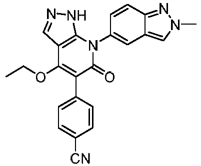 | 66 | 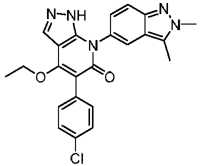 | 67 | 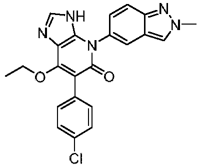 |
| 68 | 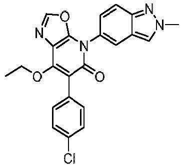 | 69 | 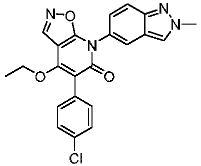 | 70 | 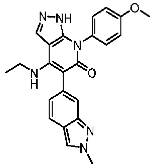 |
| 71 | 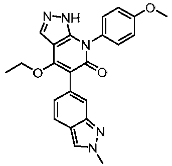 | 72 | 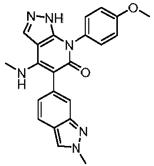 | 73 | 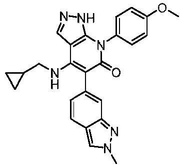 |
| 74 | 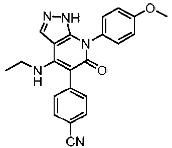 | 75 | 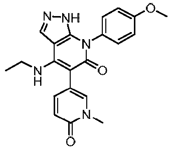 | 84 | 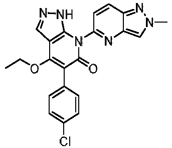 |
| 86 | 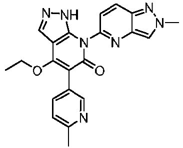 | 87 | 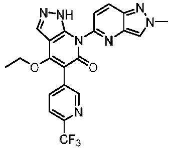 | 89 | 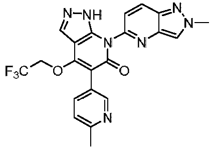 |
| 90 | 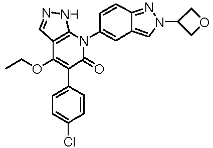 | 92 | 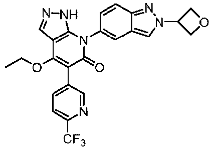 | 93 | 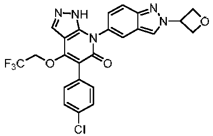 |
| 96 | 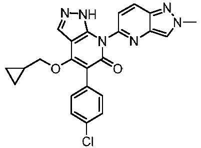 | 99 | 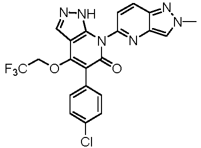 | 100 | 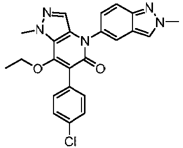 |
| 101 | 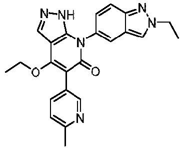 | 102 | 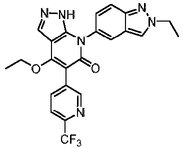 | 104 | 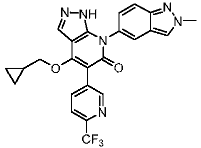 |
| 105 | 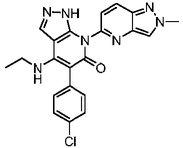 | 106 | 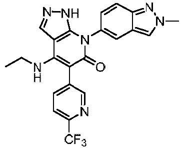 | 107 | 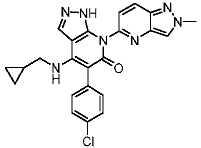 |
| 108 | 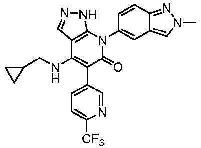 | 109 | 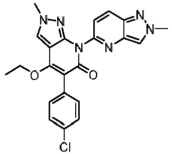 | 111 | 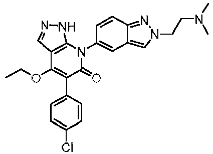 |
| 112 | 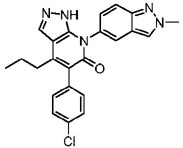 | 114 | 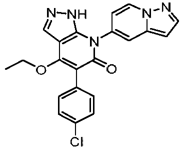 | 116 | 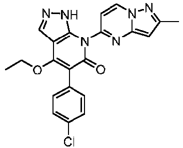 |
| 117 | 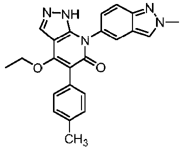 | 118 | 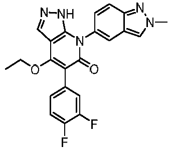 | 121 | 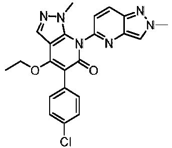 |
| 122 | 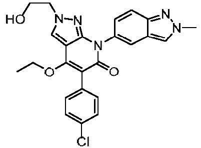 | 123 | 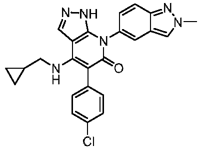 | 124 | 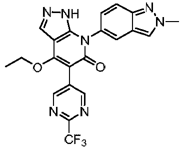 |
| 125 | 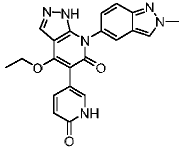 | 126 | 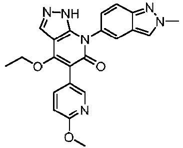 | 127 | 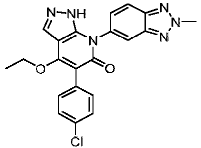 |
| 128 | 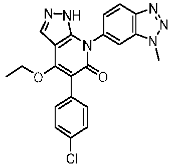 | 129 | 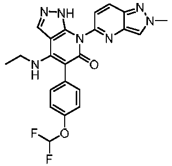 | 130 | 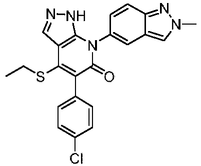 |
| 131 | 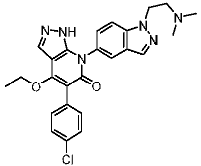 | 132 | 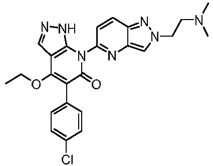 | 133 | 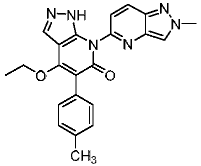 |
| 134 | 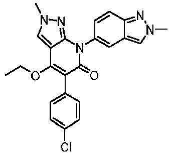 | 136 | 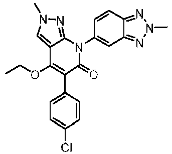 | 137 | 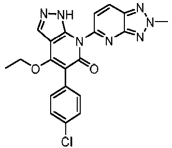 |
| 138 | 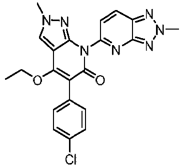 | 139 | 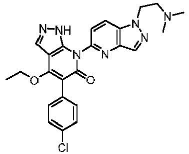 | 140 | 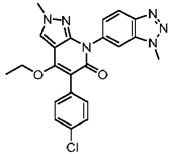 |
| 141 | 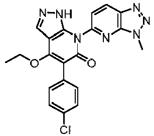 | 142 | 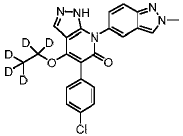 | | 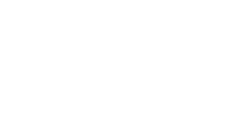 |

8. A pharmaceutical composition comprising a therapeutically effective dose of one or more selected from the bicyclic compound or a pharmaceutically acceptable salt, an enantiomer, a diastereomer, a racemate, an atropisomer, a polymorph, a solvate, or an isotope labeled compound thereof according to any one of claims 1-7, at least one pharmaceutically acceptable carrier, and optionally, one or more other therapeutic agents.

9. Use of the bicyclic compound or a pharmaceutically acceptable salt, an enantiomer, a diastereomer, a racemate, an atropisomer, a polymorph, a solvate, or an isotope labeled compound thereof according to any one of claims 1-7, or the pharmaceutical composition according to claim 8, in preparing drugs for inhibiting MAT2A activity.

10. Use of the bicyclic compound or a pharmaceutically acceptable salt, an enantiomer, a diastereomer, a racemate, an atropisomer, a polymorph, a solvate, or an isotope labeled compound thereof according to claims 1-7, or the pharmaceutical composition according to claim 8, in preparing drugs for treating and/or preventing MTAP-related diseases, especially tumors;
preferably, the tumors include MTAP deleted tumors; tumors with low expression of MTAP; tumors with abnormal expression of MAT2A; and other MAT2A dependent tumors; and
more preferably, the tumors include breast cancer, lung cancer, glioblastoma, brain cancer and spinal cancer, head and neck cancer, skin cancer, reproductive system cancer, gastrointestinal system cancer, esophageal cancer, nasopharyngeal cancer, pancreatic cancer, rectal cancer, hepatocellular carcinoma, cholangiocarcinoma, gallbladder cancer, colon cancer, multiple myeloma, kidney and bladder cancer, bone cancer, malignant mesothelioma, sarcoma, lymphoma, adenocarcinoma, thyroid cancer, heart tumor, germ cell tumor, malignant neuroendocrine tumor, malignant rhabdoid tumor, soft tissue sarcoma, midline tract cancer and an unknown primary cancer.
